# EUROPEAN PATENT APPLICATION

(11) **EP 4 545 526 A1**
(43) Date of publication of application: **30.04.2025**
(21) Application number: 23826569.8
(22) Date of filing: 21.06.2023
(51) Int. Cl.: C07D 417/14, A61K 31/4725, A61P 35/00, A61P 19/02

(54) **BENZOTHIAZOLE COMPOUND AND USE THEREOF**

(30) Priority: 24.06.2022 CN 202210727253
(71) Applicant: Nanjing Reju Therapeutics, Inc., Nanjing, Jiangsu 211199 (CN)
(72) Inventor: JI, Yin, Nanjing, Jiangsu 211199 (CN); WANG, Xueping, Nanjing, Jiangsu 211199 (CN); ZHAI, Hui, Nanjing, Jiangsu 211199 (CN)
(74) Representative: Calysta NV
(86) International application number: PCT/CN2023/101959
(87) International publication number: WO 2023/246924

(57) **Abstract**

The present invention relates to a benzothiazole compound represented by formula (I), or a pharmaceutically acceptable salt, solvate, hydrate, polymorph, eutectic, tautomer, stereoisomer, or prodrug thereof. The present invention further relates to a use of the benzothiazole compound in the preparation of a drug for preventing or treating age-related diseases.

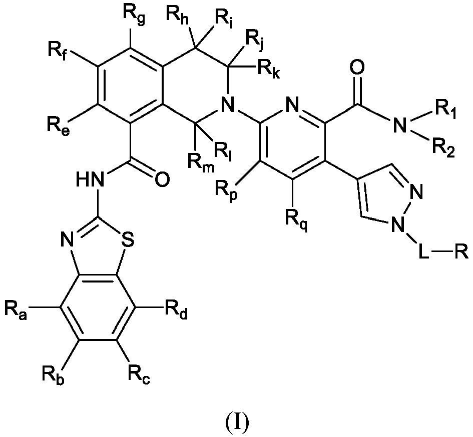

## Description

### TECHNICAL FIELD

The present invention relates to the field of medicine, and in particularly to a benzothiazole compound capable of targeted killing senescent cells, and its use in preventing or treating aging-related diseases.

### BACKGROUND

As individuals age, a gradual decline in the body's biological functions is observed, often accompanied by the onset of various age-related diseases. Therefore, the development of effective aging intervention strategies is crucial for extending the healthy lifespan of the elderly and reducing the incidence of age-related diseases. The accumulation of senescent cells in the body is an important driving force for individual aging and the emergency of age-related diseases. On one hand, the senescence of certain proliferating cells or stem cells can lead to a decline in an individual's regeneration ability, affecting the body's recovery ability and normal functions; More importantly, senescent cells secrete a large amount of inflammatory factors, known as Senescence-Associated Secretory Phenotype (SASP), which create a chronic inflammatory microenvironment, accelerate the body's aging, and promote the occurrence of age-related diseases . Researches have shown that targeted elimination of senescent cells through genetic methods or small molecules can reduce chronic inflammation, enhance tissue repair abilities, improve symptoms of age-related diseases such as osteoarthritis and idiopathic pulmonary fibrosis, and alleviate the decline in physiological functions of aging organisms. Therefore, the selective elimination of senescent cells is a promising approach for treating age-related diseases and improving the physiological functions of elderly individuals.

Researchers have demonstrated that elimination of senescent cells in the body can prolong lifespan and delay the occurrence of diseases related to aging, providing a theoretical foundation for strategies of targeted elimination of senescent cells to achieve anti-aging and treat age-related diseases. Currently, several small molecule compounds, known as "senolytics", have been identified that can selectively eliminate senescent cells. Due to the heterogeneity of senescent cells, these senolytics can achieve selective killing of senescent cells to some extent, However, there remains significant potential for improvement in reducing toxicity and improving specificity of cell elimination. The early senolytics primarily targeted key components in the Senescent Cell anti-Apoptotic Pathways (SCAP), that is, by inhibiting the anti-apoptotic signals of senescent cells and thereby inducing their death. In comparison to normal cells, the expression of the anti-apoptotic protein BCL2/BCL-XL is significantly increased in senescent cells, which counteracts the activity of pro-apoptotic proteins such as Bax, making senescent cells resistant to apoptosis. As a dual inhibitor of the classic anti-apoptotic protein BCL2/BCL-XL, ABT263 (navitoclax) has been shown to specifically induce apoptosis in senescent cells.

A-1331852 (CAS1430844-80-6) is a small molecule inhibitor that specifically targets Bcl-xL. Beyond its remarkable killing efficacy against Bcl-xL-dependent cancer cells, A-1331852 also exhibits high activity in eliminating senescent cells. However, on one hand, due to its extremely strong inhibitory effect on Bcl-xL, A-1331852 causes the killing of platelets at low concentrations and exhibits significant platelet toxicity under *in vivo* conditions, restricting the clinical application of this molecule. On the other hand, there is room for improvement in the physicochemical properties of A-1331852 molecule, such as its low solubility in water and oral bioavailability of only about 11% in rats. Based on these shortcomings, A-1331852 is only used as a research tool compound as an excellent Bcl-xL-specific inhibitor.

Currently, there is a lack of a Bcl-xL inhibitor that combines strong inhibitory effect on Bcl-xL with superior physicochemical properties and enhanced safety profiles.

### SUMMARY OF THE INVENTION

To address at least one of technical challenges present in the prior art, the present invention provides a class of novel benzothiazole compounds. These compounds can significantly reduce platelet toxicity, selectively kill senescent cells and tumor cells within a broader safety window, and minimize the side effects associated with killing normal cells, thereby offering enhanced selectivity and safety. Furthermore, the compounds of the present invention may also exhibit excellent physicochemical properties, especially significantly improved water solubility.

In a first aspect, the present application provides a benzothiazole compound having a structure represented by formula I: 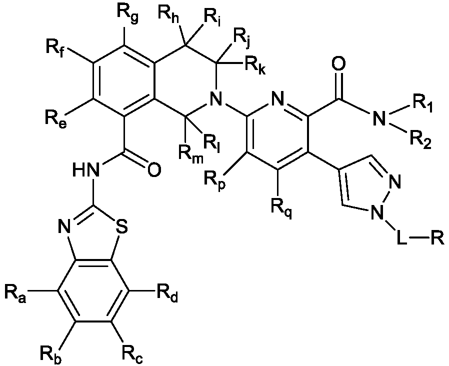 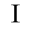 wherein,
R₁ and R₂ are independently selected from the group consisting of C1-C10 chain alkyl, C3-C8 cycloalkyl, C2-C8 chain alkenyl, C2-C8 alkynyl, C6-C20 aryl, C1-C20 heteroaryl, and C2-C20 heteroalicyclic group, wherein CH₂ in the C1-C10 chain alkyl, C3-C8 cycloalkyl, C2-C8 chain alkenyl, and C2-C8 alkynyl can be replaced with one or more groups selected from the group consisting of -O-, -S-, -SO₂-, -C(O)-, and -NR₃-, and the C1-C10 chain alkyl, C3-C8 cycloalkyl, C2-C8 chain alkenyl, C2-C8 alkynyl, C6-C20 aryl, C1-C20 heteroaryl, and C2-C20 heteroalicyclic group are optionally substituted with one or more substituents selected from the group consisting of halogen atom, cyano, nitro, C6-C20 aryl, C1-C20 heteroaryl, C1-C10 chain alkoxy, C6-C20 aryloxy, C2-C20 heteroalicyclic group, amino, hydroxyl, mercapto, 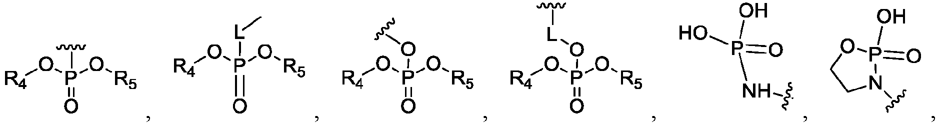 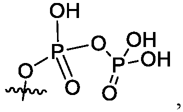 and -NR₄R₅; or
R₁ and R₂, together with the N atom to which they are connected, form a heteroalicyclic group, preferably a C2-C20 heteroalicyclic group; or
R₁ is hydrogen, and R₂ is selected from the group consisting of C1-C10 chain alkyl, C3-C8 cycloalkyl, C2-C8 chain alkenyl, C2-C8 alkynyl, C6-C20 aryl, C1-C20 heteroaryl, and C2-C20 heteroalicyclic group, wherein CH₂ in the C1-C10 chain alkyl, C3-C8 cycloalkyl, C2-C8 chain alkenyl, and C2-C8 alkynyl can be replaced with one or more groups selected from the group consisting of -O-, -S-, -SO₂-, -C(O)-, and -NR₃-, and the C1-C10 chain alkyl, C3-C8 cycloalkyl, C2-C8 chain alkenyl, C2-C8 alkynyl, C6-C20 aryl, C1-C20 heteroaryl, and C2-C20 heteroalicyclic group may be substituted with halogen atom, hydroxyl, mercapto, amino, nitro, cyano, carboxyl, acyl, C1-C10 alkoxy, C6-C20 aryl, C1-C20 heteroaryl, C2-C20 heteroalicyclic group, C1-C10 chain alkyl, C2-C8 chain alkenyl, or C2-C8 alkynyl, and wherein the substituents of at least two positions together form aliphatic ring, heteroalicyclic ring, aromatic ring, or heteroaromatic ring;
R₃, R₄, and R₅ are independently selected from the group consisting of hydrogen, aryl, heteroaryl, C1-C8 chain alkyl, C3-C8 cycloalkyl, C2-C8 chain alkenyl, and C2-C8 alkynyl, and the aryl and heteroaryl are optionally substituted with halogen atom, hydroxyl, mercapto, amino, nitro, cyano, carboxyl, acyl, alkoxy, aryl, heteroaryl, heteroalicyclic group, C1-C10 alkyl, C3-C8 cycloalkyl, C2-C8 chain alkenyl, or C2-C8 alkynyl, and wherein optionally the substituents of at least two positions together form aliphatic ring, heteroalicyclic ring, aromatic ring, or heteroaromatic ring;
L is C1-C6 alkylene, and the alkylene is optionally substituted with halogen atom, hydroxyl, mercapto, amino, nitro, cyano, carboxyl, acyl, C1-C10 alkoxy, C6-C20 aryl, C1-C20 heteroaryl, C2-C20 heteroalicyclic group, C1-C10 alkyl, C3-C8 cycloalkyl, C2-C8 chain alkenyl, or C2-C8 alkynyl;
R is adamantyl, and the adamantyl is optionally substituted with halogen atom, hydroxyl, mercapto, amino, nitro, cyano, carboxyl, acyl, C1-C10 alkoxy, C6-C20 aryl, C1-C20 heteroaryl, C2-C20 heteroalicyclic group, C1-C10 alkyl, C3-C8 cycloalkyl, C2-C8 chain alkenyl, or C2-C8 alkynyl;
Rₐ, R_{b}, R_{c}, R_{d}, Rₑ, R_{f}, R_{g}, Rₕ, Rᵢ, Rⱼ, Rₖ, Rₗ, Rₘ, Rₚ, R_{q}, and R_{z} are each independently selected from the group consisting of hydrogen, halogen substituted or unsubstituted C1-C8 chain alkyl, halogen substituted or unsubstituted C3-C8 cycloalkyl, halogen substituted or unsubstituted C2-C8 chain alkenyl, halogen substituted or unsubstituted C2-C8 alkynyl, halogen atom, hydroxyl, amino, nitro, cyano, carboxyl, acyl, and halogen substituted or unsubstituted C2-C8 alkoxy.

A pharmaceutically acceptable salt, a solvate, a hydrate, a polymorph, a co-crystal, a tautomer, a stereoisomer, an isotope labeled compound (preferably deuterated compound), or a prodrug of the benzothiazole compound represented by formula I are also within the scope of the present application. Accordingly, the present application also provides a pharmaceutically acceptable salt, a solvate, a hydrate, a polymorph, a co-crystal, a tautomer, a stereoisomer, an isotope labeled compound (preferably deuterated compound), or a prodrug of the benzothiazole compound represented by formula I.

In some embodiments, the compound is represented by formula II: 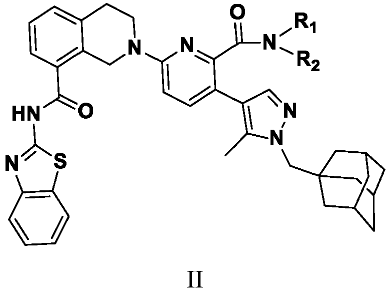 in formula II, the definitions to R₁ and R₂ are the same as those in formula I.

In some embodiments, in formula I and/or formula II, R₁ and R₂ together with the N atom to which they are connected, form a C2-C20 heteroalicyclic group, such as a C2-C10 heteroalicyclic group. The ring of the C2-C20 heteroalicyclic group optionally contains 1 or 2 additional heteroatoms selected from the group consisting of N and O.

In some embodiments, the C2-C20 heteroalicyclic group is optionally substituted with one or more substituents selected from the group consisting of halogen atom, cyano, nitro, C6-C10 aryl, C1-C10 heteroaryl, C1-C6 chain alkoxy, C6-C10 aryloxy, C2-C10 heteroalicyclic group, amino, hydroxyl, mercapto, carbonyl, carboxyl, acyl, 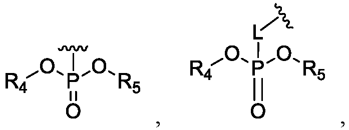 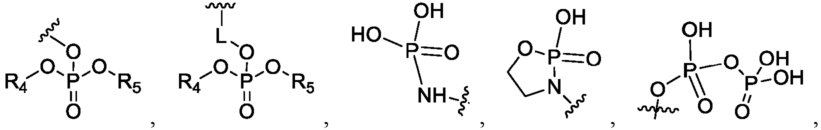 and -NR₄R₅, wherein R₄ and R₅ are independently selected from the group consisting of hydrogen, C6-C10 aryl, C1-C10 heteroaryl, C1-C8 chain alkyl, C3-C8 cycloalkyl, C2-C8 chain alkenyl, and C2-C8 alkynyl, and the aryl and heteroaryl are optionally substituted with halogen atom, hydroxyl, mercapto, amino, nitro, cyano, carboxyl, acyl, C1-C8 alkoxy, C6-C10 aryl, C1-C10 heteroaryl, C2-C10 heteroalicyclic group, C1-C8 alkyl, C3-C8 cycloalkyl, C2-C6 chain alkenyl, and C2-C8 alkynyl, optionally wherein the substituents of at least two positions together form C3-C10 aliphatic ring, C2-C10 heteroalicyclic ring, C6-C10 aromatic ring, or C1-C10 heteroaromatic ring.

In some embodiments, the C2-C20 heteroalicyclic group is optionally substituted with a substituent selected from the group consisting of halogen atom, hydroxyl, mercapto, amino, nitro, cyano, C1-C10 alkoxy, C1-C10 alkyl, C3-C8 cycloalkyl, C2-C8 chain alkenyl, C2-C8 alkynyl, 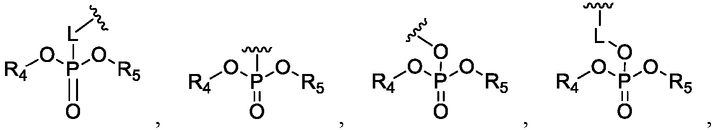 and R₄ and R₅ are independently selected from the group consisting of hydrogen and C1-C6 alkyl.

In some embodiments, the C2-C8 heteroalicyclic group is optionally substituted with a substituent selected from the group consisting of halogen, -NH₂, -OH, -NO₂, carbonyl, -CH₂OH, carboxyl, methyl, ethyl, propyl, isopropyl, methoxy, ethoxy, propoxy, and isopropoxy.

In some embodiments, R₁ and R₂ are independently selected from the group consisting of C1-C6 chain alkyl, C3-C6 cycloalkyl, C2-C6 chain alkenyl, C2-C6 alkynyl, C6-C10 aryl, C1-C10 heteroaryl, and C2-C10 heteroalicyclic group, wherein CH₂ in the C1-C6 chain alkyl, C3-C6 cycloalkyl, C2-C6 chain alkenyl, and C2-C6 alkynyl can be replaced with one or more groups selected from the group consisting of -O-, -S -, -SO₂-, -C(O)-, and -NR₃-, and the C1-C6 chain alkyl, C3-C6 cycloalkyl, C2-C6 chain alkenyl, C2-C6 alkynyl, C6-C10 aryl, C1-C10 heteroaryl, and C2-C10 heteroalicyclic group are optionally substituted with one or more substituents selected from the group consisting of halogen atom, cyano, nitro, C6-C10 aryl, C1-C10 heteroaryl, C1-C6 chain alkoxy, C6-C10 aryloxy, C2-C10 heteroalicyclic group, amino, hydroxyl, mercapto, 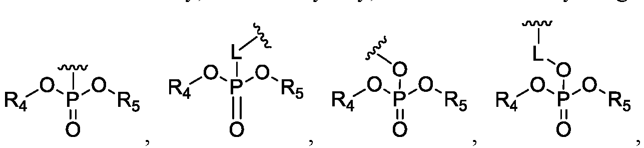 and -NR₄R₅, and R₃, R₄, and R₅ are independently selected from the group consisting of hydrogen, C6-C10 aryl, C1-C10 heteroaryl, C1-C6 chain alkyl, C3-C6 cycloalkyl, C2-C6 chain alkenyl, and C2-C6 alkynyl, and the aryl and heteroaryl are optionally substituted with halogen atom, hydroxyl, mercapto, amino, nitro, cyano, carboxyl, acyl, C1-C6 alkoxy, C6-C10 aryl, C1-C10 heteroaryl, C2-C10 heteroaliphatic group, C1-C6 alkyl, C3-C6 cycloalkyl, C2-C6 chain alkenyl, or C2-C6 alkynyl, and wherein optionally the substituents of at least two positions together form C3-C10 aliphatic ring, C2-C10 heteroalicyclic ring, C6-C10 aromatic ring, or C1-C10 heteroaromatic ring.

In some embodiments, the benzothiazole compound is represented by formula III: 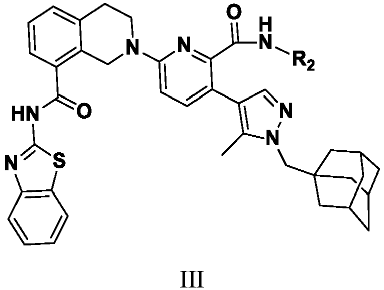 in formula III, R₂ is C1-C10 chain alkyl, C3-C8 cycloalkyl, C2-C8 chain alkenyl, C2-C8 alkynyl, C6-C20 aryl, C1-C20 heteroaryl, and C2-C20 heteroalicyclic group, wherein CH₂ in C1-C10 chain alkyl, C3-C8 cycloalkyl, C2-C8 chain alkenyl, and C2-C8 alkynyl can be replaced with one or more groups selected from the group consisting of -O-, -S-, -SO₂-, -C(O)-, and -NR₃-, and the C1-C10 chain alkyl, C3-C8 cycloalkyl, C2-C8 chain alkenyl, C2-C8 alkynyl, C6-C20 aryl, C1-C20 heteroaryl, and C2-C20 heteroalicyclic group are substituted with one or more substituents selected from the group consisting of halogen atom, cyano, nitro, C6-C20 aryl, C1-C20 heteroaryl, C1-C10 chain alkoxy, C6-C20 aryloxy, C2-C20 heteroalicyclic group, amino, hydroxyl, mercapto, 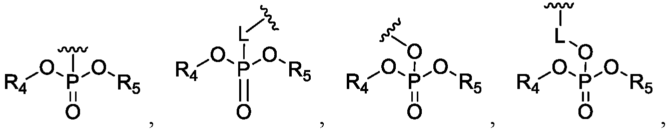 and - NR₄R₅; R₃, R₄, and R₅ are independently selected from the group consisting of hydrogen, C6-C10 aryl, C1-C10 heteroaryl, C1-C6 chain alkyl, C3-C6 cycloalkyl, C2-C6 chain alkenyl, and C2-C6 alkynyl, and the aryl and heteroaryl are optionally substituted with halogen atom, hydroxyl, mercapto, amino, nitro, cyano, carboxyl, acyl, C1-C6 alkoxy, C6-C10 aryl, C1-C10 heteroaryl, C2-C10 heteroalicyclic group, C1-C6 alkyl, C3-C6 cycloalkyl, C2-C6 chain alkenyl, or C2-C6 alkynyl, wherein optionally the substituents of at least two positions together form C3-C10 aliphatic ring, C2-C10 heteroalicyclic ring, C6-C10 aromatic ring, or C1-C10 heteroaromatic ring.

In some embodiments, the heteroalicyclic group or heteroalicyclic ring is selected from the group consisting of: substituted or unsubstituted piperidinyl, substituted or unsubstituted piperazinyl, substituted or unsubstituted morpholinyl, substituted or unsubstituted piperazin-2-one group, substituted or unsubstituted pyrrolidinyl, and substituted or unsubstituted imidazolinyl.

In some embodiments, R₁ and/or R₂ are selected from the group consisting of methyl, ethyl, propyl, isopropyl, methoxy, ethoxy, propoxy, isopropoxy, ethenyl, 6-membered aryl, 5-membered heteroaryl, 6-membered heteroaryl, 5-membered heterocyclyl, 6-membered heterocyclyl, 5-membered carbocyclyl, and 6-membered carbocyclyl, and each heteroaryl and each heterocyclyl contain 1 or 2 heteroatoms selected from the group consisting of N and O; and each group is independently optionally substituted with -F, -Cl, -Br, -I, -NH₂, -OH, -NO₂, carbonyl, -CH₂-OH, carboxyl, methyl, ethyl, propyl, isopropyl, methoxy, ethoxy, propoxy, and isopropoxy.

In some embodiments, R₁ and/or R₂ are selected from the group consisting of 6-membered aryl, 5-membered heteroaryl, 6-membered heteroaryl, 5-membered heterocyclyl, 6-membered heterocyclyl, 5-membered carbocyclyl, and 6-membered carbocyclyl, and each heteroaryl and each heterocyclyl contain 1 or 2 heteroatoms selected from the group consisting of N and O; and each group is independently optionally substituted with -F, -Cl, -Br, -I, -NH₂, -OH, -NO₂, carbonyl, =O, -CH₂-OH, carboxyl, methyl, ethyl, propyl, isopropyl, methoxy, ethoxy, propoxy, or isopropoxy.

In some embodiments, R₁ and R₂ together with the N atom to which they are connected, form 6-membered aryl, 5-membered heteroaryl, 6-membered heteroaryl, 5-membered heterocyclyl, 6-membered heterocyclyl, 5-membered carbocyclyl, or 6-membered carbocyclyl, and each heteroaryl and each heterocyclyl contain 1 or 2 heteroatoms selected from the group consisting of N and O; and each group is independently optionally substituted with -F, -Cl, -Br, -I, -NH₂, -OH, -NO₂, carbonyl, =O, -CH₂-OH, carboxyl, methyl, ethyl, propyl, isopropyl, methoxy ethoxy, propoxy, or isopropoxy.

In some embodiments, R₁ and R₂ together with the N atom to which they are connected, form a C2-C20 heteroalicyclic group.

In some embodiments, the C2-C20 heteroalicyclic group is selected from C4-C20 heteroalicyclic group.

In some embodiments, the C4-C20 heteroalicyclic group is selected from the group consisting of: substituted or unsubstituted morpholinyl, substituted or unsubstituted piperidinyl, and substituted or unsubstituted piperazinyl.

In some embodiments, the C4-C20 heteroalicyclic group is selected from the group consisting of the following groups: 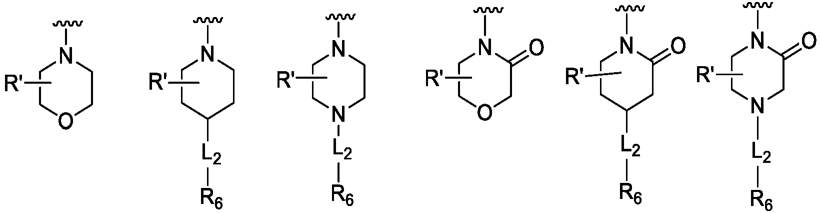 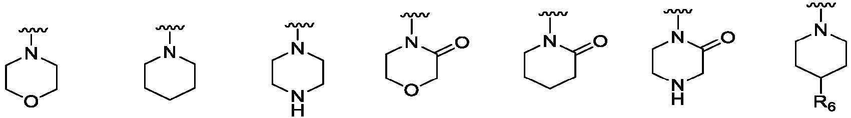 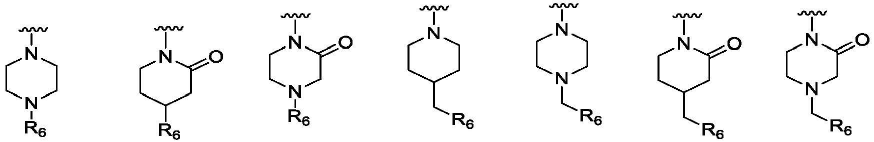 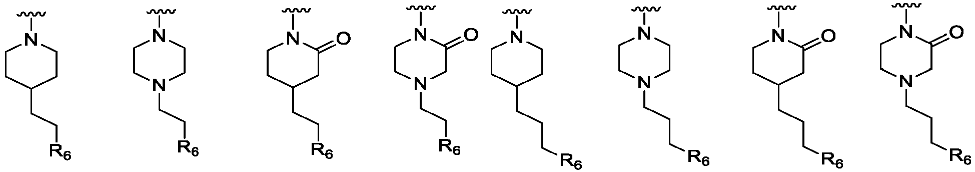 ,
R' each independently represents no substituent, single substituent, or multiple substituents, and each substituent is independently selected from the group consisting of deuterium, hydroxyl, halogen, NH₂, carboxyl (-COOH), 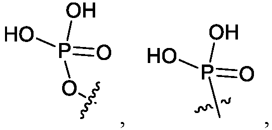 C1-C6 chain alkyl, halogen-substituted C1-C6 chain alkyl, hydroxyl-substituted chain C1-C6 alkyl, amino-substituted C1-C6 chain alkyl, morpholine-substituted C1-C6 chain alkyl, -COO-C1-C6 chain alkyl, cyano, C1-C6 chain alkoxy, C3-C6 cycloalkyl, halogen-substituted C3-C6 cycloalkyl, hydroxyl-substituted C3-C6 cycloalkyl, phenyl, and benzyl;
L₂ is absent, or C1-C6 alkylene, or-C1-C6 alkylene substituted with halogen, hydroxyl, or C1-C6 alkoxy, preferably methylene, ethylene, or propylene;
R₆ is H, deuterium, halogen, hydroxyl, NH₂, carboxyl (-COOH), -CONH₂, sulfonic acid group (-SO₃H), -SO₂-C1-C6 chain alkyl, 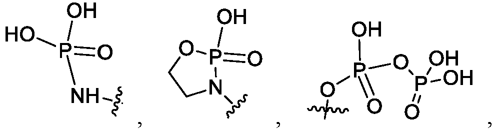 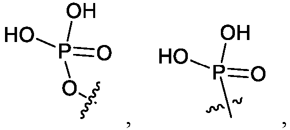 C1-C6 chain alkyl, halogen-substituted C1-C6 chain alkyl, morpholine-substituted C1-C6 chain alkyl, -COO-C1-C6 chain alkyl, cyano, C1-C6 chain alkoxy, hydroxyl-substituted C1-C6 chain alkyl, amino-substituted C1-C6 chain alkyl, C3-C6 cycloalkyl, halogen-substituted C3-C6 cycloalkyl, hydroxyl-substituted C3-C6 cycloalkyl, phenyl, or benzyl.

In some embodiments, the compound has the structure represented by formula I-1, formula I-2, formula I-3, formula I-4, or formula I-5, 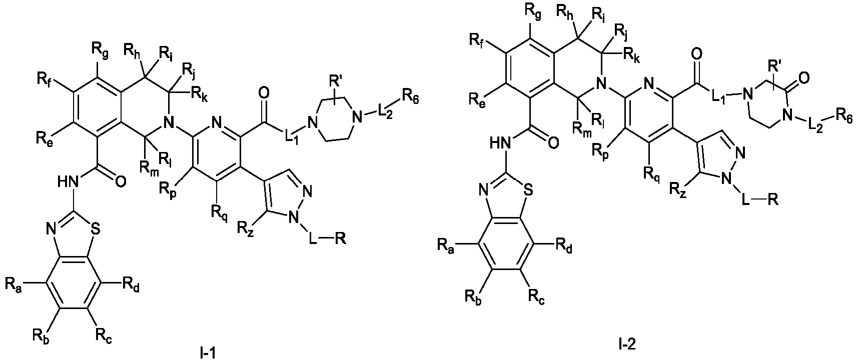 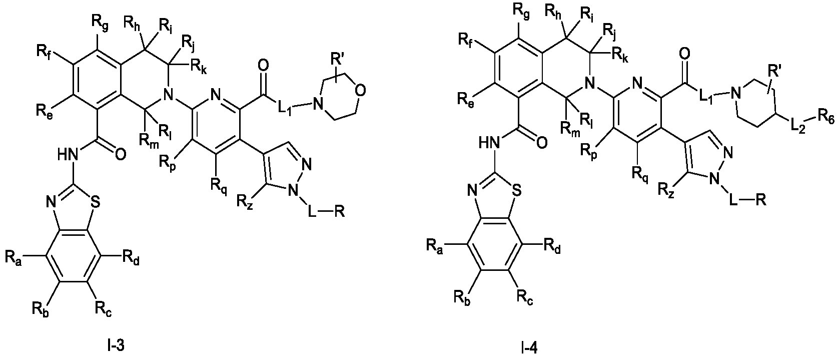 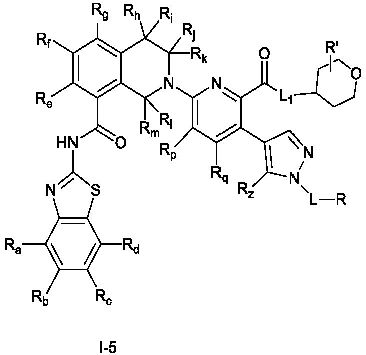
L₁ is absent or -NHL₃-, L₃ is absent or C1-C6 alkylene or C1-C6 alkylene substituted with halogen, hydroxyl, or C1-C6 alkoxy, preferably methylene, ethylene, or propylene;
L₂ is absent, or C1-C6 alkylene, or C1-C6 alkylene substituted with halogen, hydroxyl, or C1-C6 alkoxy, preferably methylene, ethylene, or propylene;
R₆ is H, deuterium, halogen, hydroxyl, NH₂, carboxyl (-COOH), -CONH₂, sulfonic acid group (-SO₃H), -SO₂-C1-C6 chain alkyl, 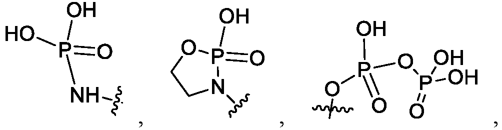 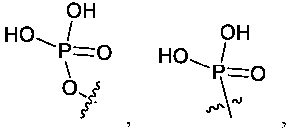 C1-C6 chain alkyl, halogen-substituted C1-C6 chain alkyl, morpholine-substituted C1-C6 chain alkyl, -COO-C1-C6 chain alkyl, cyano, C1-C6 chain alkoxy, hydroxyl-substituted C1-C6 chain alkyl, amino-substituted C1-C6 chain alkyl, C3-C6 cycloalkyl, halogen-substituted C3-C6 cycloalkyl, hydroxyl-substituted C3-C6 cycloalkyl, phenyl, or benzyl;
R' each independently represents no substituent, single substituent, or multiple substituents, and each substituent is independently selected from the group consisting of H, deuterium, halogen, hydroxyl, NH₂, carboxyl (-COOH), -CONH₂, sulfonic acid group (-SO₃H), -SO₂-C1-C6 chain alkyl, 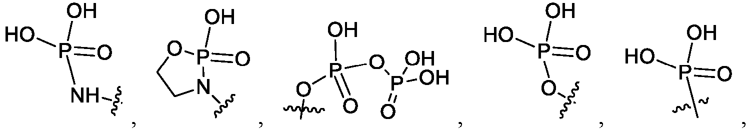 C1-C6 chain alkyl, halogen-substituted C1-C6 chain alkyl, morpholine-substituted C1-C6 chain alkyl, -COO-C1- C6 chain alkyl, cyano, C1-C6 chain alkoxy, hydroxyl-substituted C1-C6 chain alkyl, amino-substituted C1-C6 chain alkyl, C3-C6 cycloalkyl, halogen-substituted C3-C6 cycloalkyl, hydroxyl-substituted C3-C6 cycloalkyl, phenyl, or benzyl;
the definitions to the other symbols are the same as those in formula I.

In some embodiments, R' each independently represents no substituent, single substituent, or multiple substituents, and each substituent is independently selected from the group consisting of deuterium, hydroxyl, halogen, NH₂, carboxyl, 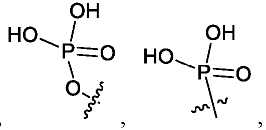 C1-C6 chain alkyl, halogen-substituted C1-C6 chain alkyl, hydroxyl-substituted C1-C6 chain alkyl, C3-C6 cycloalkyl, halogen-substituted C3-C6 cycloalkyl, hydroxyl-substituted C3-C6 cycloalkyl, phenyl, and benzyl.

In some embodiments, Rₐ, R_{b}, R_{c}, and R_{d} are each independently selected from the group consisting of hydrogen and C1-C6 alkyl. In some embodiments, Rₑ, R_{f}, and R_{g} are each independently selected from the group consisting of hydrogen and C1-C6 alkyl. In some embodiments, Rₕ, Rᵢ, Rⱼ, Rₖ, Rₗ, and Rₘ are each independently selected from the group consisting of hydrogen and C1-C6 alkyl. In some embodiments, Rₚ, R_{q}, and R_{z} are each independently selected from the group consisting of hydrogen and C1-C6 alkyl. In some embodiments, Rₐ, R_{b}, R_{c}, R_{d}, Rₑ, R_{f}, R_{g}, Rₕ, Rᵢ, Rⱼ, Rₖ, Rₗ, Rₘ, Rₚ, R_{q}, and R_{z} are all hydrogen.

In some embodiments, L is methylene.

In some embodiments, R_{z} is methyl.

In some embodiments, R is

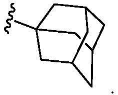

In some embodiments, L₁ is absent.

In some embodiments, R₆ is H, halogen, hydroxyl, NH₂, carboxyl, 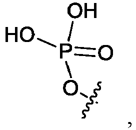 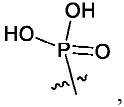 C1-C3 chain alkyl, halogen-substituted C1-C3 chain alkyl, or hydroxyl-substituted C1-C3 chain alkyl.

In some embodiments, R₆ is

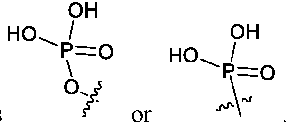

In more specific embodiments, the benzothiazole compounds represented by formula I are selected from the group consisting of the following compounds:

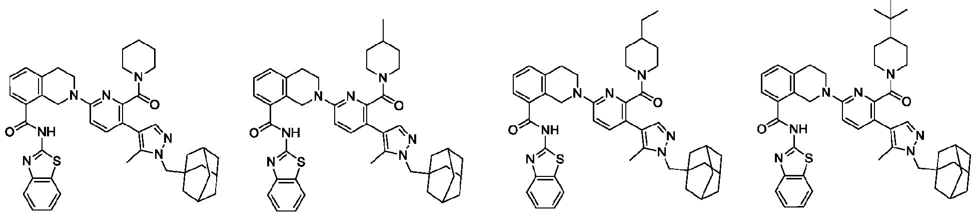

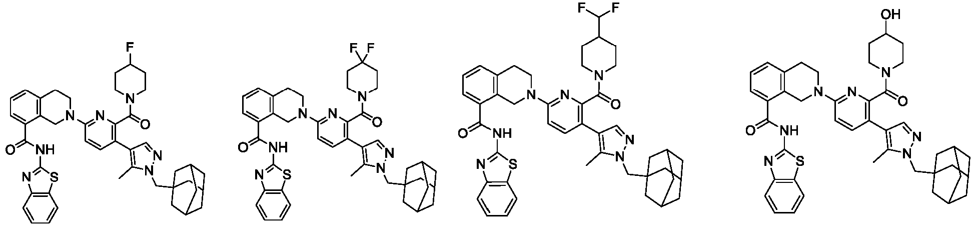

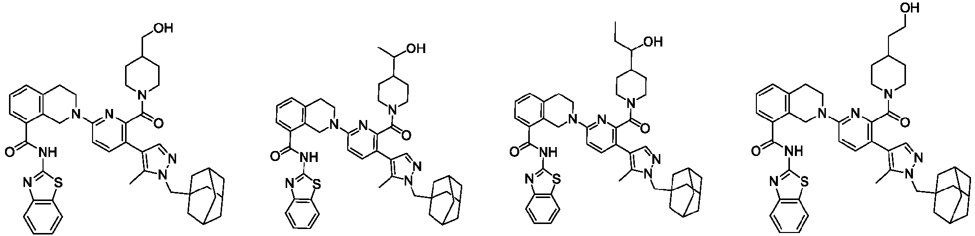

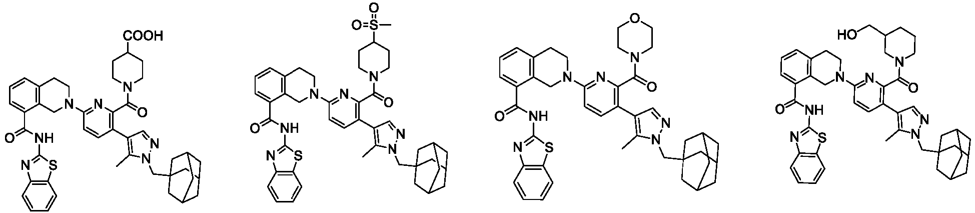

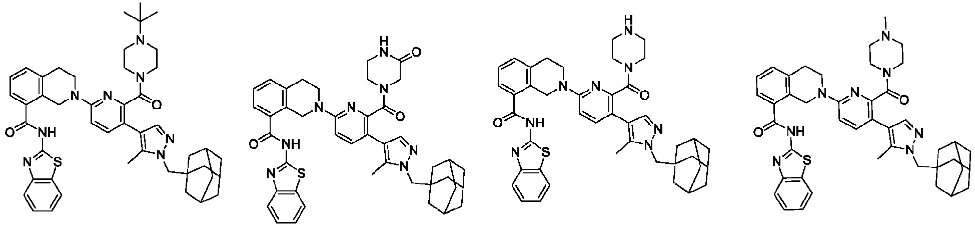

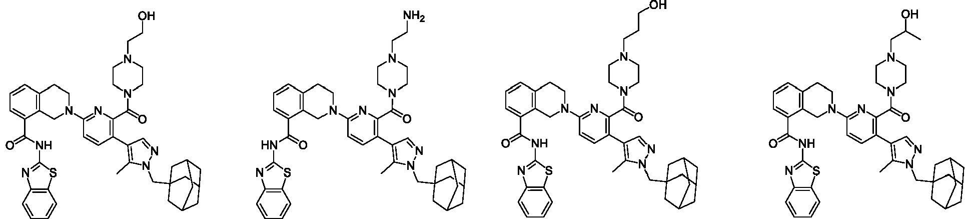

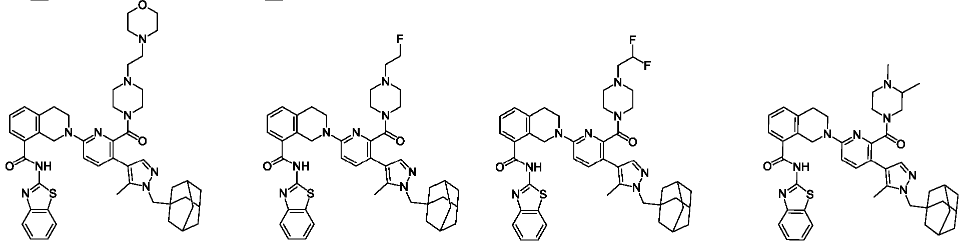

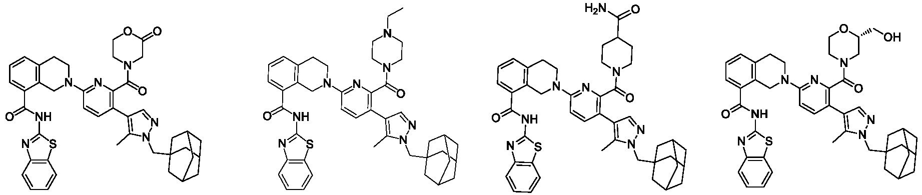

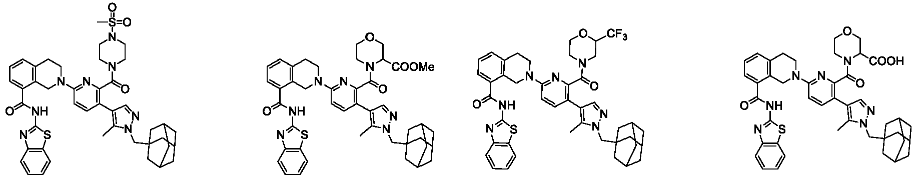

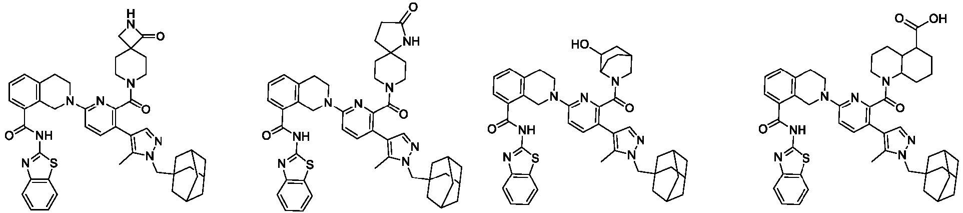

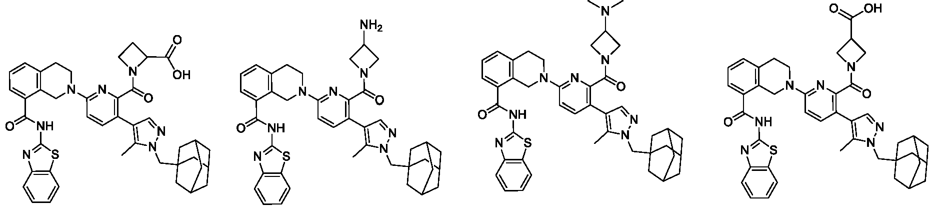

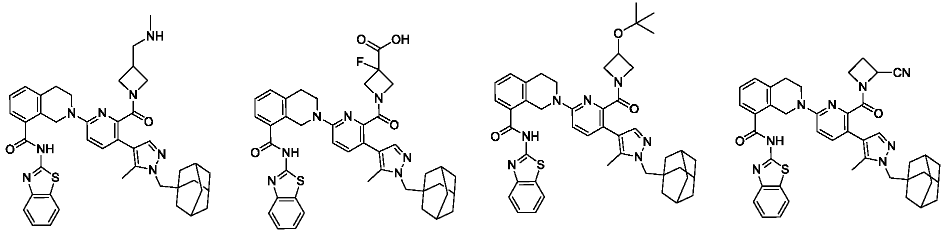

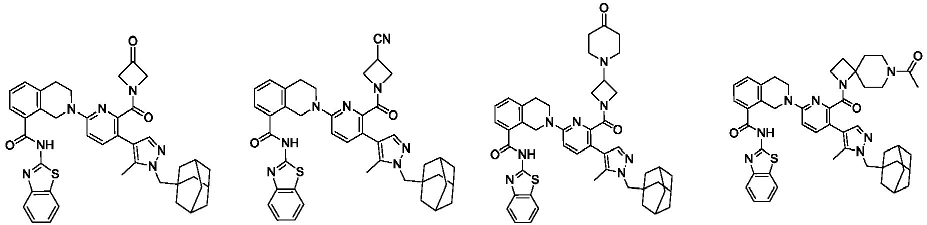

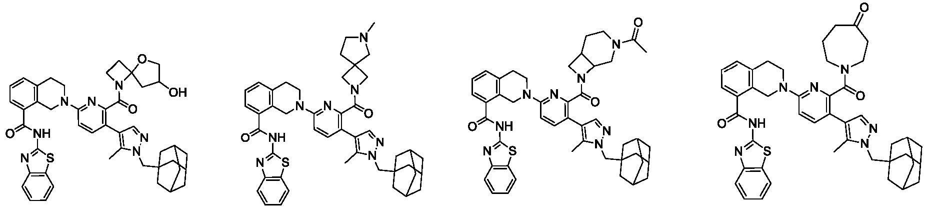

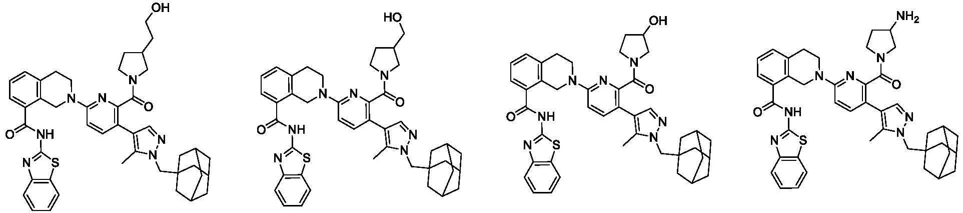

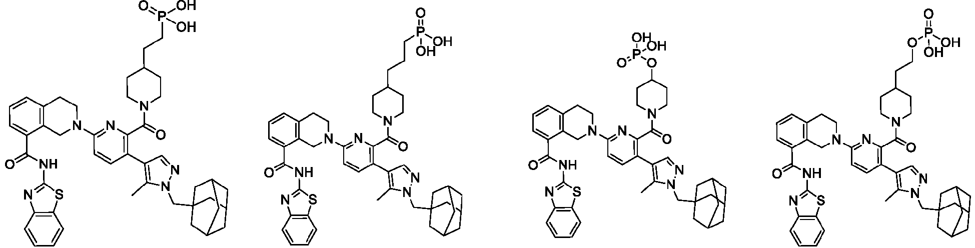

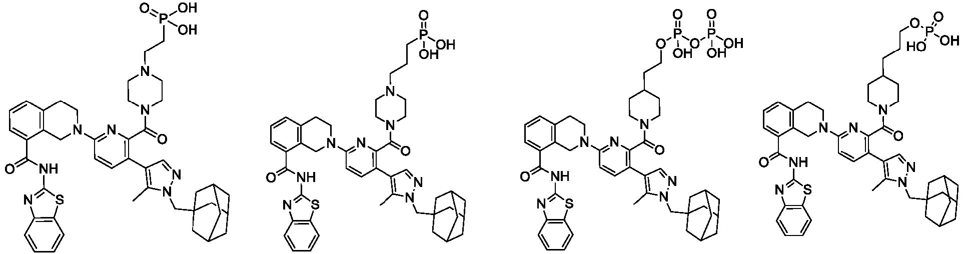

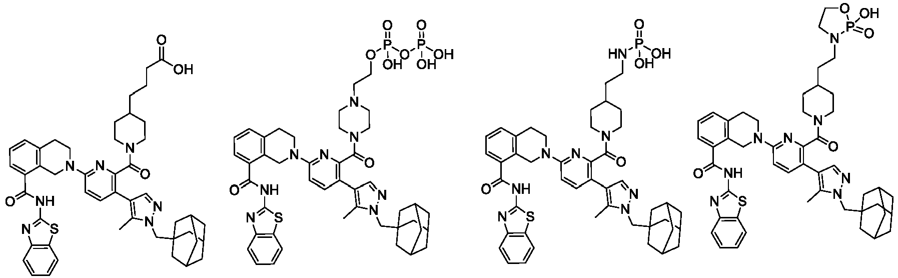

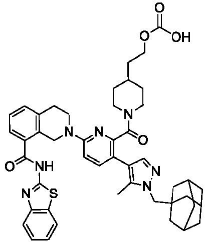

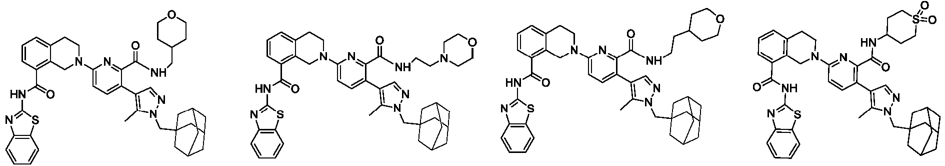

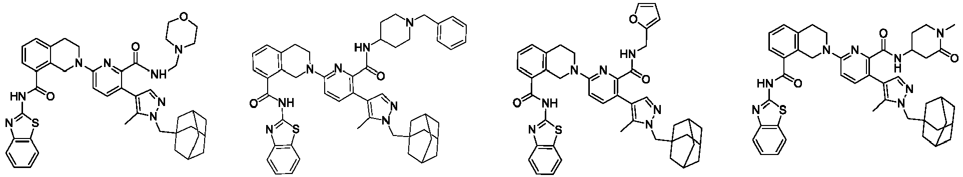

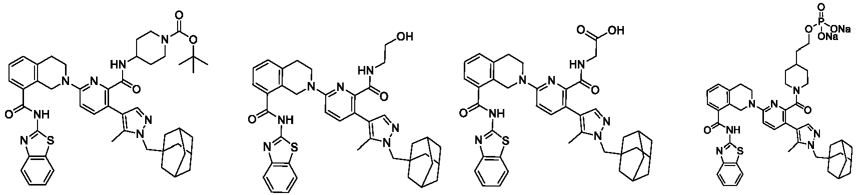

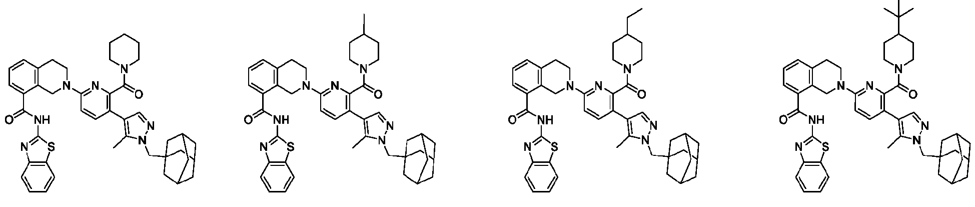

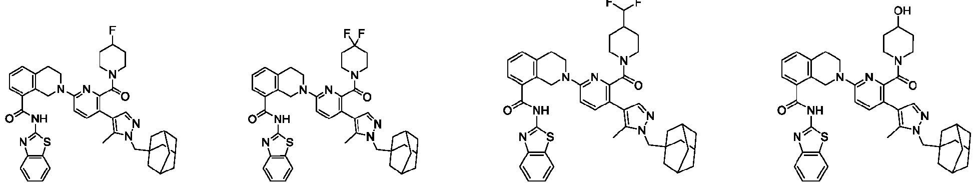

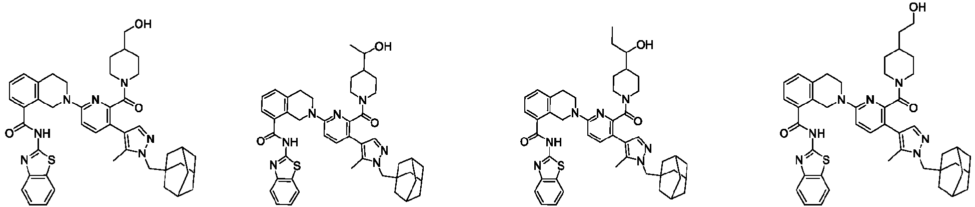

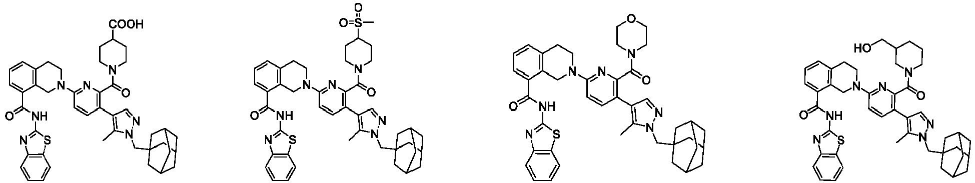

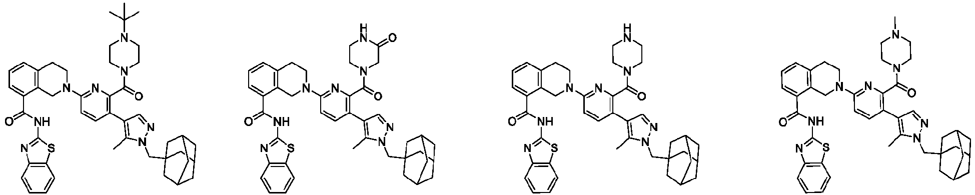

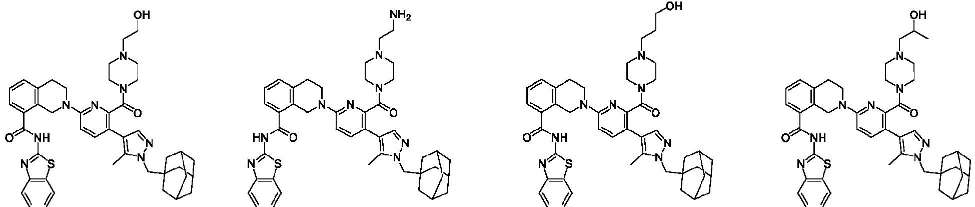

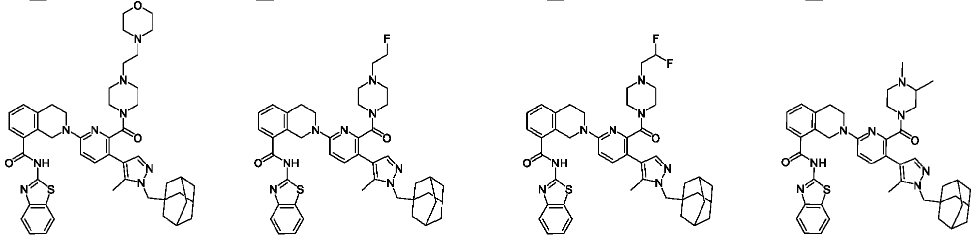

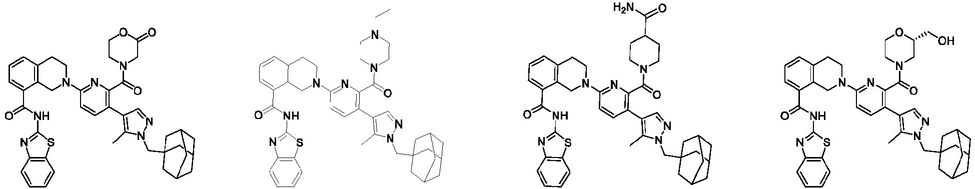

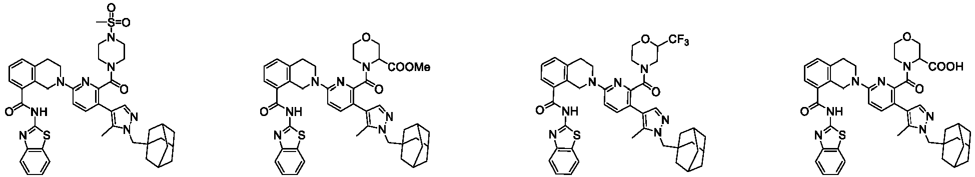

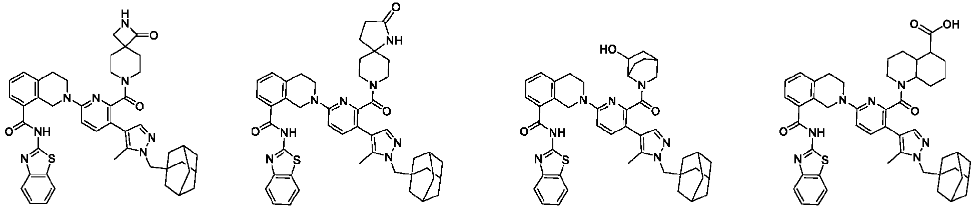

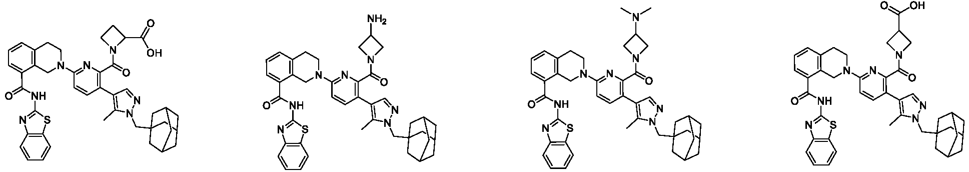

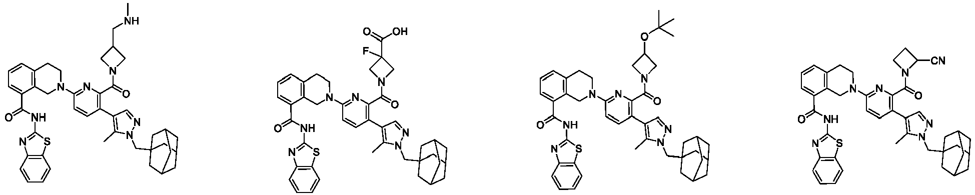

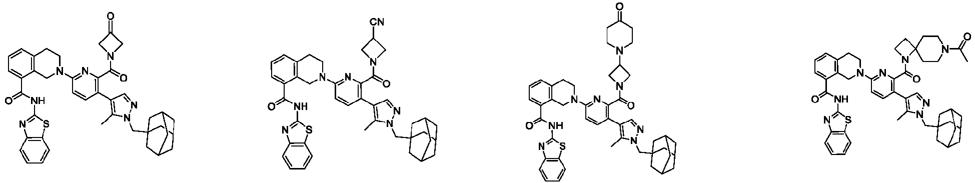

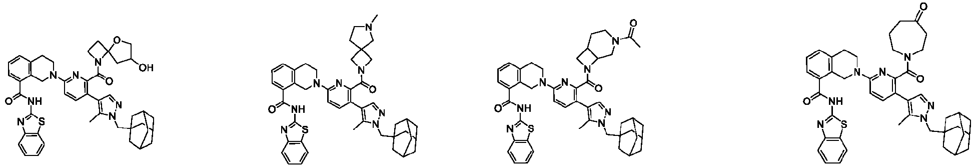

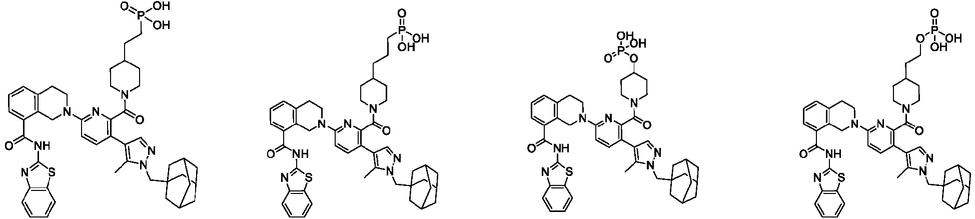

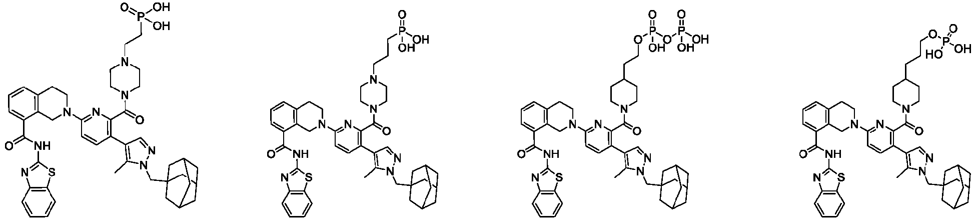

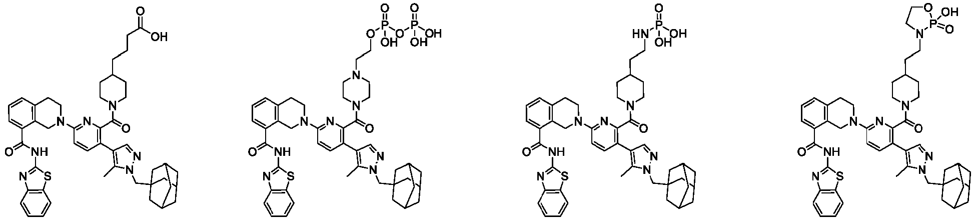

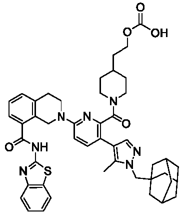

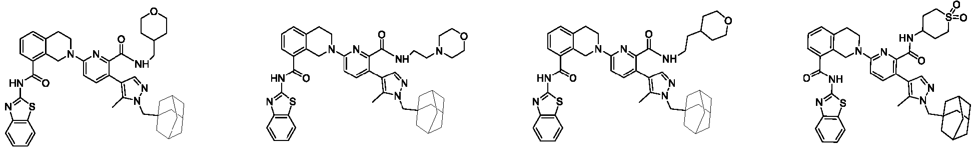

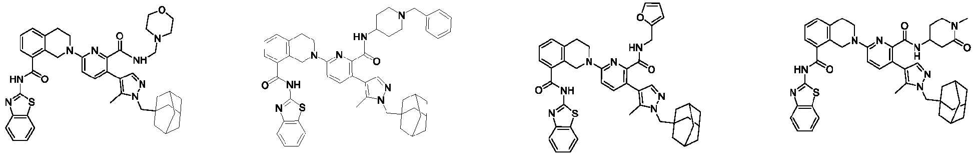

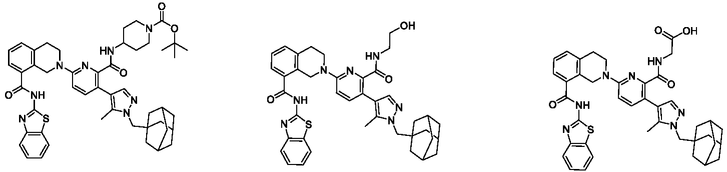

In a second aspect, the present application provides a pharmaceutical composition, comprising the compound or a pharmaceutically acceptable salt, solvate, hydrate, polymorph, co-crystal, tautomer, stereoisomer, isotope labeled compound, or prodrug thereof in the first aspect, and a pharmaceutically acceptable excipient.

In a third aspect, the present application provides a method for preventing or treating age-related disease, comprising administering to a subject in need a therapeutically effective amount of the compound or a pharmaceutically acceptable salt, solvate, hydrate, polymorph, co-crystal, tautomer, stereoisomer or prodrug thereof in the first aspect or the pharmaceutical composition in the second aspect. Specifically, the disease is selected from diseases related to the accumulation of senescent cells, and the disease is preferably one or more selected from the group consisting of idiopathic pulmonary fibrosis, pulmonary fibrosis, hepatic fibrosis, renal fibrosis, inflammation and tissue fibrosis and atrophy of upper respiratory tract and lungs caused by viruses, cystic fibrosis, myelofibrosis, myocardial fibrosis, cutaneous fibrosis, interstitial lung disease, fibrotic pancreatitis, retinopathy of prematurity, macular degeneration, diabetic macular edema, diabetic retinopathy, age-related macular degeneration, wet age-related macular degeneration, dry age-related macular degeneration, glaucoma, sickle cell retinopathy, ischemic arteritis neuropathy, keratitis sicca, Fuch's corneal dystrophy, presbyopia, cataract, degenerative vitreous disorder, including vitreomacular traction syndrome, macular hole, retinal tear, retinal detachment, and proliferative vitreoretinopathy, osteoarthritis, disc herniation, osteoporosis, Alzheimer's disease, Parkinson's disease, atherosclerosis, chronic obstructive pulmonary disease, diabetes, diabetic nephropathy, scar, superficial scar or flat scars, rope scar or contracted scar, webbed scar, depressed scar, atrophic scar, bridge scar and pedunculated scar, hypertrophic scar, keloid, scar cancer, scleroderma, morphea, linear scleroderma, guttate scleroderma, acroscleroderma, diffuse scleroderma, CREST syndrome, acute coronary syndrome, myocardial infarction, stroke, hypertension, obesity, lipodystrophy, coronary artery disease, cerebrovascular disease, periodontal disease, cancer treatment-related disabilities such as atrophy and fibrosis in various tissues, brain and heart damage and treatment-related myelodysplastic syndrome, promyelocytic syndrome, ataxia telangiectasia, Fanconi anemia, Friedreich's ataxia, congenital dyskeratosis, aplastic anemia, aneurysm, inflammatory bowel disease, lipoatrophy, renal transplant failure, sarcopenia, wound healing, alopecia, cardiomyocyte hypertrophy, glomerulosclerosis, and cancer.

In a fourth aspect, the present application provides use of the compound or a pharmaceutically acceptable salt, solvate, hydrate, polymorph, co-crystal, tautomer, stereoisomer, isotope labeled compound, or prodrug thereof in the first aspect or the pharmaceutical composition in the second aspect in the preparation of a medicament for preventing or treating a disease related to aging. Specifically, the disease is selected from diseases related to the accumulation of senescent cells, and the disease is preferably one or more selected from the group consisting of idiopathic pulmonary fibrosis, pulmonary fibrosis, hepatic fibrosis, renal fibrosis, inflammation and tissue fibrosis and atrophy of upper respiratory tract and lungs caused by viruses, cystic fibrosis, myelofibrosis, myocardial fibrosis, cutaneous fibrosis, interstitial lung disease, fibrotic pancreatitis, retinopathy of prematurity, macular degeneration, diabetic macular edema, diabetic retinopathy, age-related macular degeneration, wet age-related macular degeneration, dry age-related macular degeneration, glaucoma, sickle cell retinopathy, ischemic arteritis neuropathy, keratitis sicca, Fuch's corneal dystrophy, presbyopia, cataract, degenerative vitreous disorder, including vitreomacular traction syndrome, macular hole, retinal tear, retinal detachment, and proliferative vitreoretinopathy, osteoarthritis, disc herniation, osteoporosis, Alzheimer's disease, Parkinson's disease, atherosclerosis, chronic obstructive pulmonary disease, diabetes, diabetic nephropathy, scar, superficial scar or flat scars, rope scar or contracted scar, webbed scar, depressed scar, atrophic scar, bridge scar and pedunculated scar, hypertrophic scar, keloid, scar cancer, scleroderma, morphea, linear scleroderma, guttate scleroderma, acroscleroderma, diffuse scleroderma, CREST syndrome, acute coronary syndrome, myocardial infarction, stroke, hypertension, obesity, lipodystrophy, coronary artery disease, cerebrovascular disease, periodontal disease, cancer treatment-related disabilities such as atrophy and fibrosis in various tissues, brain and heart damage and treatment-related myelodysplastic syndrome, promyelocytic syndrome, ataxia telangiectasia, Fanconi anemia, Friedreich's ataxia, congenital dyskeratosis, aplastic anemia, aneurysm, inflammatory bowel disease, lipoatrophy, renal transplant failure, sarcopenia, wound healing, alopecia, cardiomyocyte hypertrophy, glomerulosclerosis, and cancer.

The novel benzothiazole compounds provided by the present application have improved physicochemical properties and safety, low platelet toxicity and better solubility compared with A-1331852.

### BRIEF DESCRIPTION OF DRAWINGS

Figure 1 shows the test results of platelet toxicity of compounds in mice.
Figure 2 shows the test results of the killing effect of compounds on different types of normal and senescent cells.
Figure 3 shows that the intravitreal (IVT) administration of RX001-RX006 and RX301 resulted in statistically significant improvements in avascular zone and angiogenesis.
Figure 4 shows that the intravitreal (IVT) administration of RX001, RX002, RX003, and RX301 in a diabetic macular model resulted in improved vascular permeability in both the retinal and choroidal vascular leakage at this dose level.
Figure 5 shows that the intravitreal (IVT) administration of RX001, RX002, RX003, and RX301 resulted in statistically significant improvements in fluorescence intensity indicators in a CNV macular model.
Figure 6 shows that the administration of RX001, RX002, RX003, and RX301 via intraperitoneal (IP) injection resulted in statistically significant improvements in WBC, HYP and pathology scores in a pulmonary fibrosis (IPF) model.
Figure 7 shows that the administration of RX001, RX002, RX003, and RX301 via intra-articular injection resulted in statistically significant improvements in plantar mechanical pain threshold and pathology scores of safranin fast green staining in an osteoarthritis (OA) model.
Figure 8 shows that the administration of RX001, RX002, RX003, and RX301 via intraperitoneal injection resulted in statistically significant improvements in Aβ42 pathogenic protein indicators in an Alzheimer's disease (AD) mouse model.
Figure 9 shows that the administration of RX001 and RX301 via intraperitoneal (IP) injection resulted in statistically significant improvements in serum aspartate aminotransferase (AST) and alanine aminotransferase (ALT) levels and pathology scores in a liver fibrosis model.
Figure 10 shows that the administration of RX001, RX002, RX003, and RX301 by applying resulted in statistically significant improvements in epidermal thickness scores of the skins in a naturally aging mouse model.
Figure 11 shows that the administration of RX001 and RX301 via intraperitoneal (IP) injection resulted in statistically significant improvements in serum creatinine (Cr) and urea nitrogen (BUN) levels and pathology in a renal fibrosis model.
Figure 12 shows that the administration of RX001 and RX301 resulted in a statistically significant improvement in scar hyperplasia index in a rabbit ear hypertrophic scar model.

### DETAILED DESCRIPTION OF THE INVENTION

The technical solutions of the present invention will be described in detail in combination with the following examples and figures, the examples are carried out on the premise that the present invention is a technical solution, and detailed embodiments and processes are given, but the embodiments provided herein are exemplary and are intended to be used for explaining the present invention, and are not to be construed as a limitation of the present invention. The conditions and methods not specified in the following examples are carried out conventionally.

### Definition

The term "alkyl" refers to an aliphatic hydrocarbon group, which may be branched or linear alky. Depending on the structure, the alkyl can be monovalent group or bivalent group (i.e., alkylene). In the present invention, the alkyl is preferably an alkyl having 1 to 8 carbon atoms, more preferably a "lower alkyl" having 1 to 6 carbon atoms, and even more preferably an alkyl having 1 to 4 carbon atoms. Typical alkyl includes, but is not limited to, methyl, ethyl, propyl, butyl, pentyl, hexyl, and the like. It should be understood that, the "alkyl" as used herein includes all possible configurations and conformations of the alkyl. For example, the "propyl" mentioned herein includes n-propyl and isopropyl, and the "butyl" includes n-butyl, isobutyl, and tert-butyl, and the "pentyl" includes n-pentyl, isopentyl, neopentyl, tert-pentyl, and pentyl-3-yl, and the like.

The term "alkoxy" refers to -O-alkyl, wherein the alkyl is as defined herein. Typical alkoxy includes, but is not limited to, methoxy, ethoxy, propoxy, butoxy, pentyloxy, hexyloxy, and the like.

The term "cycloalkyl" refers to monocyclic or polycyclic group containing only carbon and hydrogen. Cycloalkyl includes a group having 3 to 12 ring atoms. Depending on the structure, a cycloalkyl can be monovalent group or bivalent group (e.g., cycloalkylene). In the present invention, the cycloalkyl is preferably a cycloalkyl having 3 to 8 carbon atoms, and more preferably a "lower cycloalkyl" having 3 to 6 carbon atoms. Examples of cycloalkyl include, but are not limited to, cyclopropyl, cyclobutyl, cyclopentyl, cyclohexyl, cycloheptyl, cyclooctyl, cyclopentenyl, cyclohexenyl, cycloheptenyl, and adamantyl.

The term "aryl" refers to an aromatic ring in which each of the atoms making up the ring is a carbon atom. Aromatic ring can be composed of five, six, seven, eight, nine, or more than nine atoms. Aryl may be optionally substituted. Examples of aryl include, but are not limited to, phenyl, naphthyl, phenanthryl, anthracenyl, fluorenyl, and indenyl. Depending on the structure, an aryl may be monovalent group or bivalent group (i.e., arylene).

The term "heteroaryl" refers to an aromatic group containing one or more ring heteroatoms selected from the group consisting of nitrogen, oxygen, and sulfur. The N-containing "heteroaryl" moiety refers to an aromatic group in which at least one skeleton atom in the ring is a nitrogen atom. Depending on the structure, a heteroaryl can be monovalent group or bivalent group (i.e., heteroarylene). Examples of heteroaryl include, but are not limited to, pyridyl, imidazolyl, pyrimidinyl, pyrazolyl, triazolyl, pyrazinyl, tetrazolyl, furyl, thienyl, isoxazolyl, thiazolyl, oxazolyl, isothiazolyl, pyrrolyl, quinolyl, isoquinolyl, indolyl, benzimidazolyl, benzofuranyl, indazolyl, indolizinyl, phthalazinyl, pyridazinyl, isoindolyl, pteridinyl, purinyl, oxadiazolyl, thiadiazolyl, furazanyl, benzofuranyl, benzothienyl, benzothiazolyl, benzoxazolyl, quinazolinyl, naphthyridinyl, furopyridyl, and the like.

The term "alicyclic group" or "cycloalkyl" used herein refers to a non-aromatic ring formed by three or more carbon atoms, wherein the bond between two adjacent carbon atoms in the ring can be single bonds, double bonds, or triple bonds. The number of rings can be one or more. Non-limiting examples include cyclopropyl, cyclobutyl, cyclopentyl, cyclohexyl, cycloheptyl, cyclohexenyl, cyclopentenyl, cyclohexadienyl, and the like.

The term "heterocycloalkyl" or "heteroalicyclic group" or "heteroalicycle" used herein refers to a non-aromatic ring in which one or more atoms constituting the ring are heteroatoms selected from the group consisting of nitrogen, oxygen, and sulfur. Heterocycloalkyl may be composed of three, four, five, six, seven, eight, nine or more than nine atoms. Heterocycloalkyl may be optionally substituted. Examples of heteroalicyclic group include, but are not limited to, lactam, lactone, cyclicimine, cyclicthioimine, cyclic carbamate, tetrahydrothiopyran, 4H-pyran, tetrahydropyran, piperidine, 1,3-dioxin, 1,3-dioxane, 1,4-dioxin, 1,4-dioxane, piperazine, 1,3-oxathiane, 1,4-oxathiine, 1,4-oxathiane, tetrahydro-1,4-thiazine, 2H-1,2-oxazine, maleimide, succinimide, barbituric acid, thiobarbituric acid, dioxopiperazine, hydantoin, dihydrouracil, morpholine, trioxane, hexahydro-1,3,5-triazine, tetrahydrothiophene, tetrahydrofuran, pyrroline, pyrrolidine, imidazolidine, pyrrolidone, pyrazoline, pyrazolidine, imidazoline, imidazolidine, 1,3-dioxolene, 1,3-dioxolane, 1,3-dithiolene, 1,3-dithiolane, isoxazoline, isoxazolidine, oxazoline, oxazolidine, oxazolidinone, thiazoline, thiazolidine, and 1,3-oxathiolane. Depending on the structure, the heteroalicyclic group may be monovalent group or bivalent group (i.e., heterocycloalkylene).

The term "halo" or "halogen" used herein refers to fluorine, chlorine, bromine, and iodine.

The term "carbonyl" used herein refers to an organic functional group (C=O) formed by connecting carbon and oxygen, through a double bond.

The term "optional" used herein refers to that one or more of subsequent events may occur or may not occur, and includes both events that occur and events that do not occur.

The salts formed by the compound in the present invention are also within the scope of the present invention. Unless otherwise specified, the compound in the present invention is understood as including its salts. The term "salt" used herein refers to an acidic or basic salt formed from inorganic or organic acid and base. Furthermore, when the compound of the present invention contains a basic fragment, it includes but is not limited to pyridine or imidazole, and when it contains an acidic fragment, it includes but is not limited to carboxylic acid, and the zwitterion ("inner salt") that may be formed is included in the scope of the term "salt". Pharmaceutically acceptable (i.e., nontoxic and physiologically acceptable) salts are preferred, although other salts are also useful, for example, in isolation or purification steps during preparation. The compounds of the present invention may form salts, for example, obtained from the reaction of the compound I with a certain amount of, for example, an equivalent amount of acid or base, salting out in a medium, or freeze-drying in an aqueous solution.

The basic fragments contained in the compound of the present invention include but are not limited to amines, pyridine or imidazole ring, which may form salt with organic or inorganic acid. Typical acids that can form salts include acetates (e.g., acetic acid or trihaloacetic acids, e.g. trifluoroacetic acid), adipate, alginate, ascorbate, aspartate, benzoate, benzenesulfonate, hydrosulfate, borate, butyrate, citrate, camphorate, camphorsulfonate, cyclopentanepropionate, diglycolate, dodecyl sulfate, ethanesulfonate, fumarate, gluceptate, glycerophosphate, hemisulfate, heptylate, hexanoate, hydrochloride, hydrobromide, hydroiodide, hydroxy-ethanesulfonate (e.g., 2-hydroxy-ethanesulfonate), lactate, malate, methanesulfonate, naphthalenesulfonate (e.g., 2-naphthalenesulfonate), niacinate, nitrate, oxalate, pectinate, persulfate, phenylpropionate (e.g., 3-phenylpropionate), phosphate, picrate, neopentanoate, propionate, salicylate, succinate, sulfate (e.g., formed with sulfuric acid), sulfonate, tartrate, thiocyanate, toluenesulfonate such as p-toluenesulfonate, dodecanoate, and the like.

The acidic fragments contained in the compound of the present invention include but are not limited to carboxylic acid, which may form salt with various organic or inorganic bases. Typical salts formed with base include ammonium salt, alkali metal salt such as sodium, lithium, and potassium salts, alkaline earth metal salt such as calcium and magnesium salt, and salts formed with organic base (such as organic amine), such as benzathine, dicyclohexylamine, hypamine (salt with N,N-bis(dehydroabietyl)ethylenediamine), N-methyl-D-glucamine, *N-methyl-D-*glucamide, tert-butylamine, and salts formed with amino acids such as arginine and lysine, and the like. Basic nitrogen-containing group can be combined with halide quaternary ammonium salts, such as small molecule alkyl halide (e.g. chloride, bromide and iodide of methyl, ethyl, propyl and butyl), dialkyl sulfate (e.g. dimethyl sulfate, diethyl sulfate, dibutyl ester and dipentyl ester), long-chain halide (e.g. chloride, bromides and iodide of decyl, dodecyl, tetradecyl and tetradecyl), aralkyl halide (e.g. benzyl and phenyl bromides), and the like.

Prodrug and solvate of the compounds of the present invention are also within the scope of the invention. The term "prodrug" used herein refers to a compound that can be converted to produce the compound, salt, or solvate of the present invention undergoing chemical transformation by metabolic or chemical processes when treating related diseases. "Solvate" refers to a solvent addition form containing a stoichiometric or non-stoichiometric amount of solvent. Some compounds tend to trap a fixed molar ratio of solvent molecules in the crystalline solid state, thereby forming a solvate. If the solvent is water, the solvate formed is a hydrate; if the solvent is alcohol, the solvate formed is an alcoholate. A hydrate is formed by the combination of one or more water molecules with a molecule of substance, in which the water retains its molecular state H₂O. Non-limiting examples of solvate include ethanol solvate, acetone solvate, and the like.

The compound, salt or solvate of the present invention may exist in tautomeric forms (e.g. amide and imine ether). All such tautomers are parts of the present invention.

All stereoisomers of the compounds (e.g., those that may exist as asymmetric carbon atoms due to various substitutions), including their enantiomeric and diastereomeric forms, are contemplated by the present invention. The independent stereoisomers of the compounds of the present invention may not be coexist with other isomers (e.g. as a pure or substantially pure optical isomer with a special activity), or they may also be mixtures, such as racemates, or mixtures with all other stereoisomers or portions thereof. The chiral center of the present invention has S or R configuration, as defined by the recommendation of the International Union of Pure and Applied Chemistry (IUPAC) in 1974. The racemic form can be resolved by physical methods, such as stepwise crystallization, separation of crystals by derivatization into diastereomers, or separation by chiral column chromatography. Individual optical isomer can be obtained from the racemate by suitable methods, including but not limited to conventional methods, such as forming salt with an optically active acid, followed by recrystallisation.

The compounds in the present invention, which are obtained by preparation, separation, and purification in sequence, have a weight content equal to or greater than 90% or more, for example, equal to or greater than 95%, equal to or greater than 99% ("highly pure" compound), as listed in the description. Such "highly pure" compound of the present invention herein is also included as a part of the invention.

All isomeric forms of the compounds of the present invention are included within the scope, including mixtures, pure form or highly pure form. The definition of compounds in the present invention includes both cis (Z) and trans (E) isomers of alkenes, as well as cis and trans isomers of carbocyclic and heterocyclic rings.

Throughout the description, groups and substituents may be selected to provide stable fragments and compounds.

Definitions of specific functional groups and chemical terms are detailed below. For the purposes of the present invention, the chemical elements are defined in Periodic Table of the Elements, CAS version, Handbook of Chemistry and Physicsm, 75th Ed. Definitions of specific functional groups are also described therein. In addition, the basic principles of organic chemistry and specific functional groups and reactivities are described in "Organic Chemistry", Thomas Sorrell, University Science Books, Sausalito: 1999, the entire content of which is incorporated by reference.

Certain compounds of the present invention may exist in specific geometric or stereoisomeric forms. The present invention encompasses all compounds, including their cis and trans isomers, R and S enantiomers, diastereomers, (D) isomers, (L) isomers, racemic mixtures and other mixtures. In addition, asymmetric carbon atoms can represent a substituent, such as alkyl. All isomers, as well as mixtures thereof, are included in the present invention.

According to the present invention, the ratio of isomers in a mixture of isomers having the same molecular formula can be varied. For example, a mixture with only two isomers can have the following combinations: 50:50, 60:40, 70:30, 80:20, 90:10, 95:5, 96:4, 97:3, 98:2, 99:1, or 100:0. All ratios of isomers are within the scope of the present invention. Similar ratios, as well as ratios for more complex mixtures of isomers that are readily understood by those skilled in the art, are also within the scope of the present invention.

The present invention also includes isotope labeled compounds that are equivalent to the original compounds disclosed herein. However, in practice, it often occurs that one or more atoms are replaced with atoms with a different atomic weight or mass number. Examples of isotopes that may be listed as compounds of the present invention include hydrogen, carbon, nitrogen, oxygen, phosphorus, sulfur, fluorine, and chlorine isotopes, such as ²H, ³H, ¹³C, ¹¹C, ¹⁴C, ¹⁵N, ¹⁸O, ¹⁷O, ³¹P, ³²P, ³⁵S, ¹⁸F, and ³⁶Cl respectively. The compound, or enantiomer, diastereomer, isomer, or pharmaceutically acceptable salt or solvate thereof in the present invention, containing the isotope or other isotopic atom of the compound specified above, are included within the scope of the present invention. Certain isotope labeled compounds of the present invention, such as radioactive isotopes of ³H and ¹⁴C, are also included, which are useful in tissue distribution experiments of drugs and substrates. Tritium, i.e. ³H, and carbon-14, i.e. ¹⁴C, are preferred for their relative ease of preparation and detection. In addition, heavier isotope substitutions such as deuterium, i.e. ²H, have advantages in certain therapies due to their good metabolic stability, such as increasing half-life or reducing dosage in the body, and therefore can be preferred in some cases. Isotope labeled compounds can be prepared using general methods by replacing non-isotopic reagents with readily available isotope labeled reagents, using the protocols disclosed in the examples.

If the synthesis of a specific enantiomer of the compound of the invention is to be designed, it can be prepared by asymmetric synthesis, or derivatized with a chiral auxiliary, and the resulting diastereomeric mixture is separated and then the chiral auxiliary is removed to give the pure enantiomer. In addition, if the molecule contains a basic functional group, such as an amino acid, or an acidic functional group, such as carboxyl, a diastereomeric salt may be formed with a corresponding suitable optically active acid or base, and then separated by conventional means such as separation crystallization or chromatography and the like, to obtain a pure enantiomer.

As described herein, the compounds of the present invention may be substituted with any number of substituents or functional groups to expand their scope. Generally, whether the term "substituted" appears before or after the term "optionally", the general formula of the formulation of the present invention including the substituents, refers to that the hydrogen radical is replaced by a specified structural substituent. When a specific structure is substituted with multiple substituents at multiple positions, the substituents may be the same or different at each position. The term "substituted" used herein includes all permitted substitutions of organic compounds. Broadly, permitted substitutions include acyclic, cyclic, branched and unbranched, carbocyclic and heterocyclic, arylcyclic and non-arylcyclic organic compounds. In the present invention, heteroatoms such as nitrogen may be supplemented with hydrogen substituents or any of permitted organic compounds described above to replenish its valence. Furthermore, the present invention is not intended to be limited in any way to the permitted substitution of organic compounds. The present invention considers that combinations of substituents and variable groups are excellent in the treatment of diseases in the form of stable compounds. The term "stable" used herein refers to a compound that is stable, and maintains the structural integrity of the compound for a sufficiently long period of detection time, preferably being effective for a sufficiently long period of time, and is used herein for the above purposes.

### Example 1. Synthesis of Compound RX001

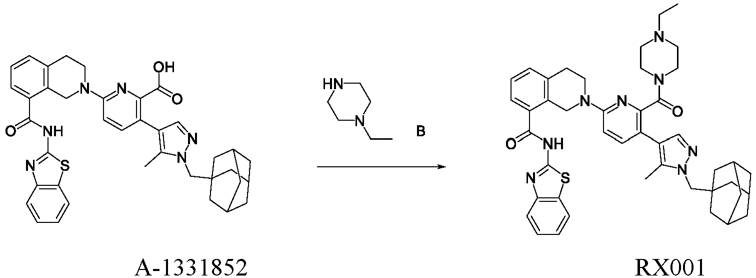

Compound A-1331852 (100 mg, 0.1519 mmol) was dissolved in 5 mL DMF, and TBTU (O-benzotriazole-N,N,N',N'-tetramethylurea tetrafluoroborate)(49 mg, 0.1519 mmol) and DIPEA (20 mg, 0.1519 mmol) were added, then Compound B (21 mg, 0.1823 mmol) was added. The reaction was allowed to proceed at room temperature for 5 hours. TLC (DCM: MeOH = 10:1) was used to monitor the reaction. H₂O (50 mL) was added to the reaction system and stirred for 1 hour. The filtrate cake was collected by suction filtration. The crude product was purified by silica gel column chromatography. MS (ESI) m/z = 755.5 [M+H]⁺; ¹H NMR (400 MHz, CDCl₃) *δ* 11.34 (s, 1H), 7.96-7.74 (m, 1H), 7.62-7.48 (m, 1H), 7.39 (m, 2H), 7.31 (m, 2H), 7.28-7.21 (m, 2H), 7.18 (m, 1H), 6.81 (d, 1H), 5.00 (s, 2H), 4.00 (m, 2H), 3.79-3.68 (m, 2H), 3.68-3.52 (m, 2H), 3.12 (t, J = 5.1 Hz, 2H), 3.01 (m, 2H), 2.32 (m, 4H), 2.19 (d, J = 4.1 Hz, 3H), 2.13-2.03 (m, 2H), 2.00 (s, 3H), 1.66 (m, 12H), 1.01 (t, 3H).

### Example 2. Synthesis of Compound RX002

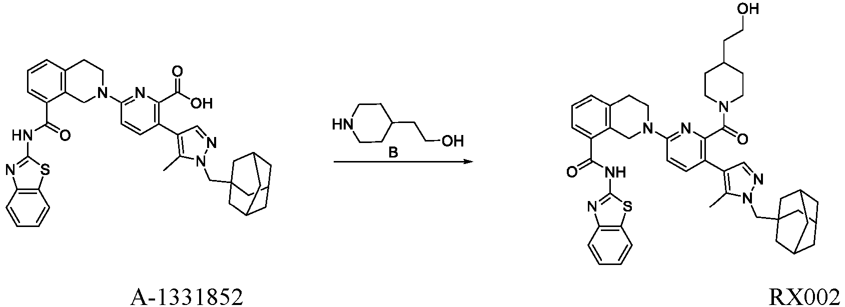

Compound A-1331852 (100 mg, 0.1519 mmol) was dissolved in 5 mL DMF, and TBTU (49 mg, 0.1519 mmol) and DIPEA (20 mg, 0.1823 mmol) were added, then Compound B (24 mg, 0.1823 mmol) was added. The reaction was allowed to proceed at room temperature for 5 hours. TLC (DCM: MeOH = 10:1) was used to monitor the reaction. H₂O (50 mL) was added to the reaction system and stirred for 1 hour. The filtrate cake was collected by suction filtration. The crude product was purified by silica gel column chromatography. MS (ESI) m/z = 770.5 [M+H]⁺; ¹H NMR (400 MHz, CDCl₃) *δ* 7.91-7.83 (d, 1H), 7.60-7.31 (m, 7H), 7.25 (m, 1H), 6.79 (d, *J* = 8.8 Hz, 1H), 5.04 (s, 2H), 4.54 (d, *J* = 13.3 Hz, 1H), 3.99 (q, *J* = 5.8 Hz, 2H), 3.77 (m, 2H), 3.72 (s, 2H), 3.60 (t, *J* = 6.5 Hz, 2H), 3.34 (d, *J* = 13.3 Hz, 1H), 3.04 (t, *J* = 6.0 Hz, 2H), 2.74 (t, 1H), 2.51 (t, 1H), 2.19 (s, 3H), 2.00 (s, 3H), 1.90-1.85 (m, 2H), 1.78-1.60 (m, 12H), 1.38 (m, 2H), 1.04 (m, 1H).

### Example 3. Synthesis of Compound RX003

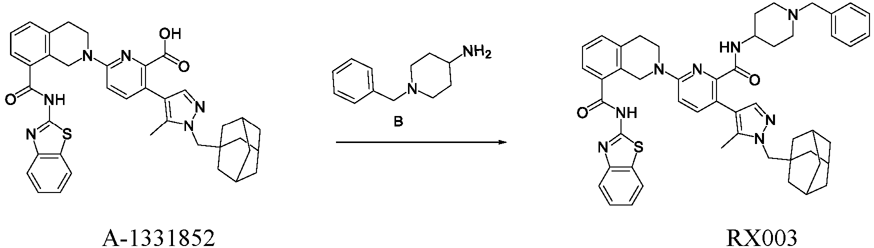

Compound A-1331852 (100 mg, 0.1519 mmol) was dissolved in 5 mL DMF, and TBTU (59 mg, 0.1823 mmol) and DIPEA (24 mg, 0.1823 mmol) were added, then Compound B (35 mg, 0.1823 mmol) was added. The reaction was allowed to proceed at room temperature for 5 hours. TLC (DCM: MeOH = 10:1) was used to monitor the reaction. H₂O (50 mL) was added to the reaction system and stirred for 1 hour. The filtrate cake was collected by suction filtration. The crude product was purified by silica gel column chromatography. MS (ESI) m/z = 831.3 [M+H]⁺; ¹H NMR (400 MHz, CDCl₃) *δ* 7.76 (d, J = 7.7 Hz, 1H), 7.63 (d, J = 7.6 Hz, 2H), 7.50-7.30 (m, 10H), 7.28-7.24 (m, 1H), 6.94 (d, J = 8.7 Hz, 1H), 5.17 (s, 2H), 3.90 (m, 3H), 3.71 (s, 4H), 3.07 (t, J = 5.9 Hz, 2H), 2.97 (s, 2H), 2.29 (s, 2H), 2.07 (s, 3H), 1.99 (m, 7H), 1.75-1.56 (m, 12H).

### Example 4. Synthesis of Compound RX004

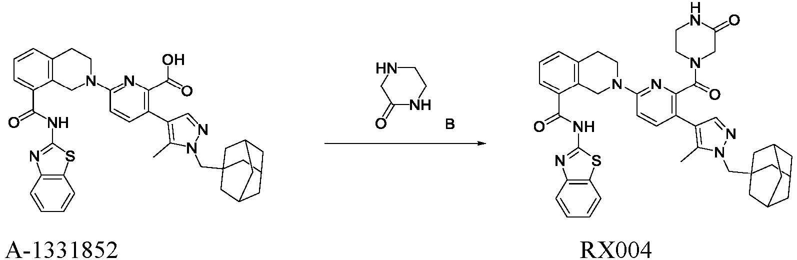

Compound A-1331852 (100 mg, 0.1519 mmol) was dissolved in 5 mL DMF, and TBTU (59 mg, 0.1823 mmol) and DIPEA (24 mg, 0.1823 mmol) were added, then Compound B (18 mg, 0.1823 mmol) was added. The reaction was allowed to proceed at room temperature for 5 hours. TLC (DCM: MeOH = 10:1) was used to monitor the reaction. H₂O (50 mL) was added to the reaction system and stirred for 1 hour. The filtrate cake was collected by suction filtration. The crude product was purified by silica gel column chromatography. MS (ESI) m/z = 741.5 [M+H]⁺; ¹H NMR (400 MHz, CDCl₃) *δ* 11.10 (s, 1H), 7.92-7.85 (m, 1H), 7.64-7.29 (m, 8H), 6.98 (s, 0.5H), 6.83 (d, 1H), 6.47 (s, 0.5H), 5.05 (s, J = 7.8 Hz, 2H), 4.23 (s, 0.5H), 3.94 (m, 2.5H), 3.78-3.67 (m, 4.5H), 3.37 (m, 0.5H), 3.24 (m, 2H), 3.03 (m, 2H), 2.18 (s, J = 2.5 Hz, 3H), 2.00 (s, 3H), 1.70 (m, J = 10.8 Hz, 12H).

### Example 5. Synthesis of Compound RX005

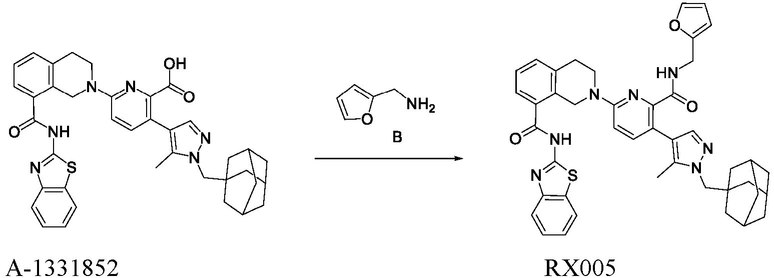

Compound A-1331852 (100 mg, 0.1519 mmol) was dissolved in 5 mL DMF, and TBTU (59 mg, 0.1823 mmol) and DIPEA (24 mg, 0.1823 mmol) were added, then Compound B (18 mg, 0.1823 mmol) was added. The reaction was allowed to proceed at room temperature for 5 hours. TLC (DCM: MeOH = 10:1) was used to monitor the reaction. H₂O (50 mL) was added to the reaction system and stirred for 1 hour. The filtrate cake was collected by suction filtration. The crude product was purified by silica gel column chromatography. MS (ESI) m/z = 738.4 [M+H]⁺; ¹H NMR (400 MHz, CDCl₃) *δ* 10.76 (s, 2H), 8.19 (t, J = 6.0 Hz, 1H), 7.90-7.82 (m, 1H), 7.58-7.31 (m, 7H), 7.25 (d, J = 15.3 Hz, 1H), 6.92 (d, J = 8.7 Hz, 1H), 6.30-6.15 (m, 2H), 5.16 (s, 2H), 4.57 (d, J = 6.0 Hz, 2H), 3.92 (t, J = 6.1 Hz, 2H), 3.73 (s, 2H), 3.08 (t, J = 6.0 Hz, 2H), 2.05 (s, 3H), 2.00 (s, 3H), 1.63 (m, 12H).

### Example 6. Synthesis of Compound RX006

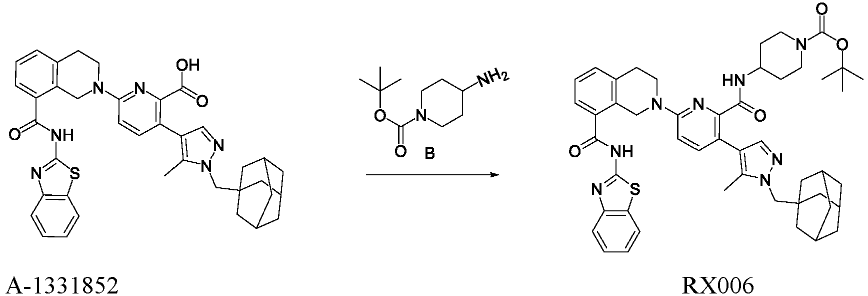

Compound A-1331852 (100 mg, 0.1519 mmol) was dissolved in 5 mL DMF, and TBTU (59 mg, 0.1823 mmol) and DIPEA (24 mg, 0.1823 mmol) were added, then Compound B (37 mg, 0.1823 mmol) was added. The reaction was allowed to proceed at room temperature for 5 hours. TLC (DCM: MeOH = 10:1) was used to monitor the reaction. H₂O (50 mL) was added to the reaction system and stirred for 1 hour. The filtrate cake was collected by suction filtration. The crude product was purified by silica gel column chromatography. MS (ESI) m/z = 841.5 [M+H]⁺; ¹H NMR (400 MHz, CDCl₃) *δ* 10.53 (s, 2H), 8.07-7.99 (m, 1H), 7.77 (d, J = 8.2 Hz, 1H), 7.63-7.32 (m, 7H), 6.92 (d, J = 8.7 Hz, 1H), 5.19 (s, 2H), 4.10 (s, 2H), 4.00 (m, 1H), 3.89 (s, 2H), 3.79-3.69 (m, 2H), 3.10 (t, J = 6.0 Hz, 2H), 2.92 (t, J = 12.6 Hz, 2H), 2.10 (s, 3H), 2.01 (s, 3H), 1.97 (s, 2H), 1.77-1.63 (m, 14H), 1.47 (s, 9H).

### Example 7. Synthesis of Compound RX007

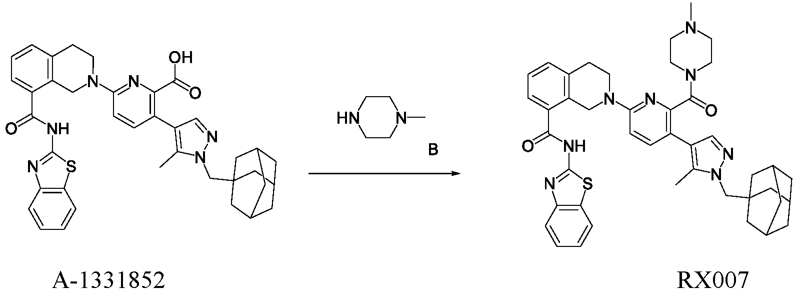

Compound A-1331852 (100 mg, 0.1519 mmol) was dissolved in 5 mL DMF, and TBTU (59 mg, 0.1823 mmol) and DIPEA (24 mg, 0.1823 mmol) were added, then Compound B (18.3 mg, 0.1823 mmol) was added. The reaction was allowed to proceed at room temperature for 5 hours. TLC (DCM: MeOH = 10:1) was used to monitor the reaction. H₂O (50 mL) was added to the reaction system and stirred for 1 hour. The filtrate cake was collected by suction filtration. The crude product was purified by silica gel column chromatography. MS (ESI) m/z = 741.5 [M+H]⁺; ¹H NMR (400 MHz, CDCl₃) *δ* 10.65 (s, 1H), 7.93-7.81 (m, 1H),7.54 (m, 2H), 7.45-7.31 (m, 5H), 7.26 (t, J = 7.6 Hz, 1H), 6.81 (d, J = 8.8 Hz, 1H), 5.03 (s, 2H), 4.01 (t, J = 5.9 Hz, 2H), 3.73 (s, 2H), 3.67-3.58 (m, 2H), 3.12 (t, J = 5.0 Hz, 2H), 3.04 (t, J = 6.0 Hz, 2H), 2.29 (t, J = 5.2 Hz, 2H), 2.20 (ds, 5H), 2.07 (t, J = 5.2 Hz, 2H), 2.01 (s, 3H), 1.87-1.63 (m, 12H).

### Example 8. Synthesis of Compound RX008

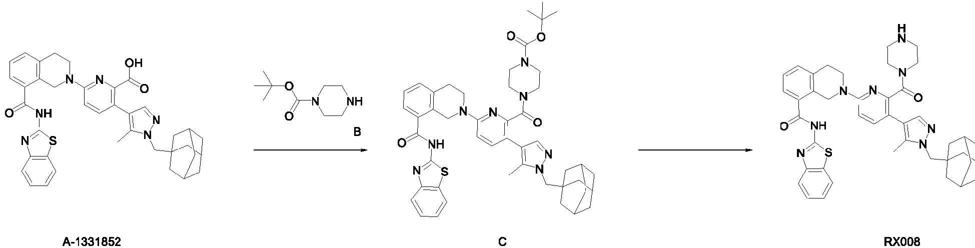

Compound A-1331852 (100 mg, 0.1519 mmol) was dissolved in 5 mL DMF, and TBTU (59 mg, 0.1823 mmol) and DIPEA (24 mg, 0.1823 mmol) were added, then Compound B (34 mg, 0.1823 mmol) was added. The reaction was allowed to proceed at room temperature for 5 hours. TLC (DCM: MeOH = 10:1) was used to monitor the reaction. H₂O (50 mL) was added to the reaction system and stirred for 1 hour. The filtrate cake was collected by suction filtration. The crude product was purified by silica gel column chromatography to obtain compound C. Compound C (85 mg, 0.1029 mmol) was dissolved in 1 mL DCM, HCl/dioxane (3 mL, 12 mmol) was added and the reaction was allowed to react at room temperature for 2 hours. The reaction solution was concentrated, triturated with the crude ether (10 mL) for 5 hours, suction-filtered and dried. MS (ESI) m/z = 727.4 [M+H]⁺; ¹H NMR (400 MHz, DMSO-*d₆*) *δ* 12.87 (s, 1H), 9.36 (s, 2H), 8.08 (d, J = 7.6 Hz, 1H), 7.80 (d, J = 7.9 Hz, 1H), 7.67-7.32 (m, 6H), 7.25 (s, 1H), 6.93 (d, J = 8.9 Hz, 1H), 4.95 (s, 2H), 3.86 (t, J = 5.9 Hz, 2H), 3.73 (s, 2H), 3.57 (s, 2H), 3.28 (t, J = 5.1 Hz, 2H), 3.02 (t, J = 6.0 Hz, 2H), 2.98 (s, 2H), 2.78 (s, 2H), 2.14 (s, 3H), 2.00-1.88 (s, 3H), 1.72-1.44 (m, 12H).

### Example 9. Synthesis of Compound RX009

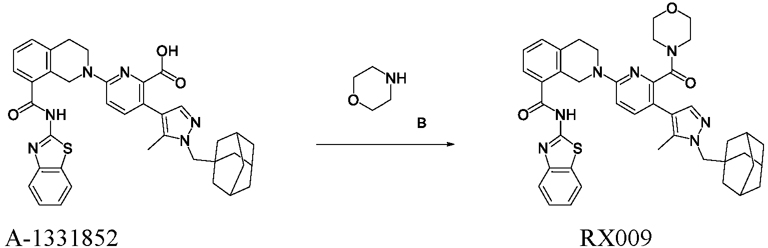

Compound A-1331852 (100 mg, 0.1519 mmol) was dissolved in 5 mL DMF, and TBTU (59 mg, 0.1823 mmol) and DIPEA (24 mg, 0.1823 mmol) were added, then Compound B (15.9 mg, 0.1823 mmol) was added. The reaction was allowed to proceed at room temperature for 5 hours. TLC (DCM: MeOH = 10:1) was used to monitor the reaction. H₂O (50 mL) was added to the reaction system and stirred for 1 hour. The filtrate cake was collected by suction filtration. The crude product was purified by silica gel column chromatography. MS (ESI) m/z = 728.3 [M+H]⁺; ¹H NMR (400 MHz, CDCl₃) *δ* 10.80 (s, 1H), 7.89-7.83 (m, 1H), 7.57 (d, J = 7.7, 1.3 Hz, 1H), 7.49-7.30 (m, 6H), 7.25 (t, J = 7.6 Hz, 1H), 6.81 (d, J = 8.8 Hz, 1H), 5.02 (s, 2H), 3.99 (t, J = 6.0 Hz, 2H), 3.75 (s, 2H), 3.64-3.53 (dt, 4H), 3.35 (dd, J = 5.7, 3.8 Hz, 2H), 3.12 (t, J = 4.7 Hz, 2H), 3.04 (t, J = 6.0 Hz, 2H), 2.20 (s, 3H), 2.01 (s, 3H), 1.73 (m, 12H).

### Example 10. Synthesis of Compound RX010

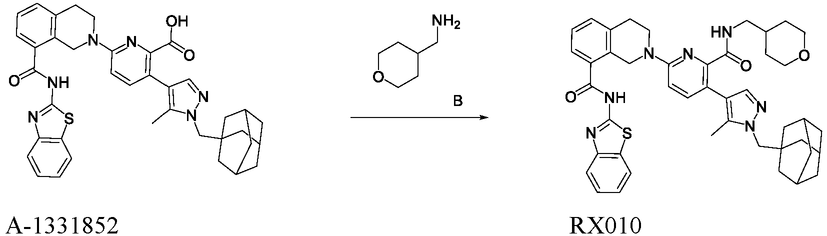

Compound A-1331852 (100 mg, 0.1519 mmol) was dissolved in 5 mL DMF, and TBTU (59 mg, 0.1823 mmol) and DIPEA (24 mg, 0.1823 mmol) were added, then Compound B (21 mg, 0.1823 mmol) was added. The reaction was allowed to proceed at room temperature for 5 hours. TLC (DCM: MeOH = 10:1) was used to monitor the reaction. H₂O (50 mL) was added to the reaction system and stirred for 1 hour. The filtrate cake was collected by suction filtration. The crude product was purified by silica gel column chromatography. MS (ESI) m/z = 756.5 [M+H]⁺; ¹H NMR (400 MHz, CDCl₃) *δ* 10.56 (s, 1H), 8.01 (m, 1H), 7.91-7.81 (m, 1H), 7.56 (m, 1H), 7.50-7.42 (m, 2H), 7.40 (s, 1H), 7.39-7.32 (m, 3H), 6.93 (d, J = 8.7 Hz, 1H), 5.21 (s, 2H), 3.97-3.86 (m, 4H), 3.73 (s, 2H), 3.36-3.28 (m, 2H), 3.26 (t, J = 6.8 Hz, 2H), 3.10 (t, J = 6.0 Hz, 2H), 2.08 (s, 3H), 2.00 (s, 3H), 1.86 (m, 1H), 1.70-1.63 (m, 12H), 1.61-1.55 (m, 2H), 1.32-1.26 (m, 2H).

### Example 11. Synthesis of Compound RX011

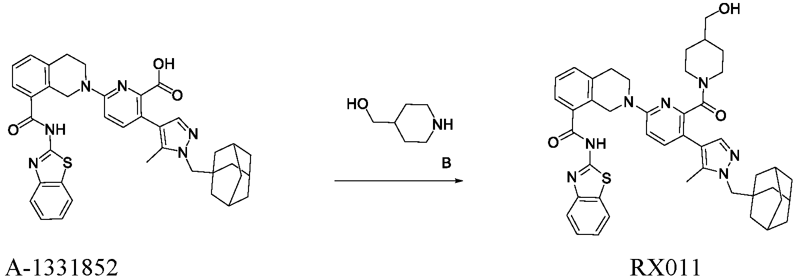

Compound A-1331852 (100 mg, 0.1519 mmol) was dissolved in 5 mL DMF, and TBTU (59 mg, 0.1823 mmol) and DIPEA (24 mg, 0.1823 mmol) were added, then Compound B (21mg, 0.1823 mmol) was added. The reaction was allowed to proceed at room temperature for 5 hours. TLC (DCM: MeOH = 10:1) was used to monitor the reaction. H₂O (50 mL) was added to the reaction system and stirred for 1 hour. The filtrate cake was collected by suction filtration. The crude product was purified by silica gel column chromatography. MS (ESI) m/z = 756.5 [M+H]⁺; ¹H NMR (400 MHz, CDCl₃) *δ* 7.87 (m, 1H), 7.62-7.49 (m, 2H), 7.47-7.30 (m, 5H), 7.25 (m, 1H), 6.82 (d, J = 8.7 Hz, 1H), 5.20 (d, J = 17.3 Hz, 1H), 4.94 (d, J = 17.3 Hz, 1H), 4.58 (d, J = 13.2 Hz, 1H), 4.06 (m, 1H), 3.91 (m, 1H), 3.72 (s, 2H), 3.48-3.40 (m, 2H), 3.30 (d, J = 13.4 Hz, 1H), 3.11-2.92 (m, 2H), 2.76-2.64 (m, 1H), 2.54-2.41 (m, 1H), 2.19 (s, 3H), 2.00 (s, 3H), 1.77-1.60 (m, 12H), 1.37 (m, J = 15.3 Hz, 2H), 1.32-1.27 (m, 2H), 1.18-1.05 (m, 1H).

### Example 12. Synthesis of Compound RX012

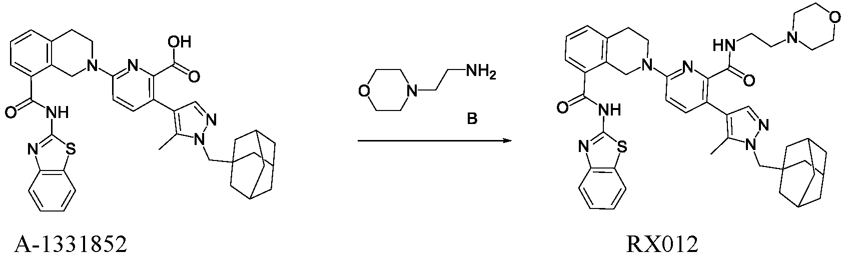

Compound A-1331852 (100 mg, 0.1519 mmol) was dissolved in 5 mL DMF, and TBTU (59 mg, 0.1823 mmol) and DIPEA (24 mg, 0.1823 mmol) were added, then Compound B (24 mg, 0.1823 mmol) was added. The reaction was allowed to proceed at room temperature for 5 hours. TLC (DCM: MeOH = 10:1) was used to monitor the reaction. H₂O (50 mL) was added to the reaction system and stirred for 1 hour. The filtrate cake was collected by suction filtration. The crude product was purified by silica gel column chromatography. MS (ESI) m/z = 771.4 [M+H]⁺; ¹H NMR (400 MHz, CDCl₃) *δ* 10.55 (s, 2H), 8.06 (d, J = 6.1 Hz, 1H), 7.91-7.84 (m, 1H), 7.57 (d, J = 7.5 Hz, 1H), 7.52-7.43 (m, 2H), 7.43-7.30 (m, 4H), 6.93 (d, J = 8.7 Hz, 1H), 5.13 (s, 2H), 4.01 (t, J = 6.0 Hz, 2H), 3.73 (s, 2H), 3.72 (dt, J = 4.7 Hz, 4H), 3.49 (q, J = 5.9, 5.3 Hz, 2H), 3.11 (t, J = 6.0 Hz, 2H), 2.57 (t, J = 6.3 Hz, 2H), 2.50 (m, 4H), 2.11 (s, 3H), 2.00 (s, 3H), 1.80-1.63 (m, 12H).

### Example 13. Synthesis of Compound RX013

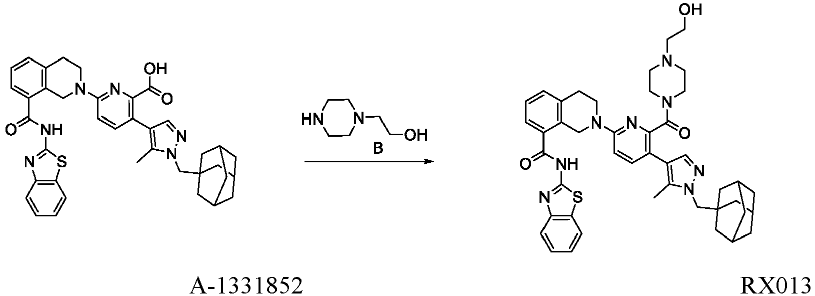

Compound A-1331852 (100 mg, 0.1519 mmol) was dissolved in 5 mL DMF, and TBTU (49 mg, 0.1519 mmol) and DIPEA (24 mg, 0.1823 mmol) were added, then Compound B (24 mg, 0.1823 mmol) was added. The reaction was allowed to proceed at room temperature for 5 hours. TLC (DCM: MeOH = 10:1) was used to monitor the reaction. H₂O (50 mL) was added to the reaction system and stirred for 1 hour. The filtrate cake was collected by suction filtration. The crude product was purified by silica gel column chromatography. MS (ESI) m/z = 771.3 [M+H]⁺; ¹H NMR (400 MHz, CDCl₃) *δ* 8.02 (d, J = 8.1 Hz, 1H), 7.72 (m, 2H), 7.57 - 7.49 (m, 2H), 7.19 (m, 2H), 7.04 - 6.94 (m, 2H), 4.58 (s, 2H), 4.18 (m, 2H), 3.66 (m, 4H), 3.59 (s, 3H), 3.63 - 3.53 (m, 2H), 2.90 (m, 2H), 2.55 (m, 2H), 2.32 (s, 3H), 2.21 (m, 4H), 2.02 (m, 3H), 1.71 (m, 12H), 1.39 (m, 1H).

### Example 14. Synthesis of Compound RX014

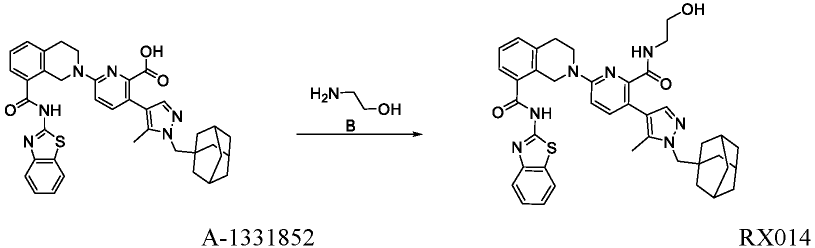

Compound A-1331852 (100 mg, 0.1519 mmol) was dissolved in 5 mL DMF, and TBTU (49 mg, 0.1519 mmol) and DIPEA (24 mg, 0.1823 mmol) were added, then Compound B (11 mg, 0.1823 mmol) was added. The reaction was allowed to proceed at room temperature for 5 hours. TLC (DCM: MeOH = 10:1) was used to monitor the reaction. H₂O (50 mL) was added to the reaction system and stirred for 1 hour. The filtrate cake was collected by suction filtration. The crude product was purified by silica gel column chromatography. MS (ESI) m/z = 702.2 [M+H]⁺; ¹H NMR (400 MHz, Chloroform*-d*) δ 8.34-8.31 (m, 1H), 7.89-7.87 (m, 1H), 7.76 - 7.64 (m, 2H), 7.69 (s, 1H), 7.56 (dd, *J* = 7.5, 2.0 Hz, 1H), 7.36 - 7.27 (m, 1H), 7.28 - 7.13 (m, 3H), 7.05 - 6.93 (m, 2H), 4.58 (s, 2H), 4.18 (t, *J* = 5.6 Hz, 2H), 3.62 - 3.47 (m, 4H), 3.14 (t, *J* = 3.9 Hz, 2H), 2.90 (td, *J= 5.6,* 1.0 Hz, 2H), 2.21 (s, 3H), 2.10 - 1.94 (m, 3H), 1.79 - 1.66 (m, 12H), 1.34 (t, *J =* 5.0 Hz, 1H).

### Example 15. Synthesis of Compound RX015

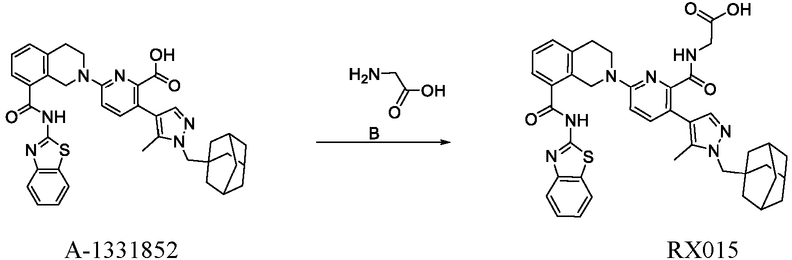

Compound A-1331852 (100 mg, 0.1519 mmol) was dissolved in 5 mL DMF, and TBTU (49 mg, 0.1519 mmol) and DIPEA (24 mg, 0.1823 mmol) were added, then Compound B (14 mg, 0.1823 mmol) was added. The reaction was allowed to proceed at room temperature for 5 hours. TLC (DCM: MeOH = 10:1) was used to monitor the reaction. H₂O (50 mL) was added to the reaction system and stirred for 1 hour. The filtrate cake was collected by suction filtration. The crude product was purified by silica gel column chromatography. MS (ESI) m/z = 716.2 [M+H]⁺; ¹H NMR (400 MHz, CDCl₃) *δ* 8.20 (s, 1H), 8.09 (d, *J* = 8.0 Hz, 1H), 7.74 (dd, *J* = 7.5, 1.6 Hz, 1H), 7.73 - 7.64 (m, 1H), 7.68 (s, 1H), 7.54 (dd, *J* = 7.3, 2.0 Hz, 1H), 7.38 - 7.28 (m, 1H), 7.26 - 7.13 (m, 2H), 7.05 - 6.93 (m, 2H), 4.58 (s, 2H), 4.18 (t, *J* = 5.6 Hz, 2H), 3.60 (d, *J* = 3.9 Hz, 4H), 2.90 (td, *J* = 5.6, 1.0 Hz, 2H), 2.22 (s, 3H), 2.10 - 1.95 (m, 3H), 1.87 - 1.66 (m, 12H).

### Example 16. Synthesis of Compound RX016

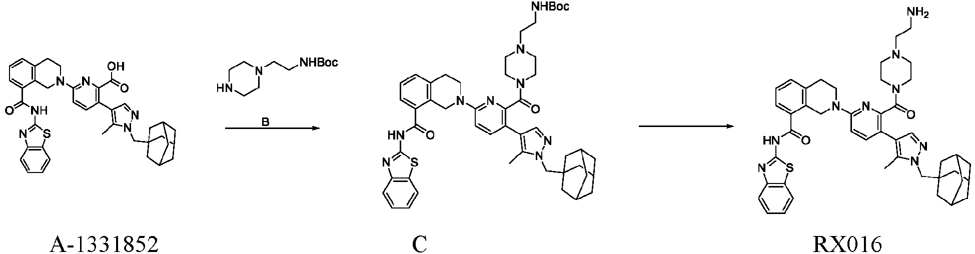

Compound A-1331852 (100 mg, 0.1519 mmol) was dissolved in 5 mL DMF, and TBTU (59 mg, 0.1823 mmol) and DIPEA (24 mg, 0.1823 mmol) were added, then Compound B (42 mg, 0.1823 mmol) was added. The reaction was allowed to proceed at room temperature for 5 hours. TLC (DCM: MeOH = 10:1) was used to monitor the reaction. H₂O (50 mL) was added to the reaction system and stirred for 1 hour. The filtrate cake was collected by suction filtration. The crude product was purified by silica gel column chromatography to obtain compound C. Compound C (90 mg) was dissolved in 1 mL DCM, HCl / dioxane (3 mL, 12 mmol) was added, and the reaction was allowed to react at room temperature for 2 hours. The reaction solution was concentrated, triturated with the crude ether (10 mL) for 5 hours, filtered and dried. MS (ESI) m/z = 770.2 [M+H]⁺; ¹H NMR (400 MHz, CDCl₃) *δ* 8.42-8.30 (brs, 1H), 7.85-7.83 (m, 1H), 7.62-7.58 (m, 2H), 7.39-7.23 (m, 6H), 6.74 (d, J = 8.8 Hz, 1H), 5.12 (s, 2H), 3.90 (m, 2H), 3.69 (s, 2H), 3.30-3.16(m, 4H), 3.08-3.05 (m, 2H), 2.95-2.91 (m, 4H), 2.47-2.43(m, 2H), 2.22 (s, 3H), 2.20-2.15(m, 2H), 1.97 (s, 3H), 1.74-1.50 (m, 12H).

### Examples 17 to 22

According to the same method as the above examples, the following example compounds in Table 1 were prepared using commercially available compounds or by referring to the preparation methods of the intermediate compounds as indicated.

**Table 1**

| Compound ID | Structure | H NMR or MS |
|---|---|---|
| RX017 | 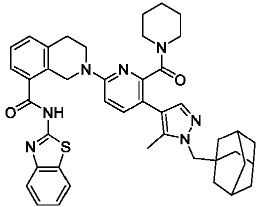 | MS(ESI) m/z = 726.1 [M+H]⁺; |
| RX018 | 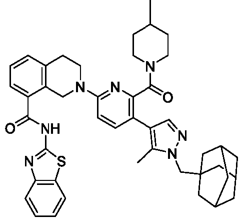 | MS(ESI) m/z = 740.4 [M+H]⁺; |
| RX019 | 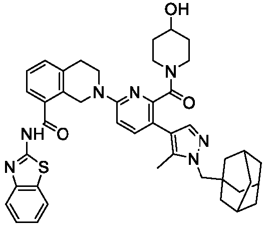 | MS(ESI) m/z = 742.4 [M+H]⁺; |
| RX020 | 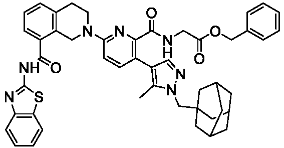 | MS(ESI) m/z = 806.6 [M+H]⁺; |
| RX021 | 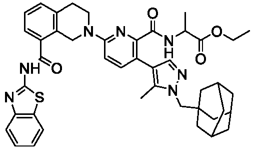 | MS(ESI) m/z = 758.7 [M+H]⁺; |
| RX022 | 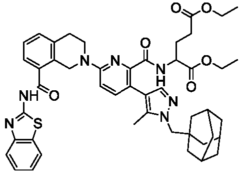 | MS(ESI) m/z = 844.6 [M+H]⁺; |

### Example 23. Synthesis of Compound RX301

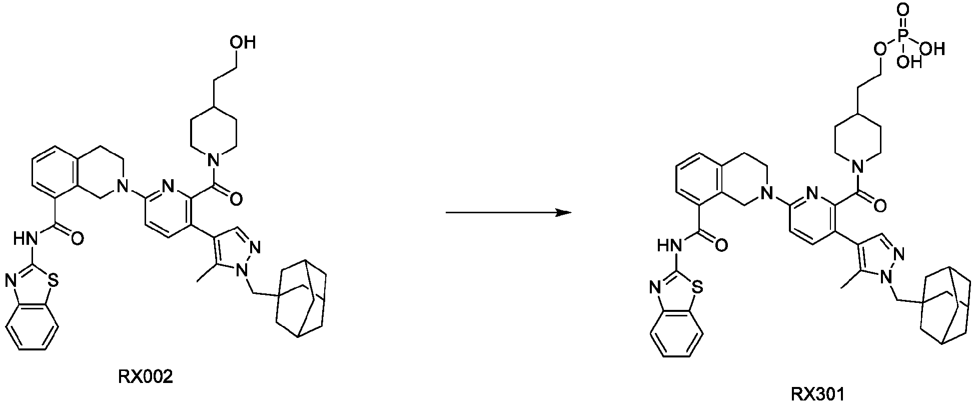

Phosphorus oxychloride (POCl₃) (70 mg, 0.42 mmol) was diluted with pyridine (2 mL) and cooled to -15 °C. RX002 (200 mg, 0.26 mmol) was dissolved in pyridine (1 mL) to prepare a solution, then the solution was added slowly to the above reaction solution dropwise and stirred to react at -15 °C for 2 hours. TLC (DCM: MeOH = 10:1) was used to monitor the reaction, then the reaction system was warmed to -5 °C, water (3 mL) was slowly added to the reaction system, and the reaction mixture was stirred for 1 hour. The pH was adjusted to 10 with 4 N of NaOH. After concentration by oil pump, the sodium salt of the target product RX301 was obtained by preparative purification by alkaline reverse-phase HPLC. MS (ESI) m/z = 850.5 [M+H]⁺; ¹H NMR (400 MHz, DMSO-d₆) δ 7.78 (dd, J = 6.2, 3.0 Hz, 1H), 7.69 (d, J = 7.7 Hz, 1H), 7.55 - 7.40 (m, 2H), 7.29 - 7.11 (m, 4H), 7.04 (t, J = 7.4 Hz, 1H), 6.81 (d, J = 8.9 Hz, 1H), 5.10 (d, J = 10.2 Hz, 2H), 4.26 (s, 1H), 3.81 (dd, J = 14.2, 7.4 Hz, 4H), 3.42 (s, 3H), 3.13 (d, J = 12.3 Hz, 1H), 2.95 (t, J = 5.8 Hz, 2H), 2.76 - 2.62 (m, 1H), 2.14 (s, 3H),1.92-1.85 (m, 2H),1.69 - 1.27 (m, 15H), 1.23 (t, J = 8.1 Hz, 3H), 1.06 (t, J = 7.0 Hz, 2H).

### Example 24. Synthesis of Compound RX302

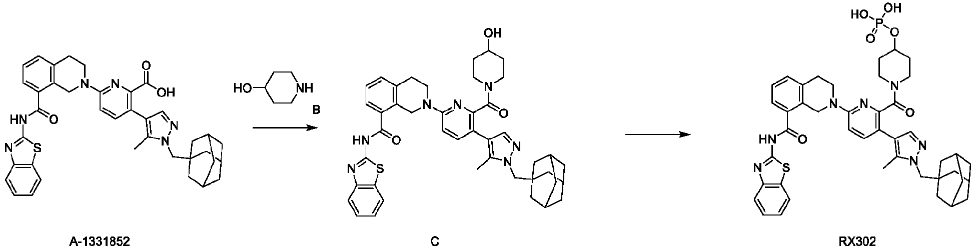

### Step 1:

Compound A-1331852 (100 mg, 0.1519 mmol) was dissolved in 5 mL DMF, and TBTU (59 mg, 0.1823 mmol) and DIPEA (24 mg, 0.1823 mmol) were added, then Compound B (19 mg, 0.1823 mmol) was added. The reaction was allowed to proceed at room temperature for 5 hours. TLC (DCM: MeOH = 10:1) was used to monitor the reaction. H₂O (50 mL) was added to the reaction system and stirred for 1 hour. The filtrate cake was collected by suction filtration. The crude product was purified by silica gel column chromatography. MS (ESI) m/z = 742.4 [M+H]⁺;

### Step 2:

Phosphorus oxychloride (POCl₃) (35 mg, 0.21 mmol) was diluted with pyridine (2 mL) and cooled to -15°C. C (100 mg, 0.13 mmol) was dissolved in pyridine (1 mL) to prepare a solution, then the solution was added slowly to the above reaction solution dropwise and stirred to react at -15 °C for 2 hours. TLC (DCM: MeOH = 10:1) was used to monitor the reaction, then the reaction system was warmed to -5°C, water (3 mL) was slowly added to the reaction system, and the reaction mixture was stirred for 1 hour. The pH was adjusted to 10 with 4 N of NaOH. After concentration by oil pump, the sodium salt of the target product RX302 was obtained by preparative purification by alkaline reverse-phase HPLC. MS (ESI) m/z = 822.5 [M+H]⁺; ¹H NMR (400 MHz, DMSO-*d*₆) δ 7.82 (d, *J =* 8.1 Hz, 1H), 7.68 - 7.58 (m, 2H), 7.49 - 7.34 (m, 3H), 7.26 - 7.14 (m, 2H), 6.99 (td, *J* = 7.5, 1.6 Hz, 1H), 4.35 (s, 2H), 4.03 - 3.88 (m, 3H), 3.61 (ddt, *J =* 34.5, 12.4, 7.0 Hz, 2H), 3.59 (s, 2H), 2.95 - 2.83 (m, 3H), 2.75 (dt, *J* = 12.4, 7.1 Hz, 1H), 2.46 (s, 3H), 2.17 - 1.90 (m, 6H), 1.82 - 1.61 (m, 14H).

### Example 25. Synthesis of Compound RX303

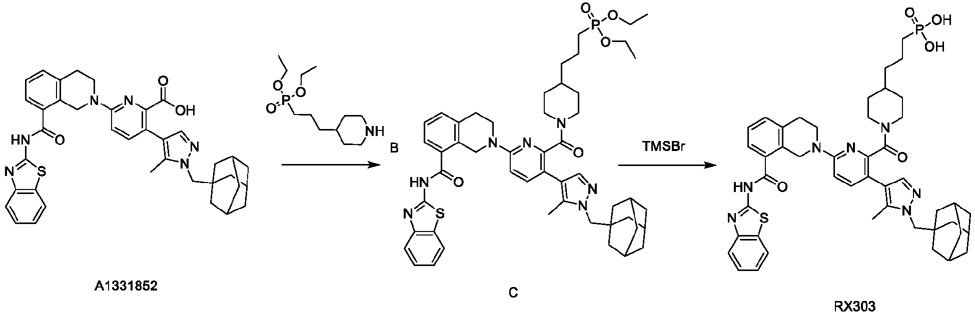

### Step 1:

Compound A-1331852 (100 mg, 0.1519 mmol) was dissolved in 5 mL DMF, and TBTU (59 mg, 0.1823 mmol) and DIPEA (24 mg, 0.1823 mmol) were added, then Compound B (48 mg, 0.1823 mmol) was added. The reaction was allowed to proceed at room temperature for 5 hours. TLC (DCM: MeOH = 10:1) was used to monitor the reaction. H₂O (50 mL) was added to the reaction system and stirred for 1 hour. The filtrate cake was collected by suction filtration. The crude product was purified by silica gel column chromatography. MS (ESI) m/z = 904.5 [M+H]⁺; and

### Step 2:

Trimethylsilyl bromide (77 mg, 0.50 mmol) was added dropwise to a solution of Compound C (90 mg, 0.10 mmol) in dichloromethane (2 mL) and the mixture was stirred at room temperature for 24 h. TLC (DCM: MeOH = 20:1) was used to monitor the reaction, then the reaction system was cooled to 0°C, water (5 mL) was slowly added to the reaction system, and the reaction mixture was stirred for 1 hour. The pH was adjusted to 10 with 4 N of NaOH. After concentration by oil pump, the sodium salt of the target product RX303 was obtained by preparative purification by alkaline reverse-phase HPLC. MS (ESI) m/z = 848.5 [M+H]⁺; ¹H NMR (400 MHz, DMSO-*d*₆) δ 7.85 (d, *J=* 7.9 Hz, 1H), 7.70 - 7.58 (m, 2H), 7.49 - 7.28 (m, 4H), 7.26 - 7.14 (m, 2H), 7.00-6.96 (m, 1H), 4.35 (s, 2H), 4.00 - 3.88 (m, 3H), 3.59 (s, 2H), 3.52 (dt, *J=* 12.6, 7.1 Hz, 1H), 2.90 (t, *J* = 5.7 Hz, 3H), 2.78-2.75(m, 1H), 2.46 (s, 3H), 2.09 - 1.93 (m, 3H), 1.77 - 1.52 (m, 15H), 1.44 - 1.28 (m, 2H), 1.32 - 1.23 (m, 2H), 1.27 - 1.13 (m, 4H).

### Examples 26-30

According to the same method as the above examples, the following example compounds and sodium salts thereof in Table 2 were prepared using commercially available compounds or by referring to the preparation methods of the intermediate compounds as indicated.

**Table 2**

| Compound ID | Structure | H NMR or MS |
|---|---|---|
| RX304 | 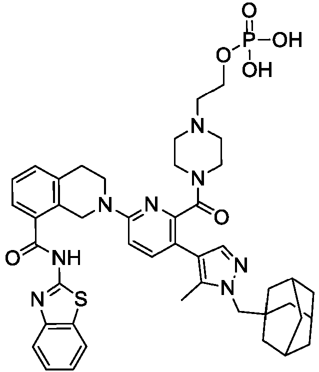 | MS(ESI) m/z = 851.5 [M+H]⁺; |
| RX305 | 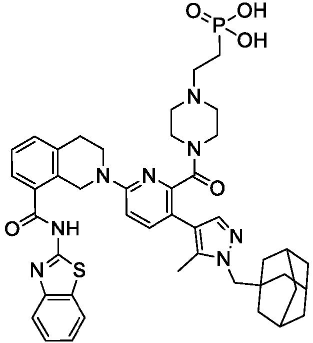 | MS(ESI) m/z = 835.6 [M+H]⁺; |
| RX306 | 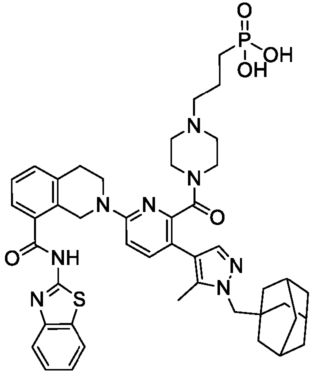 | MS(ESI) m/z = 849.4 [M+H]⁺; |
| RX307 | 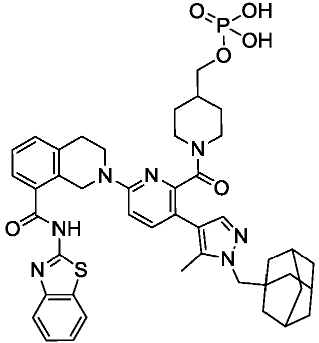 | MS(ESI) m/z = 836.4 [M+H]⁺; |
| RX308 | 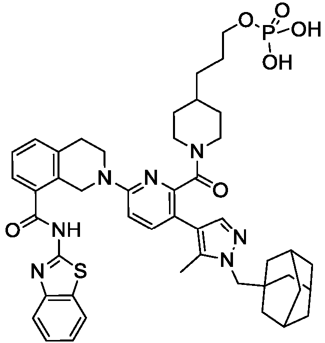 | MS(ESI) m/z = 864.4 [M+H]⁺; |

### Test Example 1: Solubility Test

The following test protocol was used for the solubility tests of the compounds of the present invention:
1). The compounds to be tested were precisely weighed respectively, and formulated to a corresponding concentration (1mg/mL or 10 mg/mL) by adding a corresponding volume of pure water vortically shook for 1 minute every 3 minutes for 15 minutes to observe the dissolution, and then the dissolution was observed again after standing for 1 hour. 2). 0.2 mL of 1 mg/mL compound or sodium salt thereof was took, diluted to 0.1 mg/mL respectively by adding 1.8 mL water, vortically shook for 5 minutes to observe the dissolution, and then the dissolution was observed again after standing for 1 hour. 3). 1 mL of 1 mg/mL compound or sodium salt thereof was took, diluted to 0.5 mg/mL by adding 1 mL water, vortically shook for 5 minutes to observe the dissolution, and then the dissolution was observed again after standing for 1 hour.

The results of the solubility test were shown in Table 3 below.

**Table 3**

| Compound | Formulated Concentration | During shaking | After standing |
|---|---|---|---|
| A-1331852 | 0.10 mg/mL | Visible solute particles | A few solids precipitate and float on the liquid surface |
| A-1331852 | 1.00 mg/mL | Visible solute particles | More solids precipitate and float on the liquid surface |
| Sodium salt of A-1331852 | 0.10 mg/mL | A few visible solute particles | A few solids precipitate and settle in centrifuge tube |
| Sodium salt of A-1331852 | 0.50 mg/mL | A few visible solute particles | Solids precipitate and settle in centrifuge tube |
| Sodium salt of A- | 1mg/mL | A few visible solute particles | Solids precipitate and settle |
| 1331852 | | | in centrifuge tube |
| RX016 hydrochloride | 0.10 mg/mL | A few visible solute particles | A few solids precipitate and settle in centrifuge tube |
| Sodium salt of RX301 | 1mg/mL | No visible solute particles | No solid precipitates |
| Sodium salt of RX301 | 10.00 mg/mL | No visible solute particles | No solid precipitates |
| Sodium salt of RX302 | 10.00 mg/ml | No visible solute particles | No solid precipitates |
| Sodium salt of RX303 | 10.00 mg/ml | No visible solute particles | No solid precipitates |

CONCLUSION: The solubility of sodium salts of compounds RX301, RX302, and RX303 in water is greater than 10 mg/ml, which is significantly better than that of sodium salts of A-1331852 (the solubility of sodium salt of A-1331852 is less than 0.1 mg/ml).

According to the above results, it can be seen that the water solubility of the compounds of the present invention is significantly improved compared with A-1331852 and the sodium salt of A-1331852. Therefore, the compounds of the present invention can be a useful drug with excellent druggability.

### Test Example 2: Platelet Toxicity Test

In this test example, C57 mice were chosen to detect the effect of the target compounds on platelets.

7 to 8 weeks old C56BL/6J mice were randomly divided into vehicle group and compound group. The test compound (0.5 mg/kg) or blank vehicle was administrated via single intraperitoneal injection. Blood was collected from the retroorbital venous plexus of the mice at 6 hours, and placed in a blood routine tube containing anticoagulant EDTA-K2. After shaking gently, the blood physiological indicators were measured using a hematology analyzer, with the platelet count as the target parameter for this platelet toxicity test.

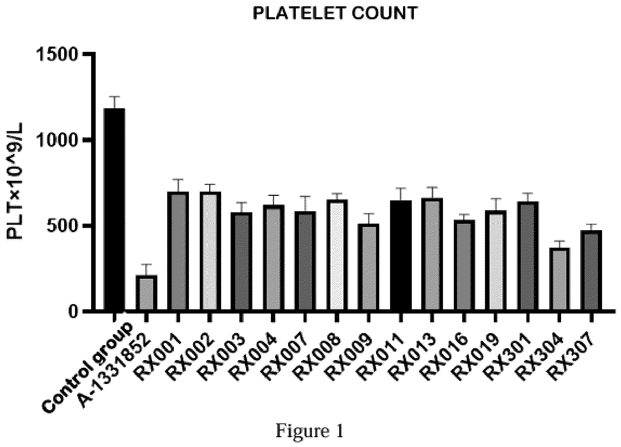

As can be seen from Figure 1, A-1331852 has severe platelet toxicity, while RX001-RX004, RX007-009, RX011, RX013, RX016, RX019, RX301, RX304, and RX307 show significantly improved platelet toxicity compared with A-1331852.

### Test Example 3: Senescent Cell Activity Data

In this test example, human non-small cell lung cancer cell line (A549 cells), human embryonic lung fibroblast cell line (IMR90 cells), human retinal pigment epithelial cells (RPE cells), mouse chondrocytes, and mouse skin fibroblasts were selected. The above cells were induced by etoposide (10 µM) to obtain their senescent cells. The effect of the target compounds on the viability of normal and senescent cells above was examined, thus assessing the specific killing effect of the compounds on senescent cells above.

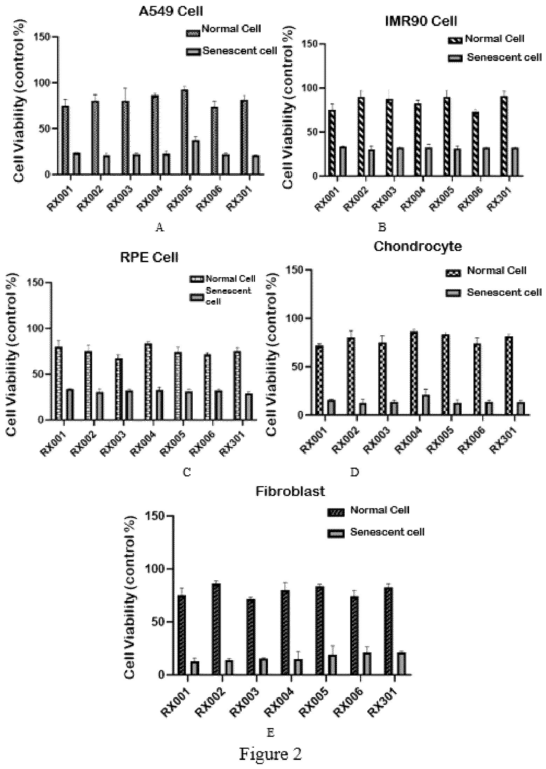

Briefly, senescent and normal cells above were selected and plated separately (96-well plates). After adding the compounds (concentration:1µM) for 72 hours, CellTiter-Glo^{®} Luminescent Cell Viability Assay Kit (Promega) was used to detect cell viability, and the results were shown in Figures 2A to 2E.

As can be seen from Figures 2A to 2E, the compounds can specifically kill senescent cells at a concentration of 1 µM, while having no obvious killing on normal cells.

Table 4 Summarized the activity of killing cells (IC₅₀) of the test compounds against senescent cells.

**Table 4**

| Compound ID | Structure | IC₅₀ (µM, Senescent cell) |
|---|---|---|
| A-1331852 | 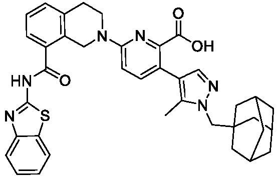 | B |
| RX001 | 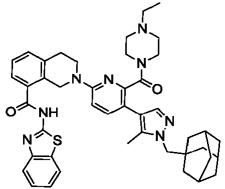 | B |
| RX002 | 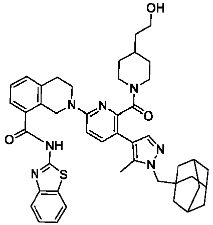 | A |
| RX004 | 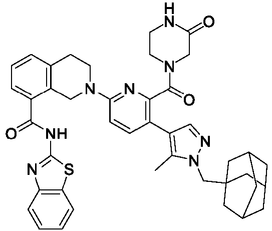 | B |
| RX005 | 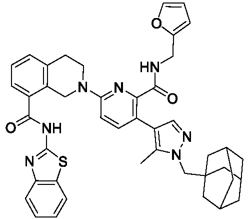 | D |
| RX007 | 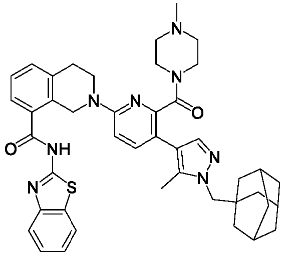 | B |
| RX008 | 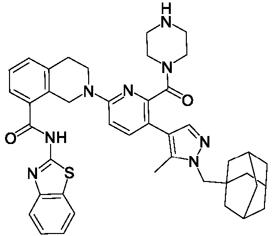 | B |
| RX009 | 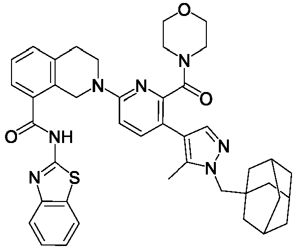 | A |
| RX010 | 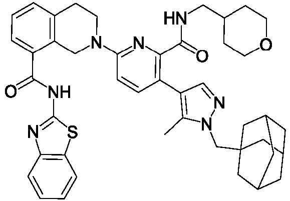 | B |
| RX011 | 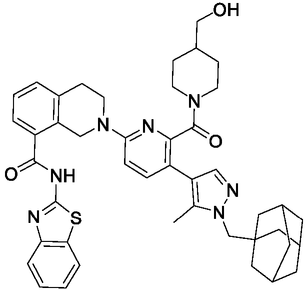 | A |
| RX012 | 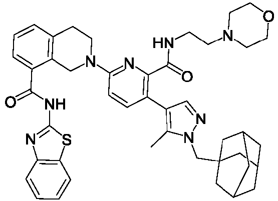 | B |
| RX013 | 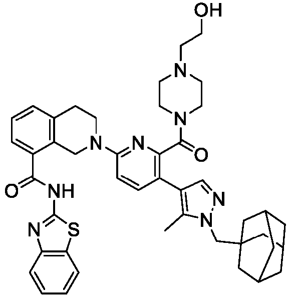 | A |
| RX014 | 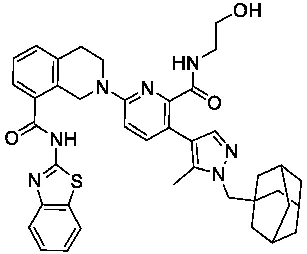 | C |
| RX015 | 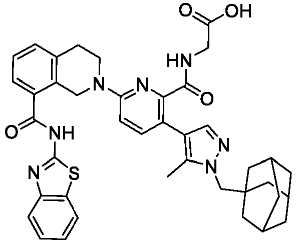 | C |
| RX016 | 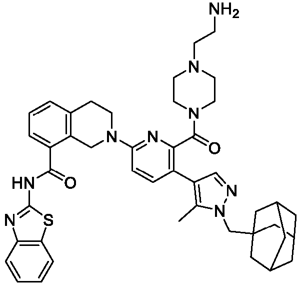 | B |
| RX019 | 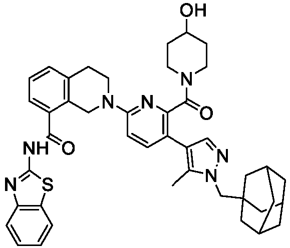 | A |
| RX020 | 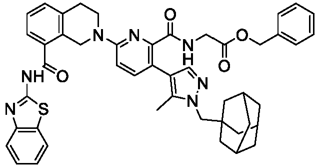 | D |
| RX021 | 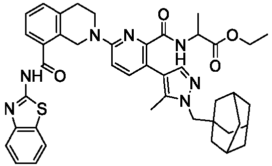 | C |
| RX022 | 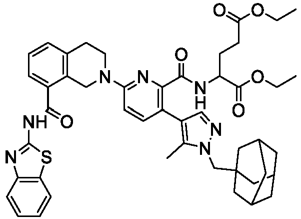 | D |
| RX301 | 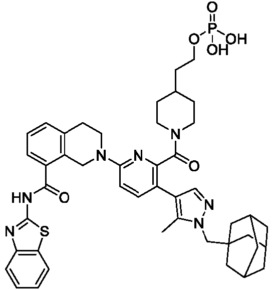 | A |
| RX302 | 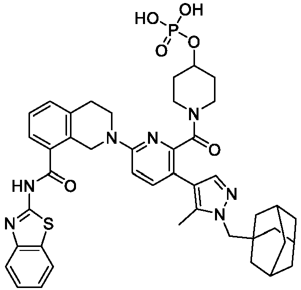 | A |
| RX304 | 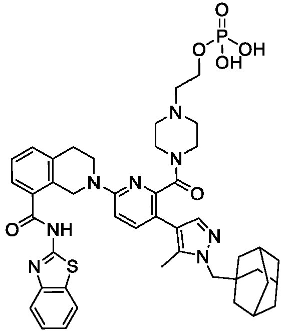 | A |
| RX307 | 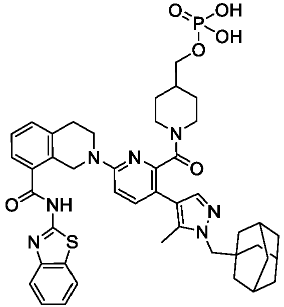 | A |

| | | |
|---|---|---|
| Note: A represents less than 0.2 µM; B represents 0.2 to 1.0 µM; C represents 1 to 10 µM; and D represents greater than 10 µM. | | |

### Test Example 4: Efficacy in Animal Model of Hyperoxia-Induced Retinopathy

The efficacy of the prodrugs RX001-RX006 as well as RX301 were investigated in a mouse model of oxygen-induced retinopathy (OIR) which to some extent indicates an *in vivo* model of retinopathy of prematurity (ROP), diabetic retinopathy, and wet age-related macular degeneration.

From postnatal Day 7 (P7) to Day 12 (P12), C57Bl/6 mouse pups and their CD1 foster mothers were exposed to hyperoxia (75% O₂). At P12, the animals were injected intravitreally with 1µL of the test composition formulated in 1% DMSO, 10% Tween 80, and 20% PEG-400 (200 µM of the compound) and returned to room air until day 17 (P17). On P17, the eyeballs were enucleated, and the retinas were dissected for vascular staining. To determine the area of avascularity or angiogenesis, the retinas were flattened and stained with isolectin B4 (IB4).

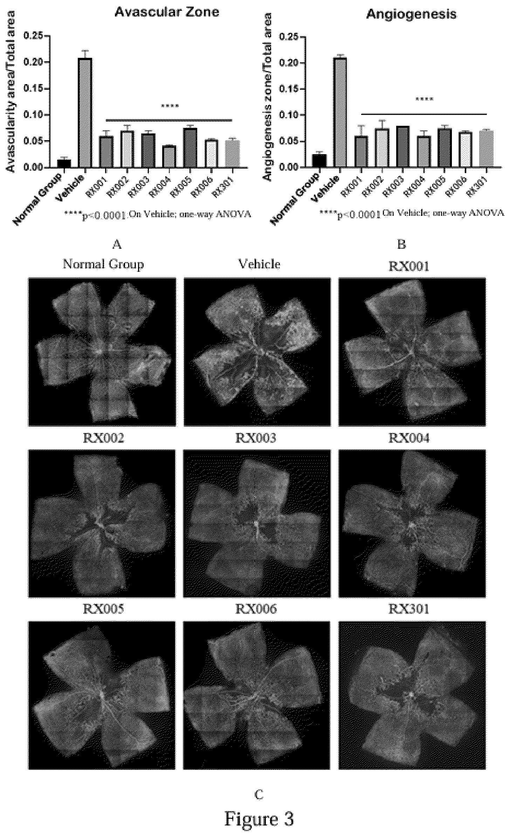

Figure 3 shows that the intravitreal (IVT) administration of RX001-RX006 and RX301 resulted in statistically significant improvements in avascular zone and angiogenesis.

### Test Example 5: Efficacy in Animal Model of Diabetes-Induced Retinopathy

The efficacy of the compounds of the present disclosure was studied in a mouse model of diabetic retinopathy by multiple administrations of streptozotocin (STZ). C57BL/6J mice at 6 to 7 weeks were weighed and their baseline blood glucose was measured (Sino). STZ (Sigma-Alderich) was injected intraperitoneally into the mice at 55 mg/Kg for five consecutive days. Age-matched controls were injected with buffer only. Blood glucose was measured again one week after the last STZ injection. 1µL of the test compound (200 µM, a suspension formulated in 0.015% polysorbate 80, 0.2% sodium phosphate, 0.75% sodium chloride, pH 7.2) was injected intravitreally into the STZ-treated diabetic C57BL/6J mice at 8 and 9 weeks after STZ administration. 10 weeks after STZ treatment, the retinal Evans blue penetration assay was performed.

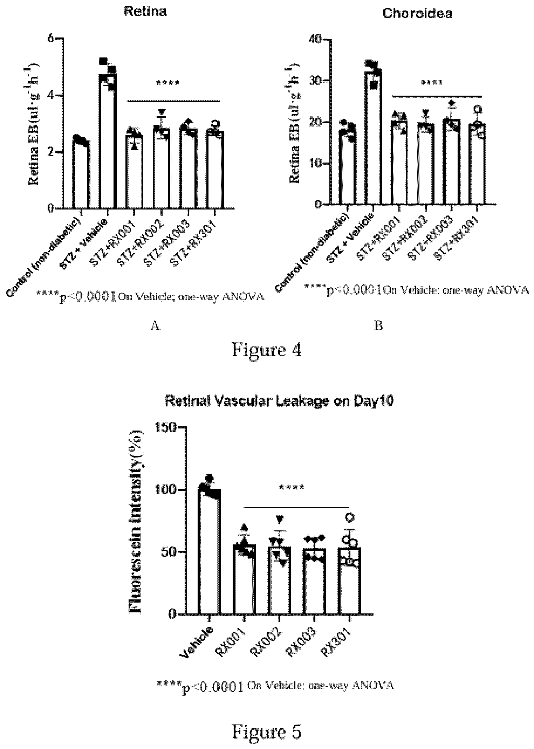

Figure 4 shows the intravitreal (IVT) administration of RX001, RX002, RX003 and RX301 resulted in improved vascular permeability in both the retinal and choroidal vascular leakage at this dose level.

### Test Example 6: Efficacy in CNV Animal Model of Wet-Age-Related Macular Degeneration

In this test example, the efficacy of the compounds of the present disclosure was tested on wet-age-related macular degeneration in a CNV animal model.

Male mice were administered 0.5% probacaine hydrochloride (Alcon) for local anesthesia, and 1% tropicamide to dilate the pupils of the mice. Laser cauterization was performed using a laser photocoagulator (Novus Varia, LUMENIS) with a slit lamp delivery system. And artificially torn adherent glass coverslips were used as contact lenses. Bleeding spots caused by lasers were not included in the statistics. In each experimental group, six mice were analyzed with four impacts per eye to overcome inherent variability.

Figure 5 shows that the intravitreal (IVT) administration of RX001, RX002, RX003 and RX301 resulted in statistically significant improvements in fluorescence intensity indicators.

### Test Example 7: Efficacy in Animal Model of Idiopathic Pulmonary Fibrosis

In this test example, the efficacy of the compounds of the present disclosure was tested on idiopathic pulmonary fibrosis in an idiopathic pulmonary fibrosis animal model.

Briefly, anesthetized 6-week-old SD rats were fixed on a foam board with medical tape. Firstly, the skin on the surface of the trachea was cut with surgical scissors to fully expose the trachea, and a 100-microlitre micro-syringe containing a certain volume of bleomycin (BLM 5 mg/kg) solution drawn based on the body weight of the rat was inserted into the trachea through the oral cavity to administer by slow bolus injection. After administration of the BLM solution, the foam plate was immediately rotated and shaken to distribute the BLM solution as evenly as possible in the lung tissue. Then the skin on the surface of the trachea was sutured with surgical sutures and about 0.5 to 1.0 mL of penicillin solution was dripped onto the epidermis to prevent infection of the surgical wound. After the 7th day of modelling, the compound solution of the present disclosure was administered via intraperitoneal (IP) injection (5 mg/kg) according to the weight of the rat for 3 consecutive weeks.

Rats were euthanized by carbon dioxide on Day 28, the lung tissues were removed, the left alveolar lavage fluid was collected for white blood cell (WBC) counting, the right lung tissue portion was used for hydroxyproline (HYP) assay, and the remaining lung tissues were stained for pathology by H&E staining and Masson staining.

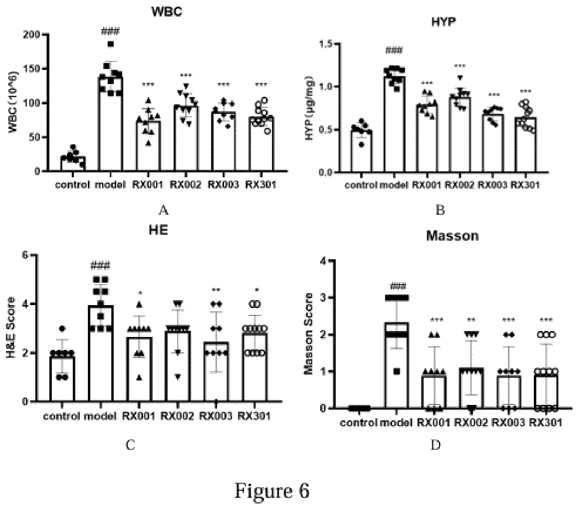

Figure 6 shows that the administration of RX001, RX002, RX003 and RX301 via intraperitoneal (IP) injection resulted in statistically significant improvements in WBC, HYP and pathology scores in the IPF model.

### Test Example 8: Efficacy in Animal Models of Osteoarthritis

In this test example, the efficacy of the compounds of the present disclosure was tested on osteoarthritis in an osteoarthritis animal model.

The osteoarthritis animal model was constructed by the following steps. The mice were anesthetized with isoflurane, and fixed. The hair on the right hind limb of the mice was shaved and the skin was disinfected with alcohol, and the skin of the joints was cut. Under an optical microscope, the inside of the right knee joint was incised longitudinally with a blade, without cutting the patellar ligament. The patellar ligament was pulled to one side with constant hemostasis by compression with sterile cotton swabs such that the joint cavity was fully exposed. Saline was added dropwise in time to avoid the drying up of the joint cavity. The excess muscle tissue was gently separated with a blunt forceps to expose the meniscus ligament. The anterior cruciate ligament was found in the depth of the joint cavity and cut with a micro scissor. Then the wound was disinfected with penicillin solution, the muscle was sutured with sterile needle and thread, and the skin tissue was finally sutured. The efficacy of the drug was determined by intra-articular injection administration. At the endpoint of the test, continued pain threshold of the plantar was detected, and the bone joints were stained with safranin fast green.

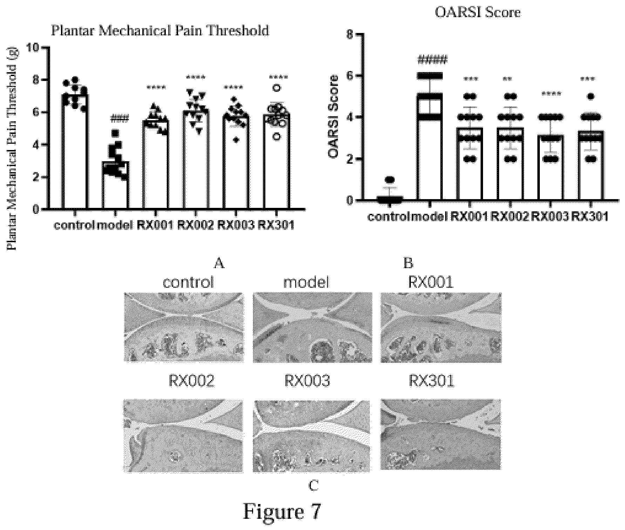

Figure 7 shows that the administration of RX001, RX002, RX003 and RX301 via intra-articular injection resulted in statistically significant improvements in plantar mechanical pain threshold and pathology scores of safranin fast green staining in an osteoarthritis (OA) model.

### Test Example 9: Efficacy in Animal Model of Alzheimer's Disease

In this test example, the efficacy of the compounds of the present disclosure was tested on Alzheimer's disease in an animal model of Alzheimer's disease.

Briefly, a transgenic mouse model of APP×PS1 (purchased from Cyagen Biosciences) was selected, and the compound of the present disclosure was administered by intraperitoneal injection for 11 consecutive weeks, twice a week. Alterations in amyloid in the cerebral cortex of the mice were observed.

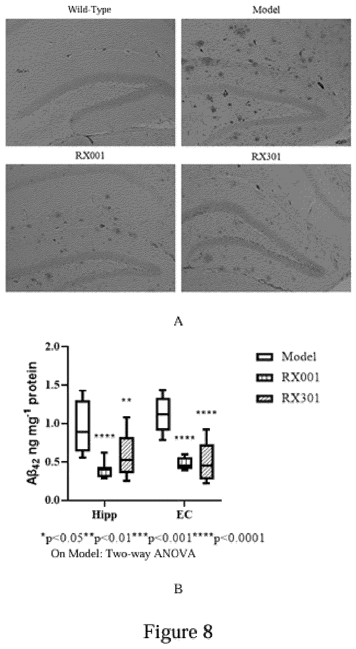

Figure 8 shows that the administration of RX001, RX002, RX003 and RX301 via intraperitoneal injection resulted in statistically significant improvements in Aβ42 pathogenic protein indicators in an AD mouse model.

### Test Example 10: Efficacy in Animal Models of Hepatic Fibrosis

In this test example, the efficacy of the compounds of the present disclosure was tested on hepatic fibrosis in an animal model of hepatic fibrosis.

Briefly, C57BL/6N mice, male, 7 to 8 weeks old, 24 to 28 g of body weight, were used. The mouse model of hepatic fibrosis was induced by intraperitoneal injection of CCl₄ solution at a dosage of 1 mL/kg, with a dosing frequency of 2 times/week, and a modelling cycle of 6 weeks. Dosing via intraperitoneal injection was started starting from the third week of modelling twice a week. At the end of the test, serum was collected, and liver tissue was taken for Masson staining index assay.

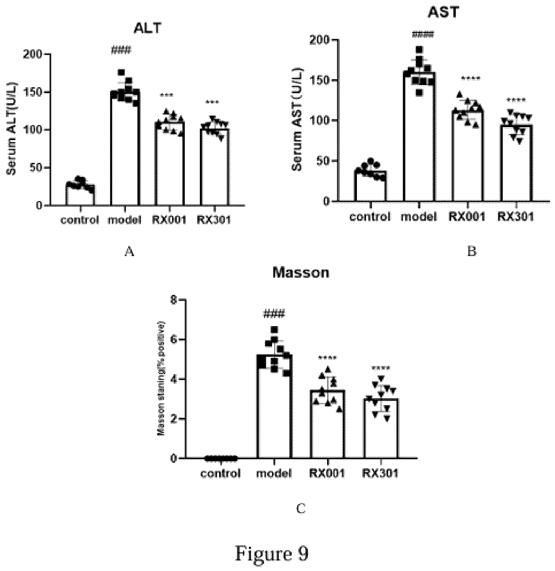

Figure 9 shows that the administration of RX001 and RX301 via intraperitoneal (IP) injection resulted in statistically significant improvements in serum aspartate aminotransferase (AST) and alanine aminotransferase (ALT) levels and pathology scores in a liver fibrosis model.

### Test Example 11: Efficacy on Aging Skin

In this test example, aged female C57bl/6J mice (22 months old) were used. The drug was administered by skin application twice a week for 6 consecutive weeks. Improvement in the epidermal thickness of the skin was observed in aged mice.

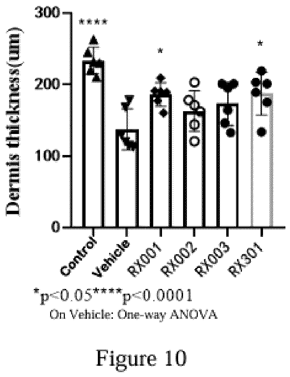

Figure 10 shows that the administration of RX001, RX002, RX003 and RX301 by applying resulted in statistically significant improvements in epidermal thickness scores of the skins in a naturally aging mouse model.

### Test Example 12: Efficacy in Renal Fibrosis Animal Model

In this test example, the efficacy of the compound of the present disclosure was tested on renal fibrosis in an animal model of renal fibrosis. Briefly, C57BL/6J mice, male, 7 to 8 weeks old, were used. The mice were anaesthetized, made to lie supine on a foam board, and disinfected with iodophor, and a longitudinal incision was made in the skin in the middle of the lower abdomen to locate the white line of the abdominal muscle, where the abdominal muscle, fascia and peritoneal layer were dissected. After exposing the surgical field, the kidneys were exposed by pushing the stomach and mesentery of the mice to the right side with a gauze soaked with saline. The model of renal fibrosis was constructed by locating the transparent ureter from the renal pelvis along the renal artery, separating the left ureter, ligating the upper 1/3 of the left ureter, suturing the incision layer by layer, and finally disinfecting with iodophor. Dosing via intraperitoneal injection started from the 2nd week of modelling. At the end of the experiment, the mouse serum was collected, and kidney tissues were taken for Masson staining index assay.

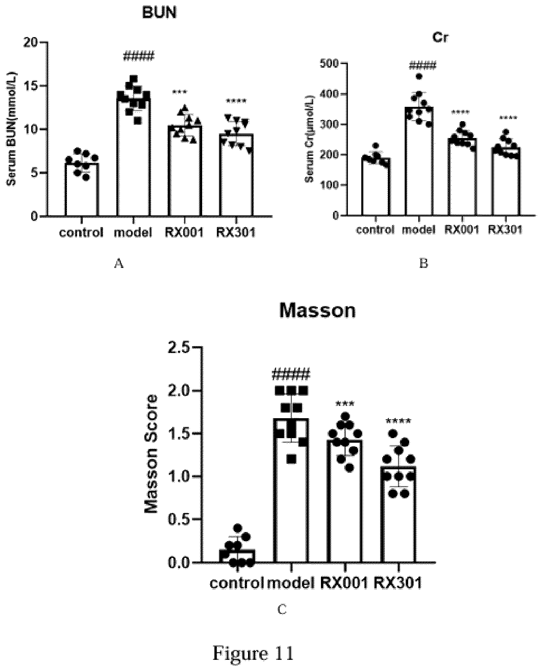

Figure 11 shows that the administration of RX001 and RX301 via intraperitoneal (IP) injection resulted in statistically significant improvements in serum creatinine (Cr) and urea nitrogen (BUN) levels and pathology in a renal fibrosis model.

### Test Example 13: Efficacy in Rabbit Ear Hypertrophic Scar Model

In this test example, the efficacy of the compounds of the present disclosure was tested on hypertrophic scars in a rabbit ear hypertrophic scar animal model. Briefly, 15 New Zealand white rabbits, male, 3 months old, were used. The New Zealand rabbits were anaesthetized with sodium pentobarbital and six circular full-thickness wounds with a diameter of 10 mm were made on the ventral surface of each ear by removing the epidermis, dermis and perichondrium to expose the cartilage. The rabbits were randomly divided into control, RX001 experimental and RX301 experimental groups. On postoperative day 14, after complete re-epithelialization of the wound surface, the compound (150 µM, 100 µL, dissolved in DMSO solution) or negative control DMSO 100 µL was injected into the center of each lesion from the wound edge once a week for a total of 4 injections. Improvement of scar formation by the drugs was assessed by SEI (Scar Excess Index).

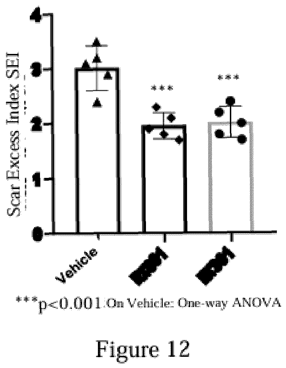

Figure 12 shows that the administration of RX001 and RX301 resulted in a statistically significant improvement in scar excess index in rabbit ear hypertrophic scar model.

## Claims

1. A benzothiazole compound, having a structure represented by formula I, or a pharmaceutically acceptable salt, solvate, hydrate, polymorph, co-crystal, tautomer, stereoisomer, or isotope labeled compound thereof, 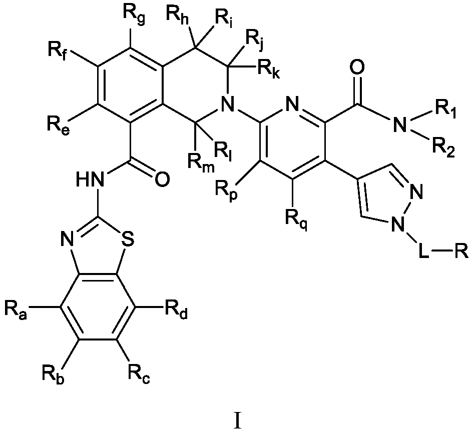 wherein,
R₁ and R₂ are independently selected from the group consisting of C1-C10 chain alkyl, C3-C8 cycloalkyl, C2-C8 chain alkenyl, C2-C8 alkynyl, C6-C20 aryl, C1-C20 heteroaryl, and C2-C20 heteroalicyclic group, wherein CH₂ in the C1-C10 chain alkyl, C3-C8 cycloalkyl, C2-C8 chain alkenyl, and C2-C8 alkynyl can be replaced with one or more groups selected from the group consisting of -O-, -S-, -SO₂-, -C(O)-, and -NR₃-, and the C1-C10 chain alkyl, C3-C8 cycloalkyl, C2-C8 chain alkenyl, C2-C8 alkynyl, C6-C20 aryl, C1-C20 heteroaryl, and C2-C20 heteroalicyclic group are optionally substituted with one or more substituents selected from the group consisting of halogen atom, cyano, nitro, C6-C20 aryl, C1-C20 heteroaryl, C1-C10 chain alkoxy, C6-C20 aryloxy, C2-C20 heteroalicyclic group, amino, hydroxyl, mercapto, 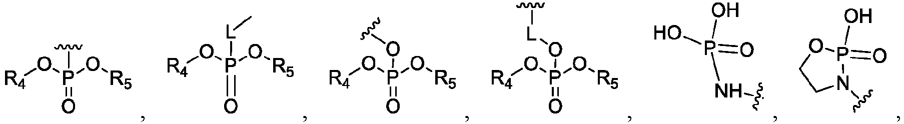 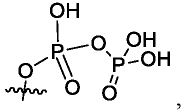 and -NR₄R₅; or
R₁ and R₂, together with the N atom to which they are connected, form a heteroalicyclic group, preferably a C2-C20 heteroalicyclic group; or
R₁ is hydrogen, and R₂ is selected from the group consisting of C1-C10 chain alkyl, C3-C8 cycloalkyl, C2-C8 chain alkenyl, C2-C8 alkynyl, C6-C20 aryl, C1-C20 heteroaryl, and C2- C20 heteroalicyclic group, wherein CH₂ in the C1-C10 chain alkyl, C3-C8 cycloalkyl, C2-C8 chain alkenyl, and C2-C8 alkynyl can be replaced with one or more groups selected from the group consisting of -O-, -S-, -SO₂-, -C(O)-, and -NR₃-, and the C1-C10 chain alkyl, C3-C8 cycloalkyl, C2-C8 chain alkenyl, C2-C8 alkynyl, C6-C20 aryl, C1-C20 heteroaryl, and C2-C20 heteroalicyclic group are substituted with halogen atom, hydroxyl, mercapto, amino, nitro, cyano, carboxyl, acyl, C1-C10 alkoxy, C6-C20 aryl, C1-C20 heteroaryl, C2-C20 heteroalicyclic group, C1-C10 chain alkyl, C2-C8 chain alkenyl, or C2-C8 alkynyl, and wherein the substituents of at least two positions together form aliphatic ring, heteroalicyclic ring, aromatic ring, or heteroaromatic ring;
R₃, R₄, and R₅ are independently selected from the group consisting of hydrogen, aryl, heteroaryl, C1-C8 chain alkyl, C3-C8 cycloalkyl, C2-C8 chain alkenyl, and C2-C8 alkynyl, and the aryl and heteroaryl are optionally substituted with halogen atom, hydroxyl, mercapto, amino, nitro, cyano, carboxyl, acyl, alkoxy, aryl, heteroaryl, heteroalicyclic group, C1-C10 alkyl, C3-C8 cycloalkyl, C2-C8 chain alkenyl, or C2-C8 alkynyl, and wherein optionally the substituents of at least two positions together form aliphatic ring, heteroalicyclic ring, aromatic ring, or heteroaromatic ring;
L is C1-C6 alkylene, and the alkylene is optionally substituted with halogen atom, hydroxyl, mercapto, amino, nitro, cyano, carboxyl, acyl, C1-C10 alkoxy, C6-C20 aryl, C1-C20 heteroaryl, C2-C20 heteroalicyclic group, C1-C10 alkyl, C3-C8 cycloalkyl, C2-C8 chain alkenyl, or C2-C8 alkynyl;
R is adamantyl, and the adamantyl is optionally substituted with halogen atom, hydroxyl, mercapto, amino, nitro, cyano, carboxyl, acyl, C1-C10 alkoxy, C6-C20 aryl, C1-C20 heteroaryl, C2-C20 heteroalicyclic group, C1-C10 alkyl, C3-C8 cycloalkyl, C2-C8 chain alkenyl, or C2-C8 alkynyl;
Rₐ, R_{b}, R_{c}, R_{d}, Rₑ, R_{f}, R_{g}, Rₕ, Rᵢ, Rⱼ, Rₖ, Rₗ, Rₘ, Rₚ, R_{q}, and R_{z} are each independently selected from the group consisting of hydrogen, halogen substituted or unsubstituted C1-C8 chain alkyl, halogen substituted or unsubstituted C3-C8 cycloalkyl, halogen substituted or unsubstituted C2-C8 chain alkenyl, halogen substituted or unsubstituted C2-C8 alkynyl, halogen atom, hydroxyl, amino, nitro, cyano, carboxyl, acyl, and halogen substituted or unsubstituted C2-C8 alkoxy.

2. The compound according to claim 1, **characterized in that**, the compound is represented by formula II: 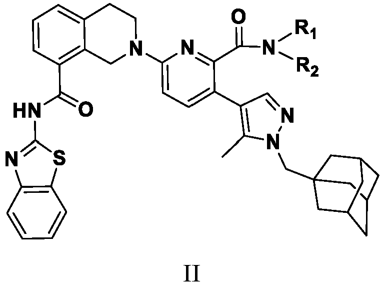 in formula II, the definitions to R₁ and R₂ are the same as those in formula I.

3. The compound according to claim 1 or 2, **characterized in that**, R₁ and R₂ together with the N atom to which they are connected, form a C2-C20 heteroalicyclic group, such as a C2-C10 heteroalicyclic group, wherein the ring of the C2-C20 heteroalicyclic group optionally contains 1 or 2 additional heteroatoms selected from the group consisting of N and O,
the C2-C20 heteroalicyclic group is optionally substituted with one or more substituents selected from the group consisting of halogen atom, cyano, nitro, C6-C10 aryl, C1-C10 heteroaryl, C1-C6 chain alkoxy, C6-C10 aryloxy, C2-C10 heteroalicyclic group, amino, hydroxyl, mercapto, carbonyl, carboxyl, acyl, 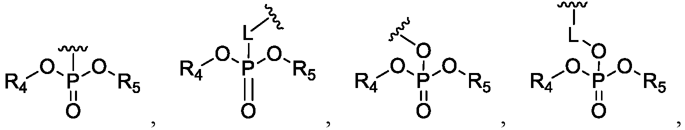 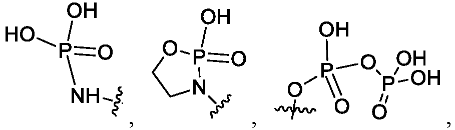 and -NR₄R₅, wherein R₄ and R₅ are independently selected from the group consisting of hydrogen, C6-C10 aryl, C1-C10 heteroaryl, C1-C8 chain alkyl, C3-C8 cycloalkyl, C2-C8 chain alkenyl, and C2-C8 alkynyl, and the aryl and heteroaryl are optionally substituted with halogen atom, hydroxyl, mercapto, amino, nitro, cyano, carboxyl, acyl, C1-C8 alkoxy, C6-C10 aryl, C1-C10 heteroaryl, C2-C10 heteroalicyclic group, C1-C8 alkyl, C3-C8 cycloalkyl, C2-C6 chain alkenyl, and C2-C8 alkynyl, optionally wherein the substituents of at least two positions together form C3-C10 aliphatic ring, C2-C10 heteroalicyclic ring, C6-C10 aromatic ring, or C1-C10 heteroaromatic ring;
for example, the C2-C20 heteroalicyclic group is optionally substituted with a substituent selected from the group consisting of halogen atom, hydroxyl, mercapto, amino, nitro, cyano, C1-C10 alkoxy, C1-C10 alkyl, C3-C8 cycloalkyl, C2-C8 chain alkenyl, C2-C8 alkynyl, 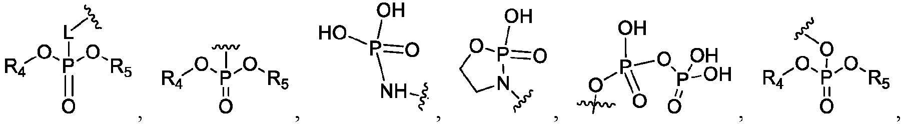 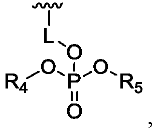 and R₄ and R₅ are independently selected from the group consisting of hydrogen and C1-C6 alkyl;
for example, the C2-C8 heteroalicyclic group is optionally substituted with a substituent selected from the group consisting of halogen, -NH₂, -OH, -NO₂, carbonyl, -CH₂OH, carboxyl, methyl, ethyl, propyl, isopropyl, methoxy, ethoxy, propoxy, and isopropoxy.

4. The compound according to claim 1 or 2, **characterized in that**, R₁ and R₂ are independently selected from the group consisting of C1-C6 chain alkyl, C3-C6 cycloalkyl, C2-C6 chain alkenyl, C2-C6 alkynyl, C6-C10 aryl, C1-C10 heteroaryl, and C2-C10 heteroalicyclic group, wherein CH₂ in the C1-C6 chain alkyl, C3-C6 cycloalkyl, C2-C6 chain alkenyl, and C2-C6 alkynyl can be replaced with one or more groups selected from the group consisting of -O-, - S -, -SO₂-, -C(O)-, and -NR₃-, and the C1-C6 chain alkyl, C3-C6 cycloalkyl, C2-C6 chain alkenyl, C2-C6 alkynyl, C6-C10 aryl, C1-C10 heteroaryl, and C2-C10 heteroalicyclic group are optionally substituted with one or more substituents selected from the group consisting of halogen atom, cyano, nitro, C6-C10 aryl, C1-C10 heteroaryl, C1-C6 chain alkoxy, C6-C10 aryloxy, C2-C10 heteroalicyclic group, amino, hydroxyl, mercapto, 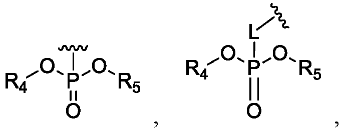 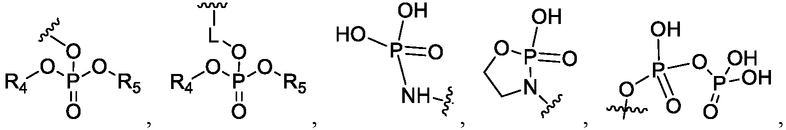 and -NR₄R₅, R₃, R₄, and R₅ are independently selected from the group consisting of hydrogen, C6-C10 aryl, C1-C10 heteroaryl, C1-C6 chain alkyl, C3-C6 cycloalkyl, C2-C6 chain alkenyl, and C2-C6 alkynyl, and the aryl and heteroaryl are optionally substituted with halogen atom, hydroxyl, mercapto, amino, nitro, cyano, carboxyl, acyl, C1-C6 alkoxy, C6-C10 aryl, C1-C10 heteroaryl, C2-C10 heteroaliphatic group, C1-C6 alkyl, C3-C6 cycloalkyl, C2-C6 chain alkenyl, or C2-C6 alkynyl, and wherein optionally the substituents of at least two positions together form C3-C10 aliphatic ring, C2-C10 heteroalicyclic ring, C6-C10 aromatic ring, or C1-C10 heteroaromatic ring.

5. The compound according to any one of claims 1 to 4, **characterized in that** the compound is represented by formula III, 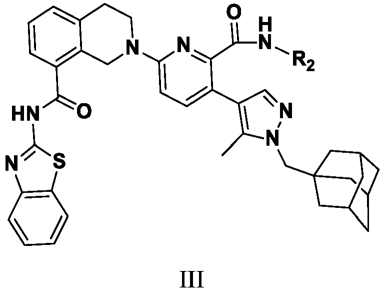 R₂ is C1-C10 chain alkyl, C3-C8 cycloalkyl, C2-C8 chain alkenyl, C2-C8 alkynyl, C6-C20 aryl, C1-C20 heteroaryl, and C2-C20 heteroalicyclic group, wherein CH₂ in the C1-C10 chain alkyl, C3-C8 cycloalkyl, C2-C8 chain alkenyl, and C2-C8 alkynyl can be replaced with one or more groups selected from the group consisting of -O-, -S-, -SO₂-, -C(O)-, and -NR₃-, and the C1-C10 chain alkyl, C3-C8 cycloalkyl, C2-C8 chain alkenyl, C2-C8 alkynyl, C6-C20 aryl, C1-C20 heteroaryl, and C2-C20 heteroalicyclic group are substituted with one or more substituents selected from the group consisting of halogen atom, cyano, nitro, C6-C20 aryl, C1-C20 heteroaryl, C1-C10 chain alkoxy, C6-C20 aryloxy, C2-C20 heteroalicyclic group, amino, hydroxyl, mercapto, 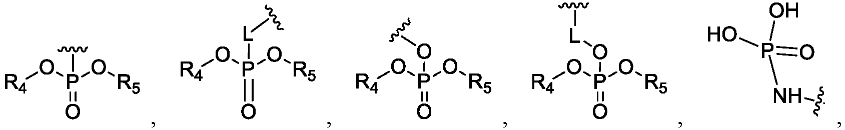 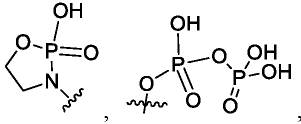 and -NR₄R₅; R₃, R₄, and R₅ are independently selected from the group consisting of hydrogen, C6-C10 aryl, C1-C10 heteroaryl, C1-C6 chain alkyl, C3-C6 cycloalkyl, C2-C6 chain alkenyl, and C2-C6 alkynyl, and the aryl and heteroaryl are optionally substituted with halogen atom, hydroxyl, mercapto, amino, nitro, cyano, carboxyl, acyl, C1-C6 alkoxy, C6-C10 aryl, C1-C10 heteroaryl, C2-C10 heteroalicyclic group, C1-C6 alkyl, C3-C6 cycloalkyl, C2-C6 chain alkenyl, or C2-C6 alkynyl, wherein optionally the substituents of at least two positions together form C3-C10 aliphatic ring, C2-C10 heteroalicyclic ring, C6-C10 aromatic ring, or C1-C10 heteroaromatic ring.

6. The compound according to any one of claims 1 to 5, **characterized in that** the heteroalicyclic group or heteroalicyclic ring is selected from the group consisting of: substituted or unsubstituted piperidinyl, substituted or unsubstituted piperazinyl, substituted or unsubstituted morpholinyl, substituted or unsubstituted piperazin-2-one group, substituted or unsubstituted pyrrolidinyl, and substituted or unsubstituted imidazolinyl.

7. The compound according to any one of claims 1 to 6, **characterized in that** R₁ and/or R₂ are selected from the group consisting of methyl, ethyl, propyl, isopropyl, methoxy, ethoxy, propoxy, isopropoxy, ethenyl, 6-membered aryl, 5-membered heteroaryl, 6-membered heteroaryl, 5-membered heterocyclyl, 6-membered heterocyclyl, 5-membered carbocyclyl, and 6-membered carbocyclyl, and each heteroaryl and each heterocyclyl contain 1 or 2 heteroatoms selected from the group consisting of N and O; and each group is independently optionally substituted with -F, -Cl, -Br, -I, -NH₂, -OH, -NO₂, carbonyl, -CH₂-OH, carboxyl, methyl, ethyl, propyl, isopropyl, methoxy, ethoxy, propoxy, and isopropoxy.

8. The compound according to any one of claims 1 to 7, **characterized in that** R₁ and/or R₂ are selected from the group consisting of 6-membered aryl, 5-membered heteroaryl, 6-membered heteroaryl, 5-membered heterocyclyl, 6-membered heterocyclyl, 5-membered carbocyclyl, and 6-membered carbocyclyl, and each heteroaryl and each heterocyclyl contain 1 or 2 heteroatoms selected from the group consisting of N and O; and each group is independently optionally substituted with -F, -Cl, -Br, -I, -NH₂, -OH, -NO₂, carbonyl, =O, -CH₂-OH, carboxyl, methyl, ethyl, propyl, isopropyl, methoxy, ethoxy, propoxy, or isopropoxy;
or R₁ and R₂ together with the N atom to which they are connected, form 6-membered aryl, 5-membered heteroaryl, 6-membered heteroaryl, 5-membered heterocyclyl, 6-membered heterocyclyl, 5-membered carbocyclyl, or 6-membered carbocyclyl, and each heteroaryl and each heterocyclyl contain 1 or 2 heteroatoms selected from the group consisting of N and O; and each group is independently optionally substituted with -F, -Cl, -Br, -I, -NH₂, -OH, -NO₂, carbonyl, =O, -CH₂-OH, carboxyl, methyl, ethyl, propyl, isopropyl, methoxy ethoxy, propoxy, or isopropoxy.

9. The compound according to any one of claims 1 to 8, **characterized in that** R₁ and R₂ together with the N atom to which they are connected, form a C2-C20 heteroalicyclic group, and/or
the C2-C20 heteroalicyclic group is selected from C4-C20 heteroalicyclic group,
preferably, the C4-C20 heteroalicyclic group is selected from the group consisting of: substituted or unsubstituted morpholinyl, substituted or unsubstituted piperidinyl, and substituted or unsubstituted piperazinyl;
preferably, the C4-C20 heteroalicyclic group is selected from the group consisting of the following groups: 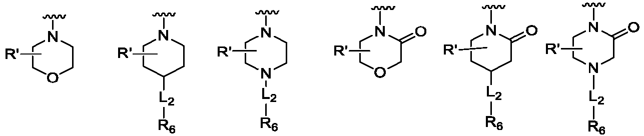 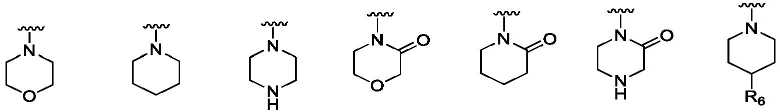 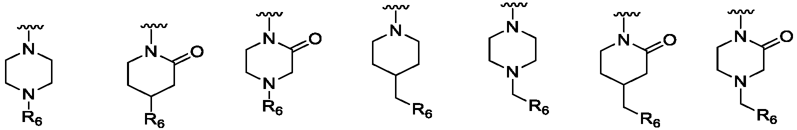 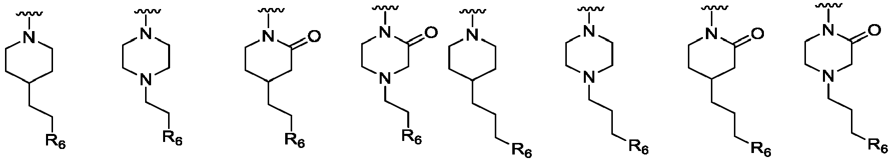
R' each independently represents no substituent, single substituent, or multiple substituents, and each substituent is independently selected from the group consisting of deuterium, hydroxyl, halogen, NH₂, carboxyl (-COOH), 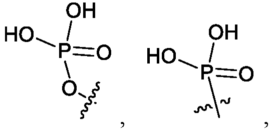
C1-C6 chain alkyl, halogen-substituted C1-C6 chain alkyl, hydroxyl-substituted C1-C6 chain alkyl, amino-substituted C1-C6 chain alkyl, morpholine-substituted C1-C6 chain alkyl, -COO-C1-C6 chain alkyl, cyano, C1-C6 chain alkoxy, C3-C6 cycloalkyl, halogen-substituted C3-C6 cycloalkyl, hydroxyl-substituted C3-C6 cycloalkyl, phenyl, and benzyl;
L₂ is absent, or C1-C6 alkylene, orC1-C6 alkylene substituted with halogen, hydroxyl, or C1-C6 alkoxy, preferably methylene, ethylene, or propylene;
R₆ is H, deuterium, halogen, hydroxyl, NH₂, carboxyl (-COOH), -CONH₂, sulfonic acid group (-SO₃H), -SO₂-C1-C6 chain alkyl, 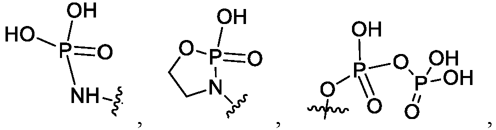 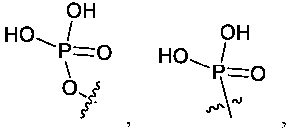 C1-C6 chain alkyl, halogen-substituted C1-C6 chain alkyl, morpholine-substituted C1-C6 chain alkyl, -COO-C1-C6 chain alkyl, cyano, C1-C6 chain alkoxy, hydroxyl-substituted C1-C6 chain alkyl, amino-substituted C1-C6 chain alkyl, C3-C6 cycloalkyl, halogen-substituted C3-C6 cycloalkyl, hydroxyl-substituted C3-C6 cycloalkyl, phenyl, or benzyl.

10. The compound according to claim 1, **characterized in that** the compound has the structure represented by formula I-1, formula I-2, formula I-3, formula I-4, or formula I-5, 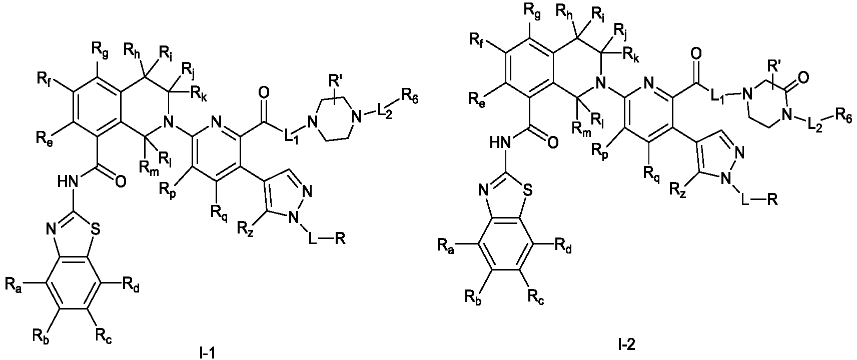 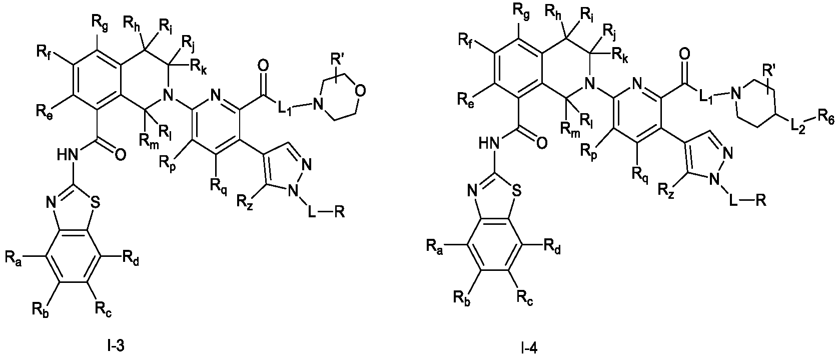 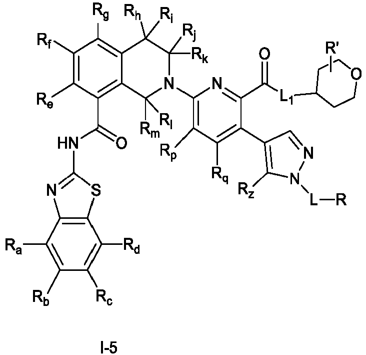
L₁ is absent or -NHL₃-, L₃ is absent, or C1-C6 alkylene or C1-C6 alkylene substituted with halogen, hydroxyl, or C1-C6 alkoxy, preferably methylene, ethylene, or propylene;
L₂ is absent, or C1-C6 alkylene, or C1-C6 alkylene substituted with halogen, hydroxyl, or C1-C6 alkoxy, preferably methylene, ethylene, or propylene;
R₆ is H, deuterium, halogen, hydroxyl, NH₂, carboxyl (-COOH), -CONH₂, sulfonic acid group (-SO₃H), -SO₂-C1-C6 chain alkyl, 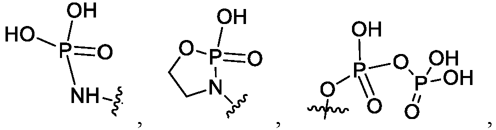 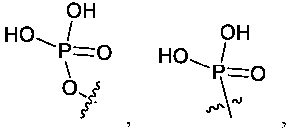 C1-C6 chain alkyl, halogen-substituted C1-C6 chain alkyl, morpholine-substituted C1-C6 chain alkyl, -COO-C1-C6 chain alkyl, cyano, C1-C6 chain alkoxy, hydroxyl-substituted C1-C6 chain alkyl, amino-substituted C1-C6 chain alkyl, C3-C6 cycloalkyl, halogen-substituted C3-C6 cycloalkyl, hydroxyl-substituted C3-C6 cycloalkyl, phenyl, or benzyl;
R' each independently represents no substituent, single substituent, or multiple substituents, and each substituent is independently selected from the group consisting of H, deuterium, halogen, hydroxyl, NH₂, carboxyl (-COOH), -CONH₂, sulfonic acid group (-SO₃H), -SO₂-C1-C6 chain alkyl, 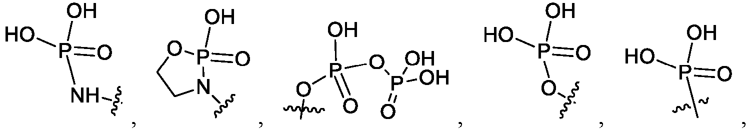 C1-C6 chain alkyl, halogen-substituted C1-C6 chain alkyl, morpholine-substituted C1-C6 chain alkyl, -COO-C1- C6 chain alkyl, cyano, C1-C6 chain alkoxy, hydroxyl-substituted C1-C6 chain alkyl, amino-substituted C1-C6 chain alkyl, C3-C6 cycloalkyl, halogen-substituted C3-C6 cycloalkyl, hydroxyl-substituted C3-C6 cycloalkyl, phenyl, or benzyl;
the definitions to the other symbols are the same as those in formula I.

11. The compound according to any one of claims 1 to 10, **characterized in that**,
Rₐ, R_{b}, R_{c}, and R_{d} are each independently selected from the group consisting of hydrogen and C1-C6 alkyl; and/or
Rₑ, R_{f}, and R_{g} are each independently selected from the group consisting of hydrogen and C1-C6 alkyl; and/or
Rₕ, Rᵢ, Rⱼ, Rₖ, Rₗ, and Rₘ are each independently selected from the group consisting of hydrogen and C1-C6 alkyl; and/or
Rₚ, R_{q}, and R_{z} are each independently selected from the group consisting of hydrogen and C1-C6 alkyl; and/or
L is methylene; and/or
R_{z} is methyl; and/or
R is

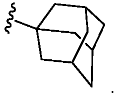

12. The compound according to claim 10 or 11, **characterized in that**, L₁ is absent; and/or
R₆ is H, halogen, hydroxyl, NH₂, carboxyl, 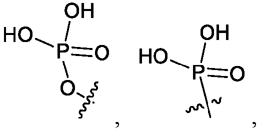 C1-C3 chain alkyl, halogen-substituted C1-C3 chain alkyl, or hydroxyl-substituted C1-C3 chain alkyl;
preferably, R₆ is

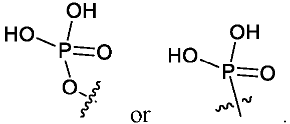

13. The compound according to any one of claims 1 to 12, **characterized in that** the benzothiazole compound is selected from the group consisting of the following compounds:

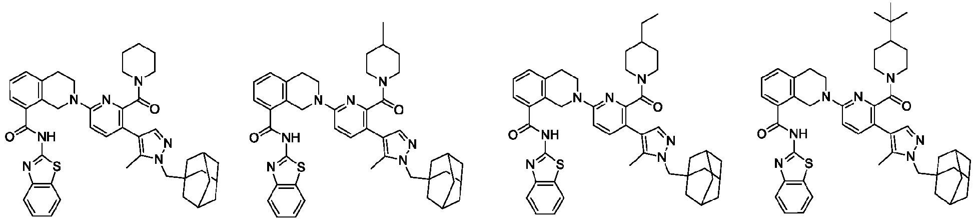

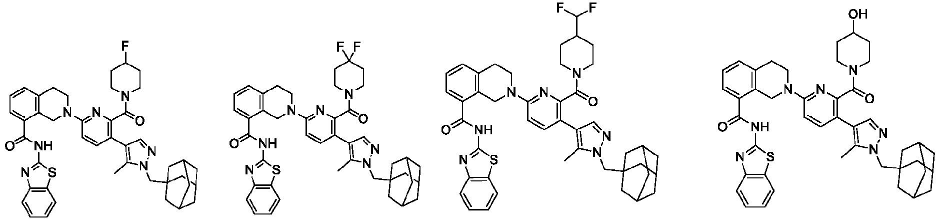

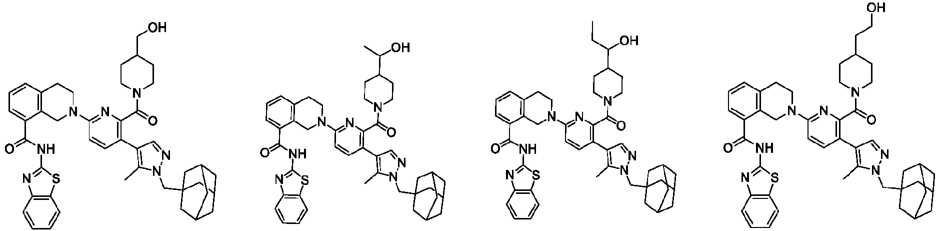

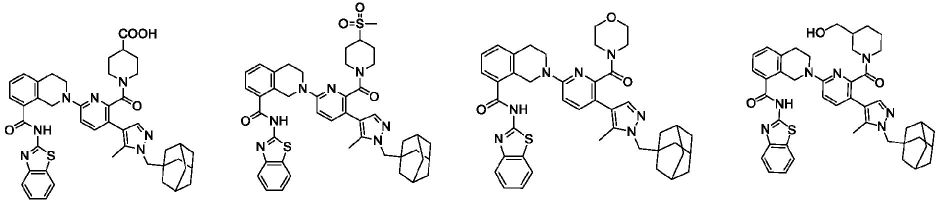

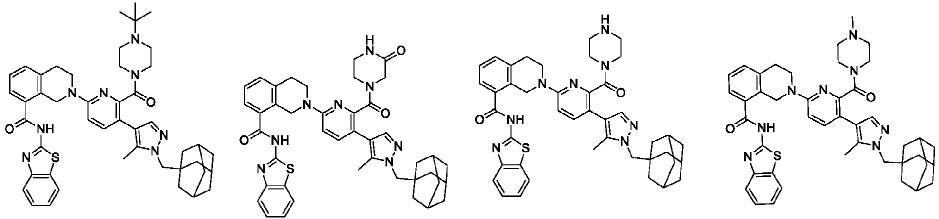

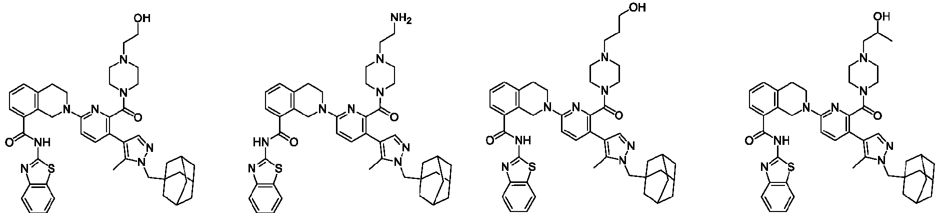

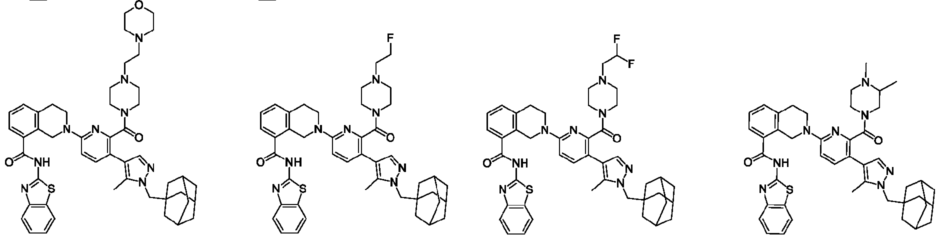

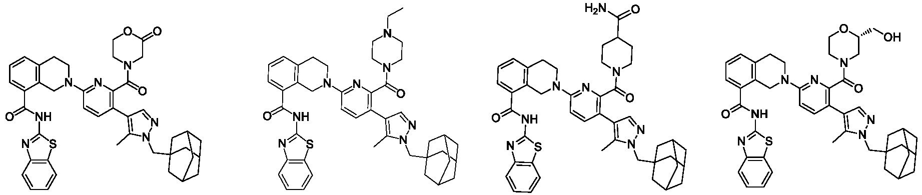

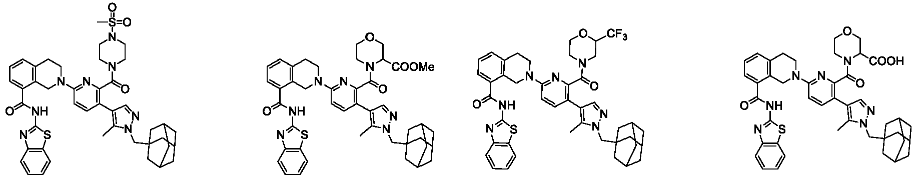

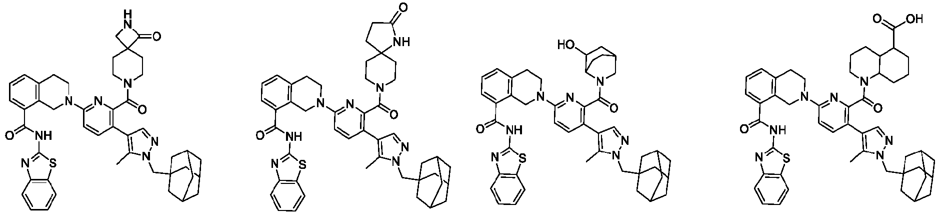

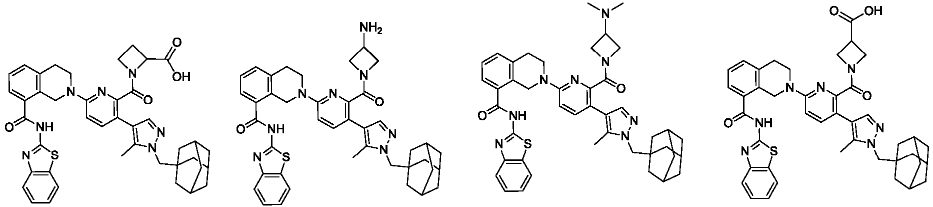

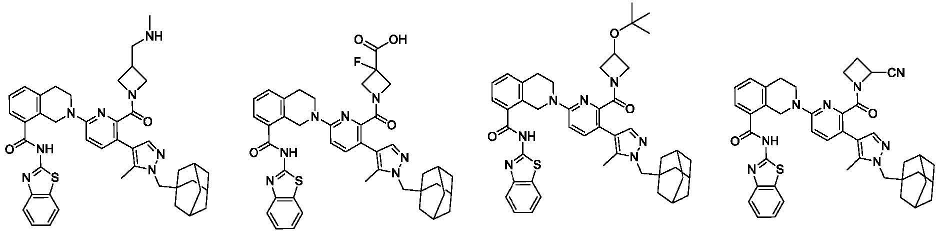

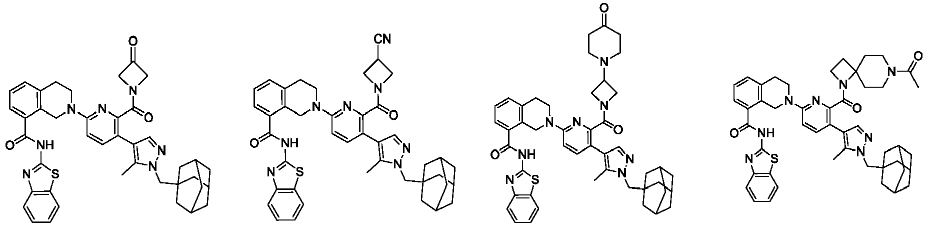

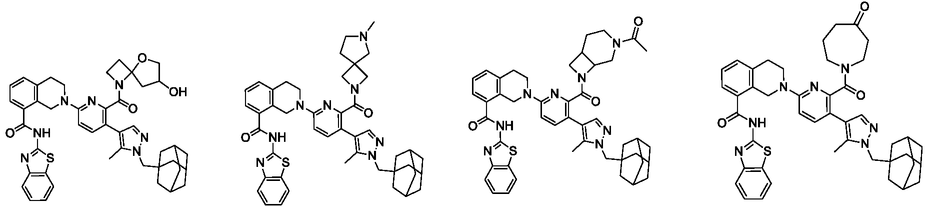

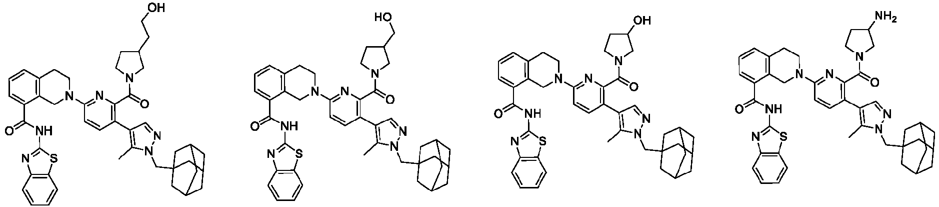

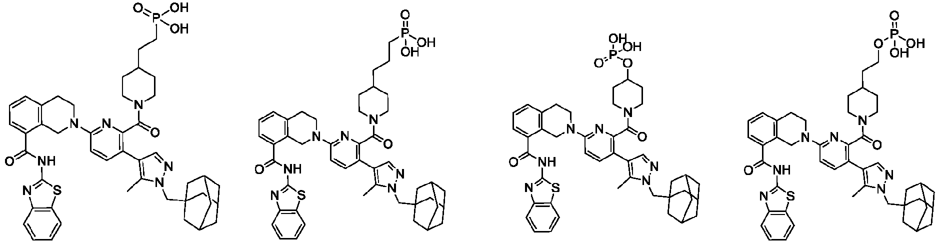

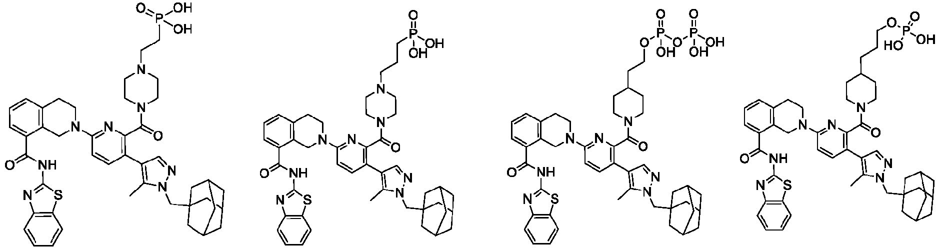

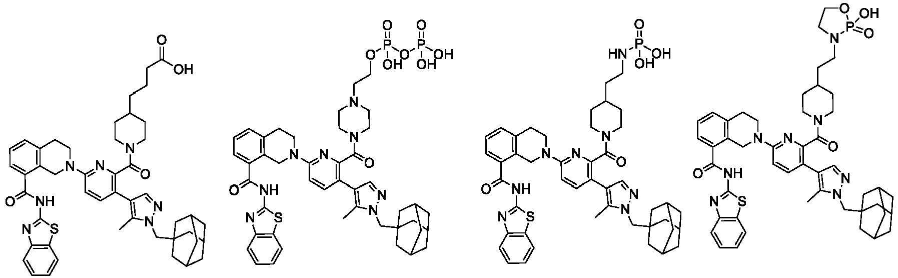

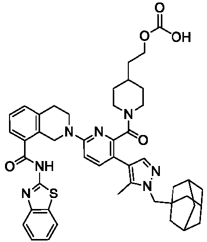

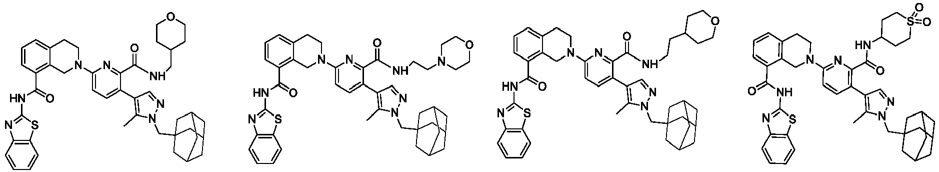

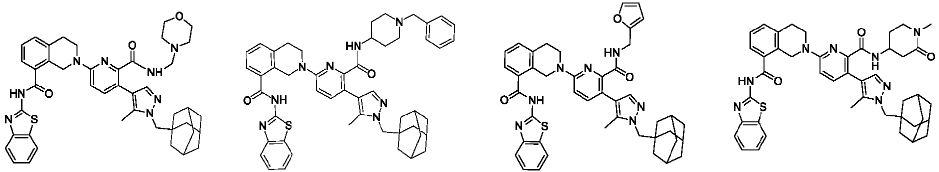

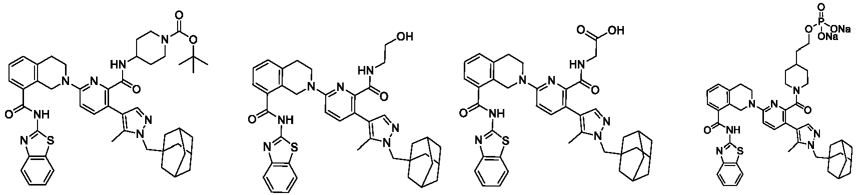

14. The compound according to any one of claims 1 to 13, or a pharmaceutically acceptable salt, a solvate, a hydrate, a polymorph, a co-crystal, a tautomer, a stereoisomer, or an isotope labeled compound thereof, **characterized in that** the salt of the compound is an alkali metal salt, preferably a sodium salt.

15. A pharmaceutical composition, comprising the compound according to any one of claims 1 to 14 or a pharmaceutically acceptable salt, a solvate, a hydrate, a polymorph, a co-crystal, a tautomer, a stereoisomer, or an isotope labeled compound thereof, and a pharmaceutically acceptable excipient.

16. A method for preventing or treating a disease related to aging, comprising administering to a subject in need a therapeutically effective amount of the compound according to any one of claims 1 to 14 or a pharmaceutically acceptable salt, a solvate, a hydrate, a polymorph, a co-crystal, a tautomer, a stereoisomer, or an isotope labeled compound thereof, or the pharmaceutical composition according to claim 15.

17. The method according to claim 16, **characterized in that** the disease is selected from the group consisting of diseases related to accumulation of senescent cells, and the disease is preferably one or more selected from the group consisting of idiopathic pulmonary fibrosis, pulmonary fibrosis, hepatic fibrosis, renal fibrosis, inflammation and tissue fibrosis and atrophy of upper respiratory tract and lungs caused by viruses, cystic fibrosis, myelofibrosis, myocardial fibrosis, cutaneous fibrosis, interstitial lung disease, fibrotic pancreatitis, retinopathy of prematurity, macular degeneration, diabetic macular edema, diabetic retinopathy, age-related macular degeneration, wet age-related macular degeneration, dry age-related macular degeneration, glaucoma, sickle cell retinopathy, ischemic arteritis neuropathy, keratitis sicca, Fuch's corneal dystrophy, presbyopia, cataract, degenerative vitreous disorder, including vitreomacular traction syndrome, macular hole, retinal tear, retinal detachment, and proliferative vitreoretinopathy, osteoarthritis, disc herniation, osteoporosis, Alzheimer's disease, Parkinson's disease, atherosclerosis, chronic obstructive pulmonary disease, diabetes, diabetic nephropathy, scar, superficial scar or flat scars, rope scar or contracted scar, webbed scar, depressed scar, atrophic scar, bridge scar and pedunculated scar, hypertrophic scar, keloid, scar cancer, scleroderma, morphea, linear scleroderma, guttate scleroderma, acroscleroderma, diffuse scleroderma, CREST syndrome, acute coronary syndrome, myocardial infarction, stroke, hypertension, obesity, lipodystrophy, coronary artery disease, cerebrovascular disease, periodontal disease, cancer treatment-related disabilities such as atrophy and fibrosis in various tissues, brain and heart damage and treatment-related myelodysplastic syndrome, promyelocytic syndrome, ataxia telangiectasia, Fanconi anemia, Friedreich's ataxia, congenital dyskeratosis, aplastic anemia, aneurysm, inflammatory bowel disease, lipoatrophy, renal transplant failure, sarcopenia, wound healing, alopecia, cardiomyocyte hypertrophy, glomerulosclerosis, and cancer.

18. Use of the compound according to any one of claims 1 to 14 or a pharmaceutically acceptable salt, a solvate, a hydrate, a polymorph, a co-crystal, a tautomer, a stereoisomer, or an isotope labeled compound thereof, or the pharmaceutical composition according to claim 15 in the preparation of a medicament for preventing or treating a disease related to aging.

19. The use according to claim 15, **characterised in that** the disease is selected from the group consisting of diseases related to accumulation of senescent cells, and the disease is preferably one or more selected from the group consisting of idiopathic pulmonary fibrosis, pulmonary fibrosis, hepatic fibrosis, renal fibrosis, inflammation and tissue fibrosis and atrophy of upper respiratory tract and lungs caused by viruses, cystic fibrosis, myelofibrosis, myocardial fibrosis, cutaneous fibrosis, interstitial lung disease, fibrotic pancreatitis, retinopathy of prematurity, macular degeneration, diabetic macular edema, diabetic retinopathy, age-related macular degeneration, wet age-related macular degeneration, dry age-related macular degeneration, glaucoma, sickle cell retinopathy, ischemic arteritis neuropathy, keratitis sicca, Fuch's corneal dystrophy, presbyopia, cataract, degenerative vitreous disorder, including vitreomacular traction syndrome, macular hole, retinal tear, retinal detachment, and proliferative vitreoretinopathy, osteoarthritis, disc herniation, osteoporosis, Alzheimer's disease, Parkinson's disease, atherosclerosis, chronic obstructive pulmonary disease, diabetes, diabetic nephropathy, scar, superficial scar or flat scars, rope scar or contracted scar, webbed scar, depressed scar, atrophic scar, bridge scar and pedunculated scar, hypertrophic scar, keloid, scar cancer, scleroderma, morphea, linear scleroderma, guttate scleroderma, acroscleroderma, diffuse scleroderma, CREST syndrome, acute coronary syndrome, myocardial infarction, stroke, hypertension, obesity, lipodystrophy, coronary artery disease, cerebrovascular disease, periodontal disease, cancer treatment-related disabilities such as atrophy and fibrosis in various tissues, brain and heart damage and treatment-related myelodysplastic syndrome, promyelocytic syndrome, ataxia telangiectasia, Fanconi anemia, Friedreich's ataxia, congenital dyskeratosis, aplastic anemia, aneurysm, inflammatory bowel disease, lipoatrophy, renal transplant failure, sarcopenia, wound healing, alopecia, cardiomyocyte hypertrophy, glomerulosclerosis, and cancer.
